# EUROPEAN PATENT SPECIFICATION

(11) **EP 3 966 344 B1**
(45) Date of publication and mention of the grant of the patent: **25.06.2025**
(21) Application number: 20724921.0
(22) Date of filing: 01.05.2020
(51) Int. Cl.: C12Q 1/48

(54) **KINASE SCREENING ASSAYS**
KINASE-SCREENING-TESTS
DOSAGES DE CRIBLAGE DE KINASE

(30) Priority: 08.05.2019 GB 201906445
(43) Date of publication of application: 16.03.2022
(73) Proprietor: University of Newcastle Upon Tyne, Newcastle upon Tyne Tyne and Wear NE1 7RU (GB)
(72) Inventor: HIGGINS, Jonathan, Newcastle upon Tyne Tyne and Wear NE1 7RU (GB); WATSON, Nikolaus, Newcastle upon Tyne Tyne and Wear NE1 7RU (GB)
(74) Representative: HGF
(86) International application number: PCT/GB2020/051073
(87) International publication number: WO 2020/225535

(56) References cited:
- WO-A1-2016/161086
- US-A1- 2010 158 968
- US-A1- 2014 287 447
- ANGELA PAPAGEORGIOU,JOSEPH RAPLEY,JILL P. MESIROV,PABLO TAMAYO,JOSEPH AVRUCH: "A Genome-Wide siRNA Screen in Mammalian Cells for Regulators of S6 Phosphorylation", PLOS ONE, 19 March 2015 (2015-03-19), pages 1 - 26, XP002799945
- DUSTIN J. MALY, JASMINA A. ALLEN, AND KEVAN M. SHOKAT: "A Mechanism-Based Cross-Linker for the Identification of Kinase-Substrate Pairs", J. AM. CHEM. SOC, vol. 126, no. 30, 13 July 2004 (2004-07-13), pages 9160 - 9161, XP002799946
- NIKOLAUS A. WATSON ET AL: "Kinase inhibition profiles as a tool to identify kinases for specific phosphorylation sites", NATURE COMMUNICATIONS, vol. 11, no. 1, 3 April 2020 (2020-04-03), XP055719965, DOI: 10.1038/s41467-020-15428-0

## Description

The invention provides a novel method for identifying or profiling a target cellular kinase. Uses of such kinases (or their corresponding inhibitors) in diagnosis or therapy are also provided.

### Background

Phosphorylation is a vital regulatory system in cells. For example, 90% of all cellular proteins undergo phosphorylation (Wilson et al., 2018). Advances in phosphoproteomics mean that tens of thousands of phosphorylation sites are now known (Lemeer and Heck, 2009; von Stechow et al., 2015). Large catalogues of specific phosphorylation events that occur within specific cell types or organelles, or after specific treatments or at particular phases of the cell cycle, have been generated (Dephoure et al., 2008; Olsen et al., 2006).

However, understanding the biology controlled by a particular phosphorylation event depends to a great extent on being able to identify the kinase(s) responsible for catalyzing it. Unfortunately, the methods available for assigning kinase-phosphosite dependencies are limited. Consequently, the ability to benefit from the expanding knowledge of cellular phosphorylation has been hampered.

Of the techniques that have been developed to interrogate kinase-phosphosite dependencies, most focus on identifying the targets of a kinase of interest; far fewer perform the similarly valuable task of identifying the kinase(s) that phosphorylate a particular residue within a substrate (Johnson and Hunter, 2005). Indeed, the current inventors are not aware of an experimental method that has found widespread use that can selectively identify direct upstream kinases for any substrate. Broadly, existing approaches can be divided into three categories: (i) *in silico* predictions; (ii) screens in intact cells, and (iii) biochemical methods, often using cell extracts or recombinant kinases. Some examples of the existing approaches in each of these categories are discussed briefly below.

For a subset of kinases, consensus or optimal protein phosphorylation motifs have been determined (Songyang et al., 1994; Miller et al., 2008). A known phosphosite can be compared to these motifs to identify candidate kinases *in silico* (Yaffe et al., 2001). However, such motifs have been produced for only about half of all human kinases and validated for far fewer. It is clear that false positives and negatives are frequent using prediction methods, even when combined with contextual information such as protein-protein interaction data, co-expression, or co-occurrence in literature abstracts (Miller et al., 2008; Linding et al., 2007; Song et al., 2012; Trost and Kusalik, 2011).

Techniques such as kinome-wide RNAi, CRISPR/Cas9 or overexpression screens can also be used to identify candidate kinases in intact cells. Such screens are tremendously useful to biologists, but they often identify pathways or networks of kinases that are indirectly required for phosphorylation of a particular substrate rather than (or in addition to) the direct kinase (Friedman and Perrimon, 2006). Indeed, these indirect effects can make it difficult to identify the direct kinase for a particular phosphorylation site. For example, if RNAi for a kinase impedes cell cycle progression, it is difficult to determine whether its effect on a substrate is direct (Moffat et al., 2006).

A number of biochemical approaches to kinase identification have also been developed. Arrays of recombinant kinases can be tested for their ability to phosphorylate substrates *in vitro* (Jansson et al., 2008), but such libraries are incomplete, expensive, not widely available, and may not reflect the activity state of kinases in cells. Co-immunoprecipitation or other methods can be used to identify proteins that bind to substrates, but these techniques are not specific for kinases, nor for particular phosphosites, and kinase-substrate interactions may not be robust enough to be detected. Methods that make use of modified forms of substrates and/or ATP in an effort to crosslink substrates specifically to their cognate kinases in cell extracts are promising. However, where tested, the specificity of kinase crosslinking has been low, these approaches require additional steps such as mass spectrometry to identify the crosslinked kinase (Maly et al., 2004; Riel-Mehan and Shokat, 2014; Dedigama-Arachchige and Pflum, 2016), and they have not yet been used to identify unknown kinases. Tracking a specific kinase activity through biochemical enrichment steps can also be carried out, but this often requires large amounts of cell extract, multiple steps, and protein sequencing methods to identify candidate kinases (Rubin and Rosen, 1975; Downward et al., 1984; Kubota et al., 2009).

A technically straightforward and broadly applicable method to identify cellular kinases responsible for specific phosphorylation events is needed.

### Brief summary of the disclosure

The inventors have developed a new method for identifying or profiling kinases that are directly responsible for a specific phosphorylation event in a cell. The novel methods described herein overcome many of the limitations associated with known techniques described above.

An example of a method according to the present invention is referred to herein as *"Kinase inhibitor Profiling to Identify Kinases"* (KiPIK). Conventional wisdom suggests small molecule inhibitors are not appropriate tools for finding direct kinases because all such inhibitors inhibit more than one target, and signalling cascades in cells mean that indirect effects are common (Davies et al., 2000). KiPIK circumvents these problems by utilizing inhibition information for recombinant kinases as *"fingerprints'"* for the identification of kinases acting on target phosphorylation sites in cell extracts. KiPIK is described in detail herein and is validated on diverse known kinase-phosphosite pairs. In addition, it is shown herein that KiPlK can be used to identify the cellular kinase for an as yet unassigned phosphosite on the Chromosomal Passenger Complex protein INCENP.

Advantages of the method described herein over methods of the prior art include (i) it identifies the kinase directly responsible for the phosphorylation event of interest; (ii) it can be applied to cell extracts and cell lysates; (iii) it is rapid and relatively easy to perform; and (iv) it can be used to indicate a group of kinases for further investigation if the kinase is unknown e.g. when there is no known inhibitor profile that exactly matches the target kinase inhibitor profile (i.e. it can aid prioritization of the right group of kinases for further investigation and thus profile the target kinase).

A method of identifying or profiling a target cellular kinase that phosphorylates a selected substrate is provided herein, comprising:
a) providing a plurality of test protein preparations comprising the target cellular kinase and at least one phosphatase inhibitor;
b) incubating each test protein preparation with the selected substrate and a kinase inhibitor, wherein the kinase inhibitor is different for each test protein preparation;
c) determining whether the selected substrate is phosphorylated in each of the test protein preparations of b), thereby obtaining an inhibition profile for the target cellular kinase; and
d) comparing the target cellular kinase inhibition profile with the inhibition profiles of known kinases, wherein the most similar known kinase inhibition profile(s) identify or profile the target cellular kinase.

Suitably, the method may further comprise the step of lysing cells comprising the target cellular kinase in the presence of at least one phosphatase inhibitor to generate the plurality of protein preparations of step a).

Suitably, the cell sample may be human.

Suitably, the protein preparations may be cell lysates or cell extracts.

Suitably, the at least one phosphatase inhibitor may be selected from: okadaic acid, sodium fluoride, β-glycerophosphate, calyculin A, microcystin LR, cantharidin, sodium molybdate, sodium tartrate, (-)-p-bromotetramisole oxalate, sodium orthovanadate, sodium pyrophosphate, imidazole, Ascomycin, Cyclosporin A, FK506, Fostriecin, Fumonisin B1, GSK2830371, 9,10-Dihydro-9,10[1',2']-benzenoanthracene-1,4-dione, Raphin 1, Salubrinal, Sal 003, Sanguinarine, Sephin 1, Tautomycetin, Tautomycin, DL-2-Amino-3-phosphonopropionic acid (AP-3), Arcapillin, Endothall, Levamisole, Rubratoxin or combinations thereof.

Suitably, the selected substrate may have a single tyrosine, threonine or serine phosphosite.

Suitably, the selected substrate may be a peptide.

Suitably, the selected substrate may be at a concentration of from about 0.01 µM to about 10 µM in step b).

Suitably, at least ten different kinase inhibitors may be used individually in step b).

Suitably, the kinase inhibitor may be at a concentration of from about 0.1 µM to about 50 µM in step b).

Suitably, step b) may be for at least 5 minutes.

Suitably, phosphorylation of the selected substrate may be determined using ³²P-orthophosphate radiolabelling, mass changes as determined by mass spectrometry or other means, phospho-specific antibodies, ELISA, immunoblotting or a combination thereof.

Suitably, the target cellular kinase inhibition profile may be compared to at least ten known inhibition profiles.

Suitably, the comparison in step d) may further comprise generating at least one of the following:
(i) ranking similarity (e.g. ranking of correlation coefficients), supported by kinase inhibition correlation plots;
(ii) plotting similarity (e.g. plotting correlation coefficients) on a sequence-based human kinome tree;
(iii) an inhibition-based kinome dendrogram derived by hierarchical clustering using similarity (e.g. correlation coefficients), optionally with bootstrapping; or
(iv) a combination of (i) to (iii).

Suitably, the method may further comprise stimulating a signalling pathway associated with phosphorylation of the selected substrate and/or synchronising the cell cycle in the cells before generating the plurality of test protein preparations.

Suitably, the target cellular kinase may be associated with aberrant phosphorylation in a disease or disorder.

Suitably, the disease or disorder may be cancer, rheumatoid arthritis, chronic immune thrombocytopenia, glaucoma, idiopathic pulmonary fibrosis.

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

The patent, scientific and technical literature referred to herein establish knowledge that was available to those skilled in the art at the time of filing.

In the case of any inconsistencies, the present disclosure will prevail.

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionary of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a", "an," and "the" include the plural reference unless the context clearly indicates otherwise. Unless otherwise indicated, polynucleotides are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art. The invention is defined in the appended claims.

Various aspects of the invention are described in further detail below.

Any of the following figures that do not fall within the scope of the appended claims are provided for comparative purposes only.

### Brief description of the drawings

Embodiments of the invention are further described hereinafter with reference to the accompanying drawings, in which:

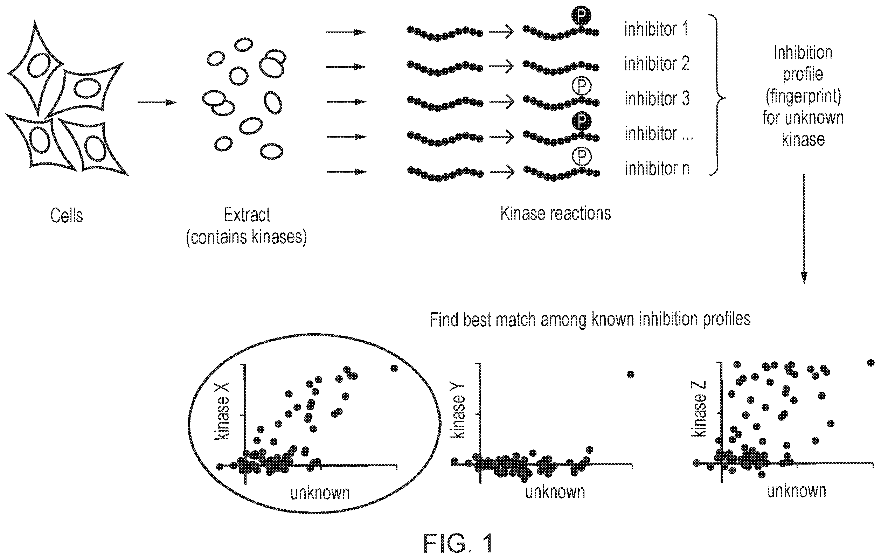

**Figure 1****.** Outline of the KiPIK method.

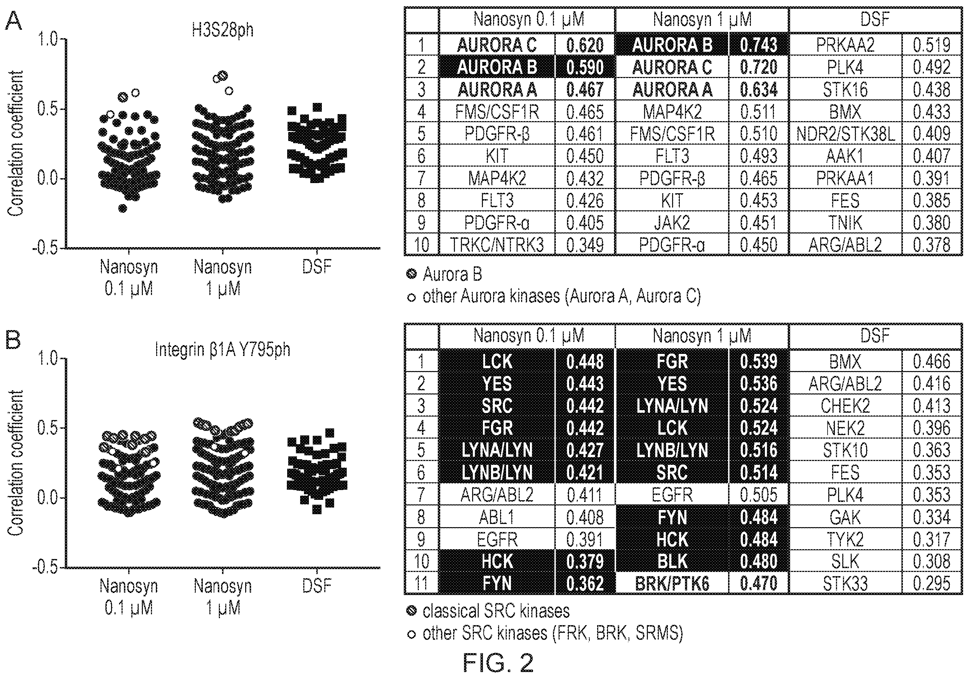

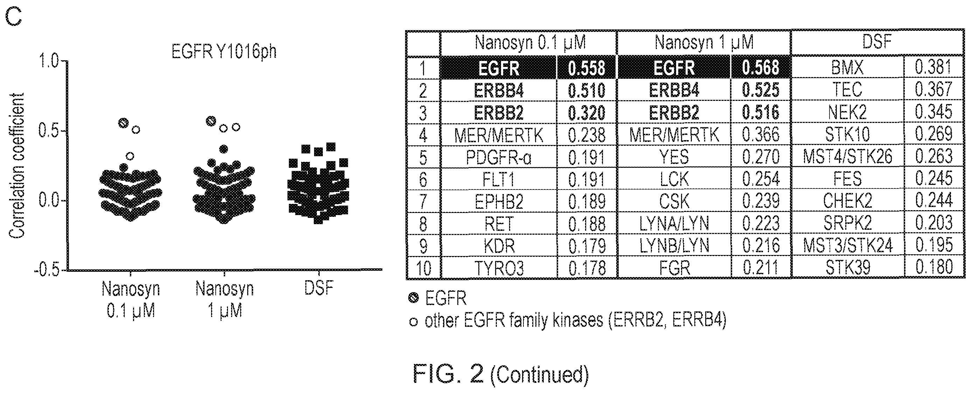

**Figure 2****.** Results of KiPlK screens using the PKIS1 inhibitor library for **A.** H3S28ph, **B.** Integrin β1A Y795ph, and C. EGFR Y1016ph. Scatterplots on the left show the Pearson correlation coefficients of the %inhibition results from the KiPIK screen against the % inhibition results for each of the kinases in the Nanosyn profiling datasets at 0.1 and 1 µM, and the DSF profiling dataset. The tables on the right show the top 10 (or 11) hits from each profiling dataset with corresponding correlation coefficients. Expected kinases are shown in inverted bold type, and closely related kinases in bold. Note that Aurora kinases are not present in the DSF dataset, and Src and EGFR-related kinases are not present in the Nanosyn datasets.

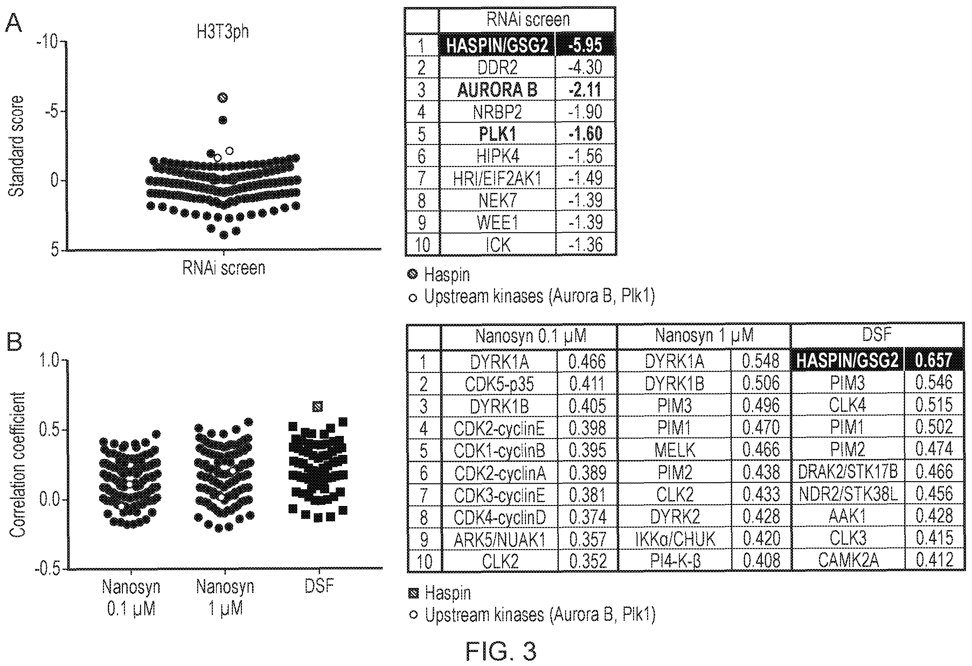

**Figure 3****.** Results of RNAi and KiPlK screens for H3T3ph. **A.** A kinome-wide RNAi screen for H3T3ph in mitosis was carried out HeLa cells using immunofluorescence and High Content Analysis. The scatterplot on the left shows the standard score (proportional to staining intensity) for each protein kinase in the library. The table on the right shows the top 10 protein kinase hits from the screen with corresponding standard scores. **B.** Results of a KiPlK screen using the PKIS1 inhibitor library for H3T3ph. The scatterplot on the left shows the Pearson correlation coefficients of the %inhibition results from the KiPlK screen against the % inhibition results for each of the kinases in the Nanosyn profiling datasets at 0.1 and 1 µM, and the DSF profiling dataset. The table on the right shows the top 10 hits from each profiling dataset with corresponding correlation coefficients. The expected kinase (Haspin/GSG2) is shown in inverted bold type, and kinases that are known to indirectly modulate H3T3ph are shown in bold. Note that Haspin/GSG2 is not present in the Nanosyn datasets.

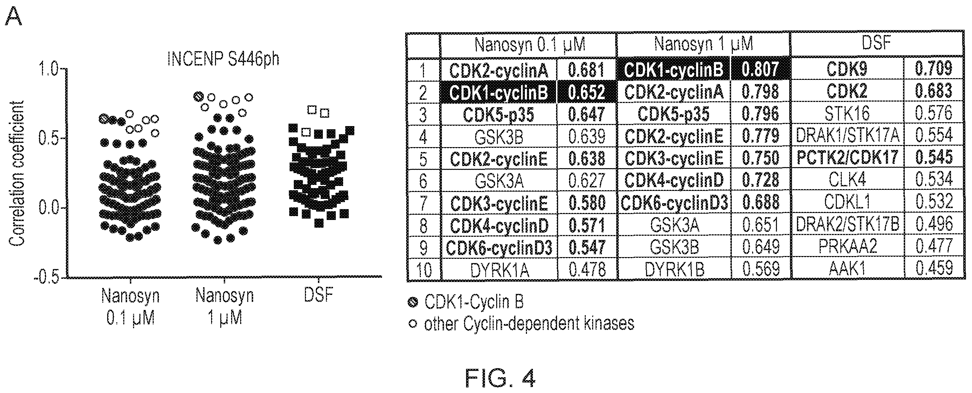

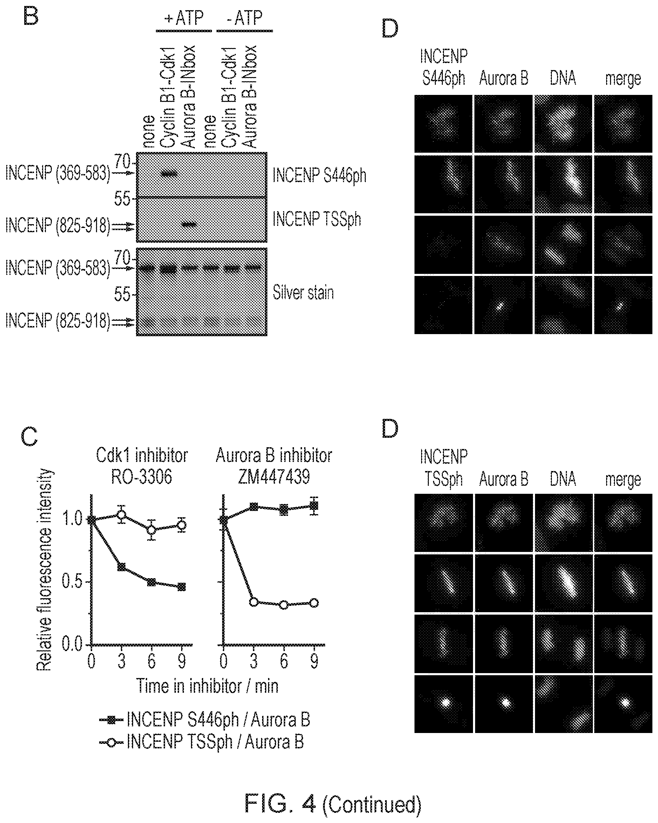

**Figure 4****.** Results of a KiPlK screen for INCENP S446ph, and confirmatory experiments in cells. **A.** The scatterplot on the left shows the Pearson correlation coefficients of the %inhibition results from the KiPlK screen against the % inhibition results for each of the kinases in the Nanosyn profiling datasets at 0.1 and 1 µM, and the DSF profiling dataset. The
table on the right shows the top 10 hits from each profiling dataset with corresponding correlation coefficients. The overall top hit kinase is shown in inverted bold type, and closely related kinases in bold. Note that Cdk1 is not present in the DSF dataset. **B.** Recombinant Cyclin B-Cdk1 efficiently phosphorylates INCENP S446, but not INCENP TSS, *in vitro.*
Recombinant INCENP(369-583) and INCENP(825-918) were provided as potential substrates for GST-CDK1/GST-CycB or Aurora B/INbox and phosphorylation was detected using the corresponding phosphospecific antibodies. A parallel gel of the same samples was silver stained. **C.** Acute inhibition of Cdk1, but not Aurora B, lowers INCENP S446ph but not INCENP TSSph in mitotic HeLa cells. In contrast, acute inhibition of Aurora B lowers INCENP TSSph,
but not S446ph. The y axis shows the ratio of INCENP phosphorylation / Aurora B intensity on chromatin. Only cells in which Aurora B was on chromatin were analyzed. **D.**
Immunofluorescence microscopy of INCENP S446ph and INCENP TSSph in mitotic HeLa cells. Both INCENP S446ph and TSSph are phosphorylated in early mitosis but, after INCENP and Aurora B move to the spindle midzone in anaphase cells, INCENP is phosphorylated at the TSSph Aurora B target residues but not at the Cdk1 target S446ph.

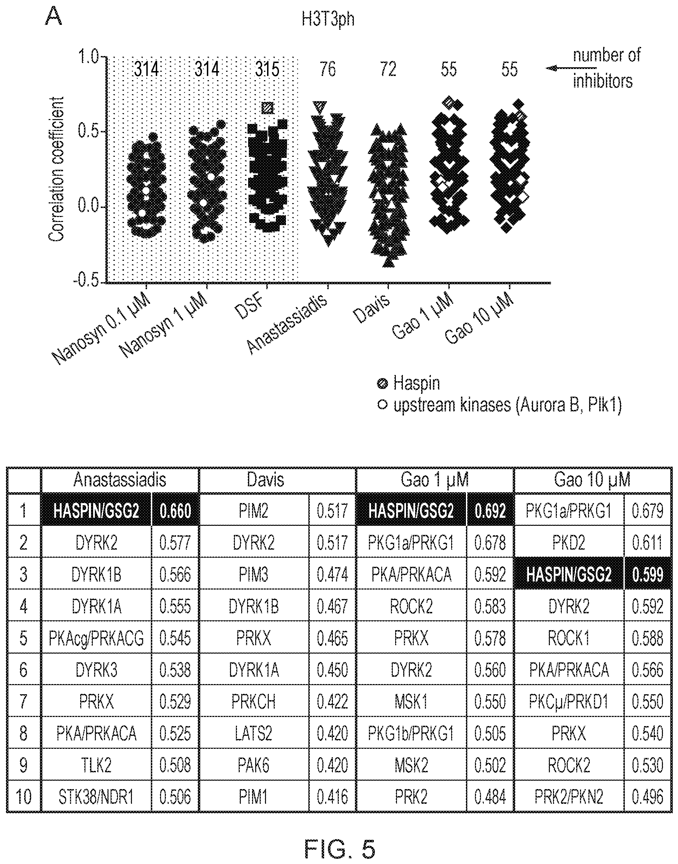

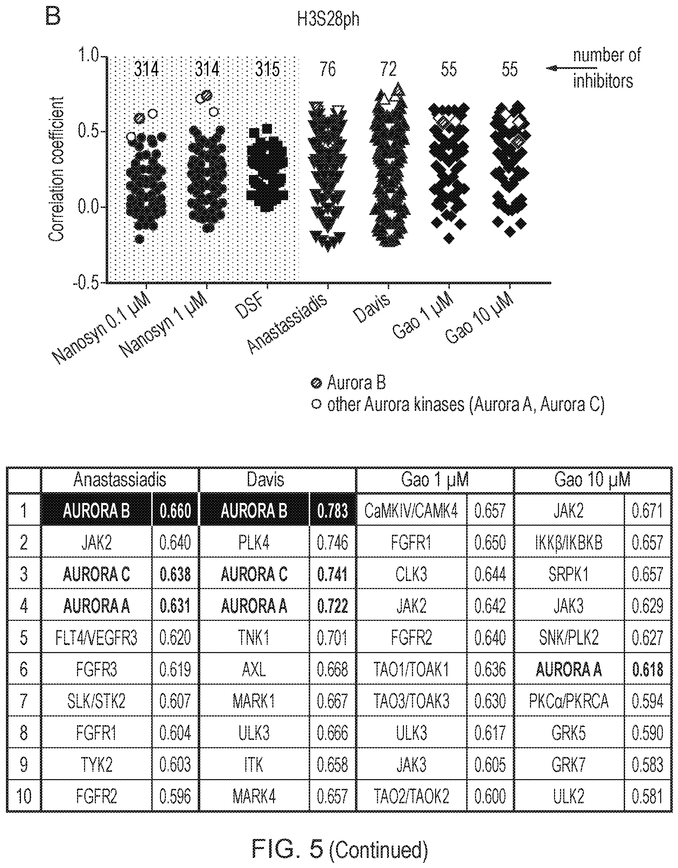

**Figure 5****.** Results of KiPlK screens using the "custom" inhibitor library. **A.** KiPlK screen for H3T3ph. Scatterplots show the Pearson correlation coefficients of the %inhibition results from the KiPlK screen against the % inhibition results for each of the kinases in the Anastassiadis, Davis and Gao (1 and 10 µM) profiling datasets. Results from the PKIS1 screen using Nanosyn and DSF datasets are shown for comparison (shaded grey). The number of inhibitors included in each dataset is indicated above the plot. The table below shows the top 10 hits from each profiling dataset with corresponding correlation coefficients. The expected kinase (Haspin/GSG2) is shown in inverted bold type. **B.** KiPlK screen for H3S28ph. Scatterplots and the table below are as indicated in (A). Expected kinase(s) are shown in inverted bold type, and closely related kinases in bold.

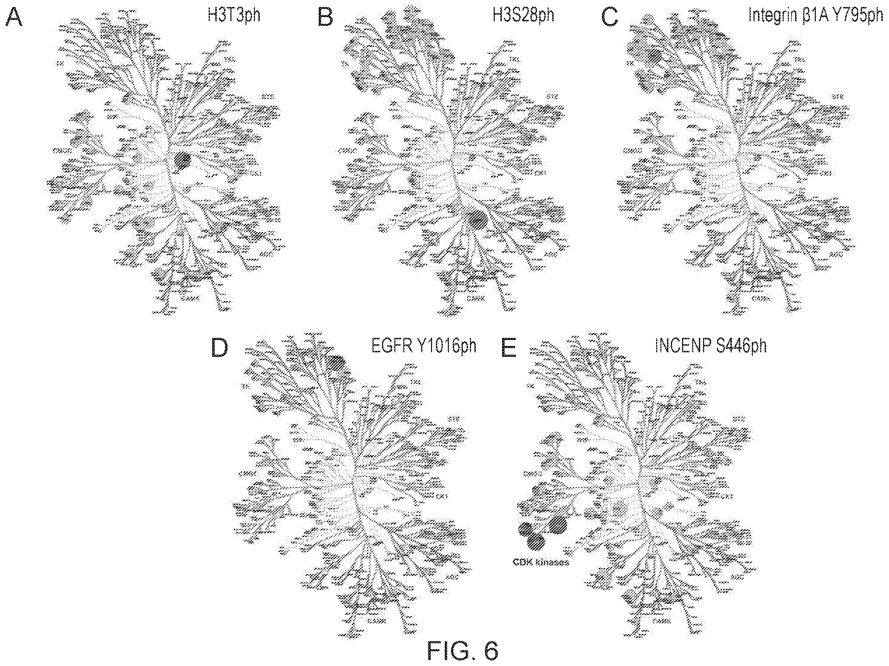

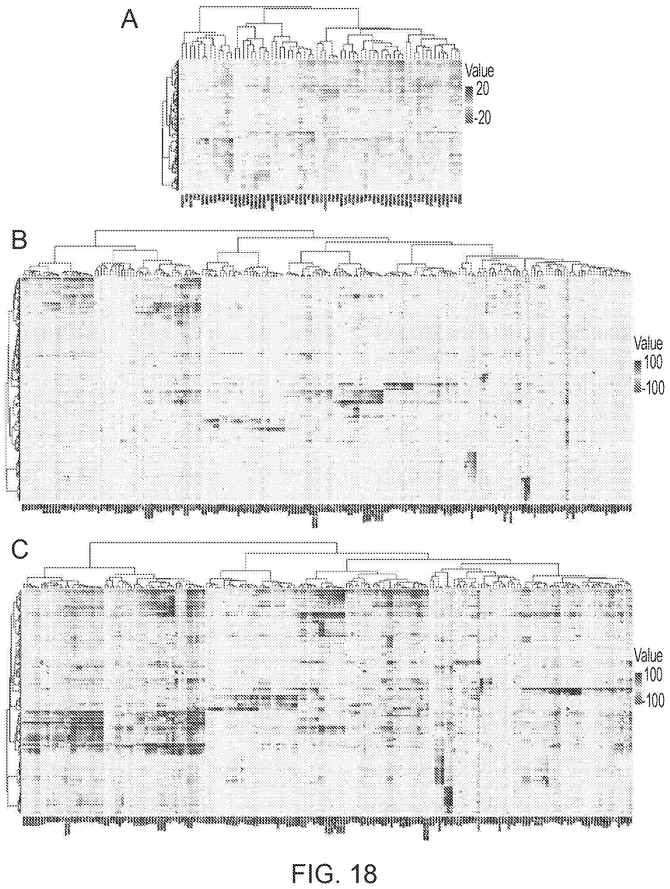

**Figure 6****.** Correlation coefficients from KiPlK screens using the PKIS1 library displayed on sequence-based human kinome dendrograms. **A.** H3T3ph results from the DSF dataset. **B.** H3S28ph results from the Nanosyn 1 µM dataset. **C.** Integrin β1A Y795ph results from the Nanosyn 1 µM dataset. **D.** EGFR Y1016ph results from the Nanosyn 1 µM dataset. **E.** INCENP S446ph results from the DSF dataset. Circle sizes indicate correlation coefficients (from no circle at r = 0 to the largest circle at r = 1). For A-D, the expected kinase, and overall top hit in each screen, is shown as dark circles. For E, strong hits on the CDK kinase branch are shown in darker circles. Trees for all results can be found in Figures 18 to 24.

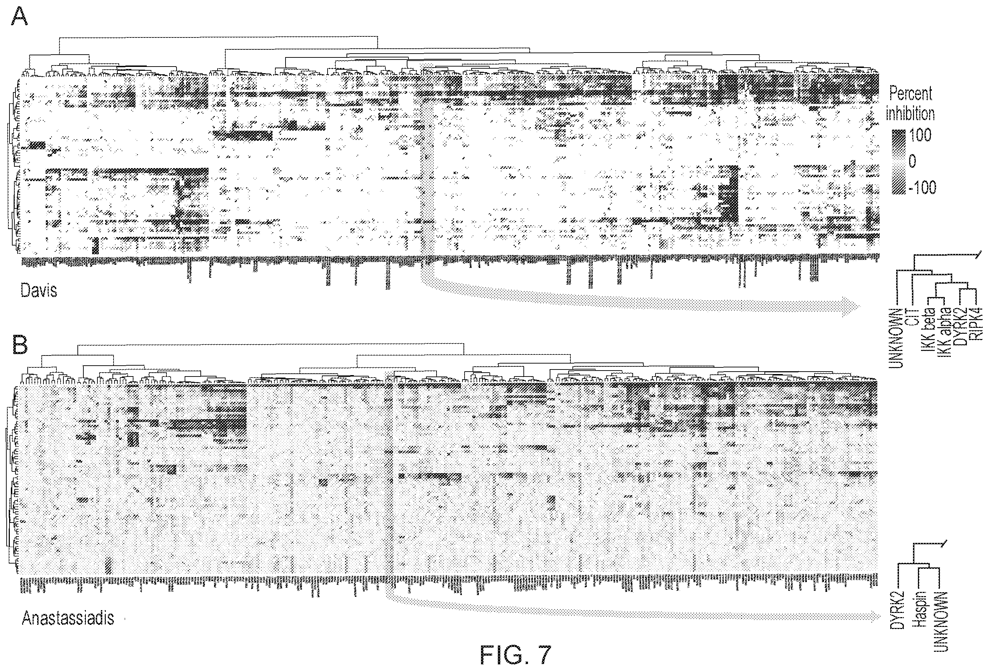

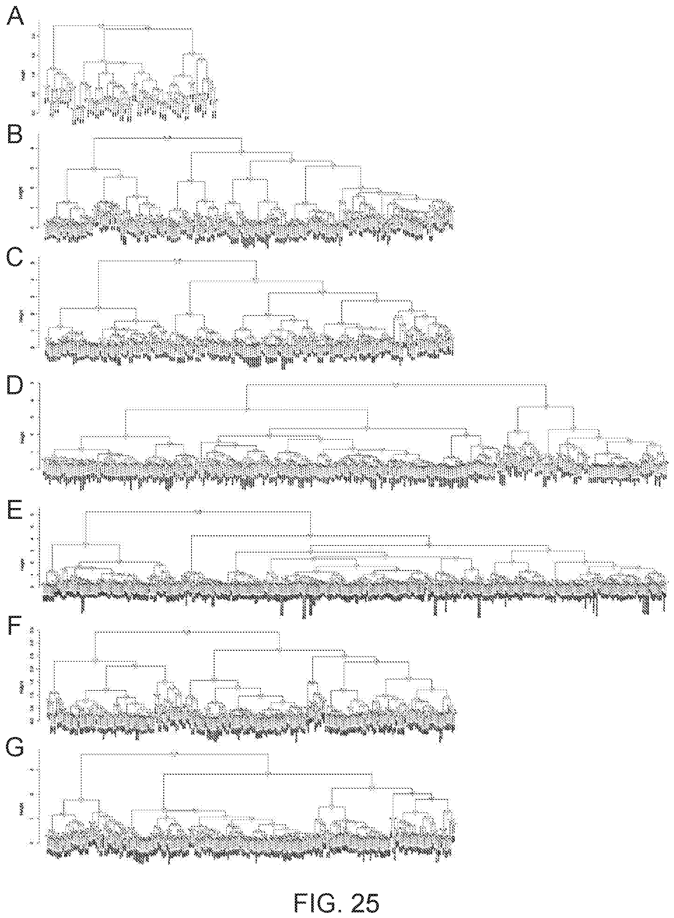

**Figure 7****.** Kinase dendrograms constructed from inhibition profiling data and KiPlK results for the H3T3ph kinase. Pearson correlation coefficients calculated from pairwise comparisons of inhibition fingerprints were used to produce dendrograms for kinases (top) and kinase inhibitors (left side). Heat maps illustrate the percent inhibition score for each kinase-inhibitor pair. Excerpted boxes show relevant portions of the dendrograms in greater detail. All dendrograms with heat maps are shown in Figures 25 to 31. **A.** Profiling data from Davis *et al.* (2011) which do not contain Haspin/GSG2. **B.** Profiling data from Anastassiadis *et al.* (2011) which do contain Haspin/GSG2.

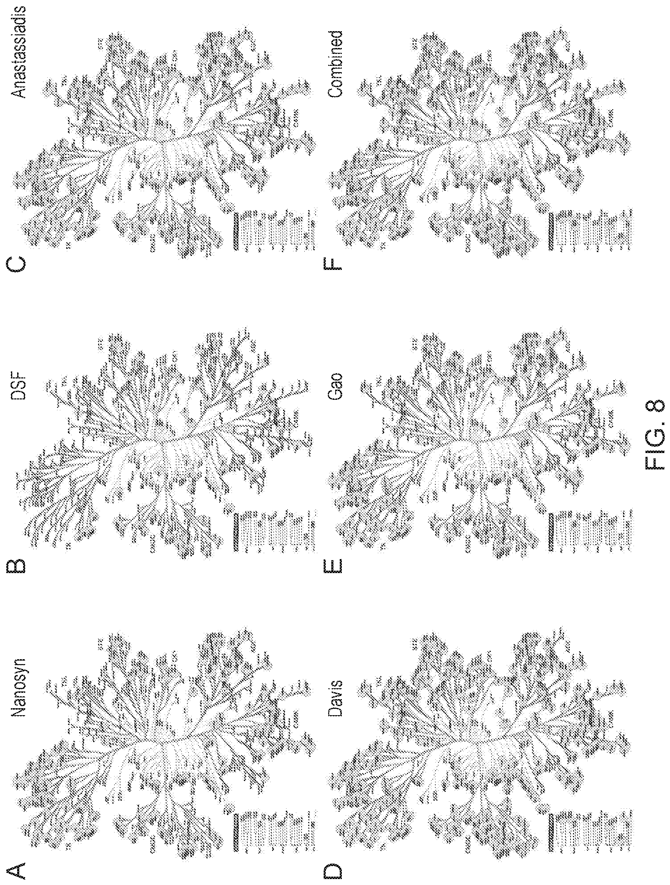

**Figure 8****.** Kinome trees showing all kinases included in the profiling datasets used in this work. A. Nanosyn set. B. DSF set. C. Anastassiadis set. D. Davis set. E. Gao set. F. All sets combined. Circles indicate kinases included in the set.

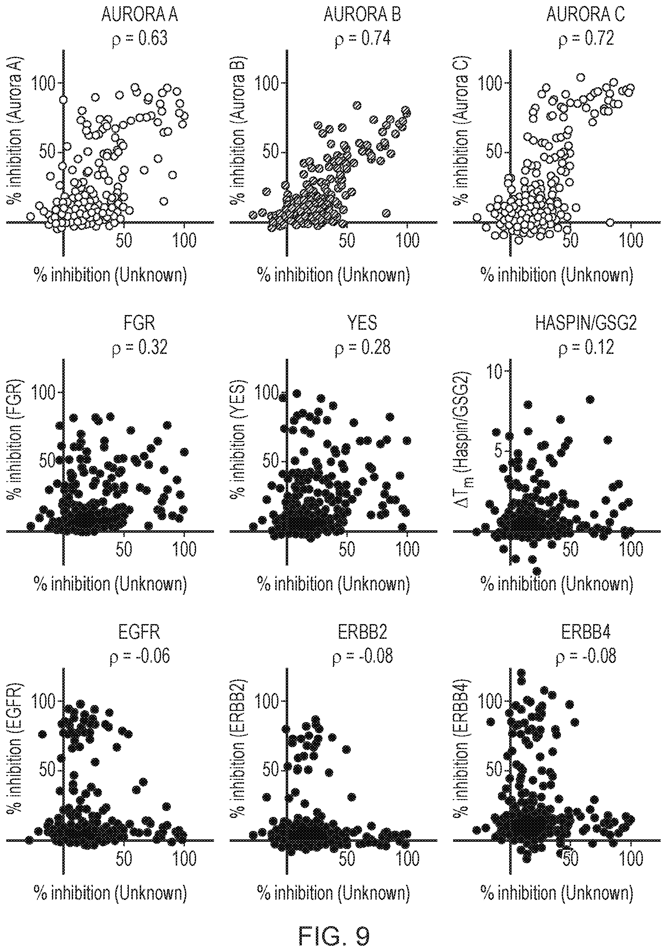

**Figure 9****.** Selected kinase inhibition correlation plots for the H3S28ph kinase screen using the PKIS1 inhibitor set. KiPlK results obtained from the H3S28ph kinase screen were compared to profiling data from Elkins *et al.* (Nanosyn 1 µM for Aurora A, Aurora B, Aurora C, FGR, YES, EGFR, ERBB2 and ERBB4; DSF for Haspin/GSG2) (Elkins et al., 2016). Profiling data are on the y axis; KiPlK (unknown) data on the x axis. Pearson correlation coefficients (r) are shown. The best correlation screen-wide is Aurora B, and data for other closely related kinases are shown in pale dots.

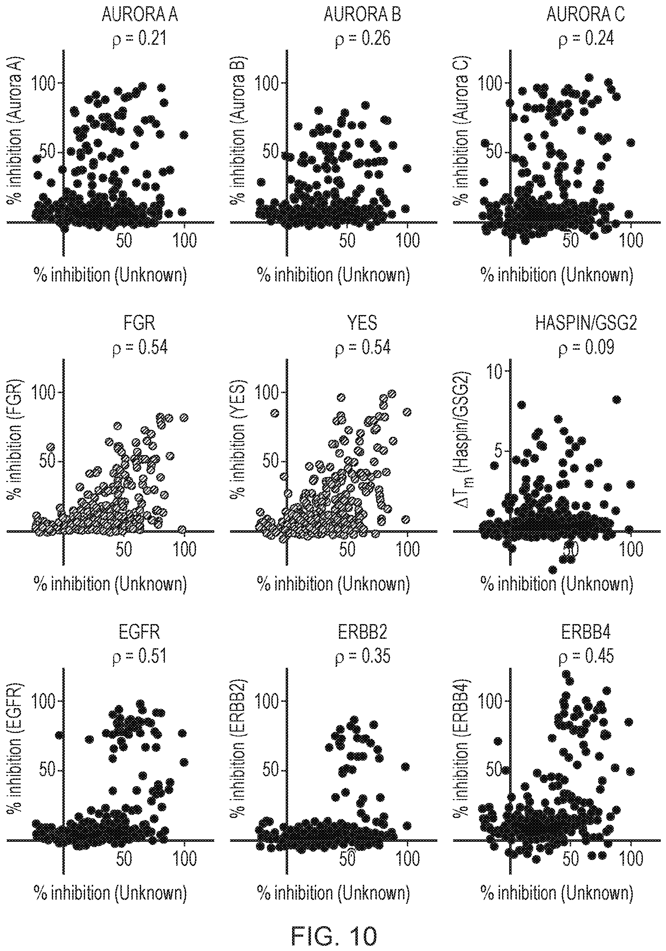

**Figure 10****.** Selected kinase inhibition correlation plots for the Integrin β1A Y795ph kinase screen using the PKIS1 inhibitor set. KiPlK results obtained from the Integrin β1A Y795ph kinase screen were compared to profiling data from Elkins *et al.* (Nanosyn 1 µM for Aurora A, Aurora B, Aurora C, FGR, YES, EGFR, ERBB2 and ERBB4; DSF for Haspin/GSG2) (Elkins et al., 2016). Profiling data are on the y axis; KiPlK (unknown) data on the x axis. Pearson correlation coefficients (r) are shown. The best correlations screen-wide are the Src family kinases FGR and YES.

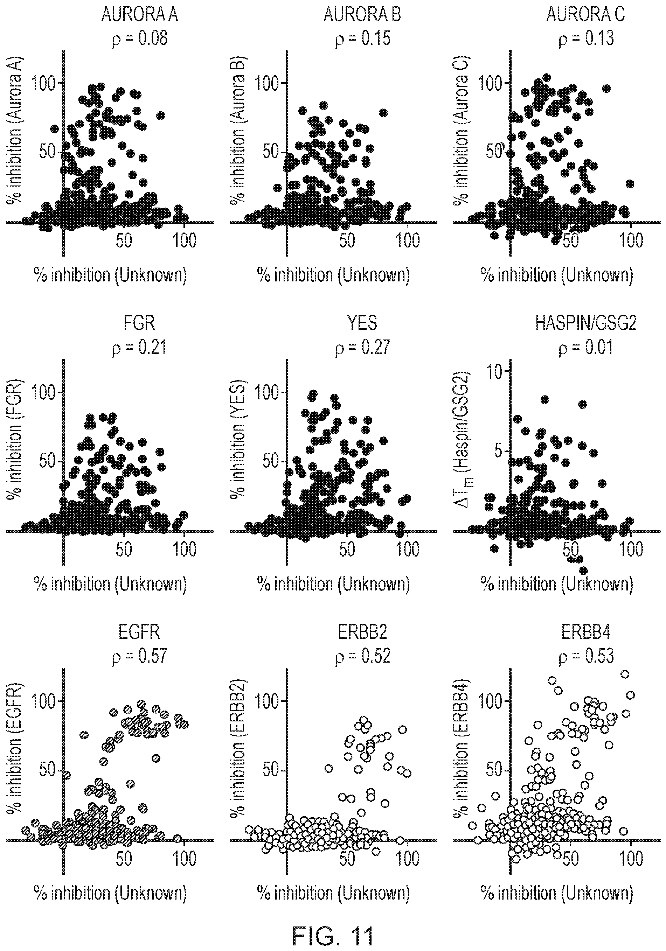

**Figure 11****.** Selected kinase inhibition correlation plots for the EGFR Y1016ph kinase screen using the PKIS1 inhibitor set. KiPlK results obtained from the EGFR Y1016ph kinase screen were compared to profiling data from Elkins *et al.* (Nanosyn 1 µM for Aurora A, Aurora B, Aurora C, FGR, YES, EGFR, ERBB2 and ERBB4; DSF for Haspin/GSG2) (Elkins et al., 2016). Profiling data are on the y axis; KiPlK (unknown) data on the x axis. Pearson correlation coefficients (r) are shown. The best correlation screen-wide is EGFR, and data for other closely related kinases are shown in pale dots (ERBB2 and ERRB4).

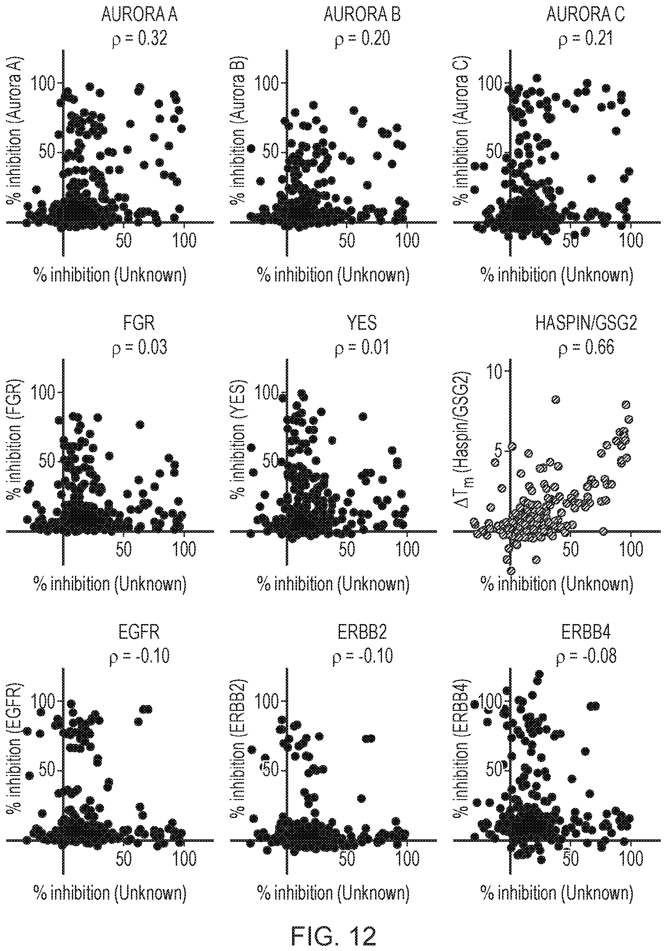

**Figure 12****.** Selected kinase inhibition correlation plots for the H3T3ph kinase screen using the PKIS1 inhibitor set. KiPlK results obtained from the H3T3ph kinase screen were compared to profiling data from Elkins *et al.* (Nanosyn 1 µM for Aurora A, Aurora B, Aurora C, FGR, YES, EGFR, ERBB2 and ERBB4; DSF for Haspin/GSG2) (Elkins et al., 2016). Profiling data are on the y axis; KiPlK (unknown) data on the x axis. Pearson correlation coefficients (r) are shown. The best correlation screen-wide is Haspin/GSG2.

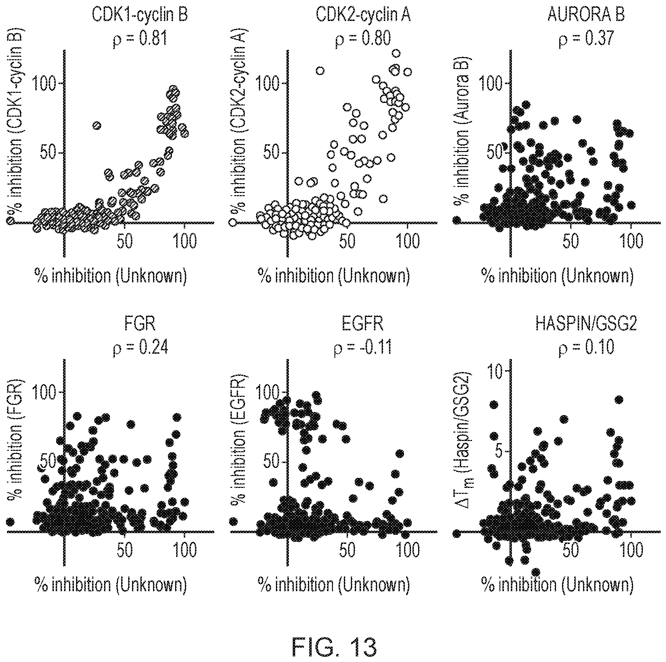

**Figure 13****.** Selected kinase inhibition correlation plots for the INCENP S446ph kinase screen using the PKIS1 inhibitor set. KiPlK results obtained from the INCENP S446ph kinase screen were compared to profiling data from Elkins *et al.* (Nanosyn 1 µM for Aurora A, Aurora B, Aurora C, FGR, YES, EGFR, ERBB2 and ERBB4; DSF for Haspin/GSG2) (Elkins et al., 2016). Profiling data are on the y axis; KiPlK (unknown) data on the x axis. Pearson correlation coefficients (r) are shown. The best correlation screen-wide is CDK1-cyclin B and data for the closest related kinase (CDK2-cyclin A) are shown as pale dots.

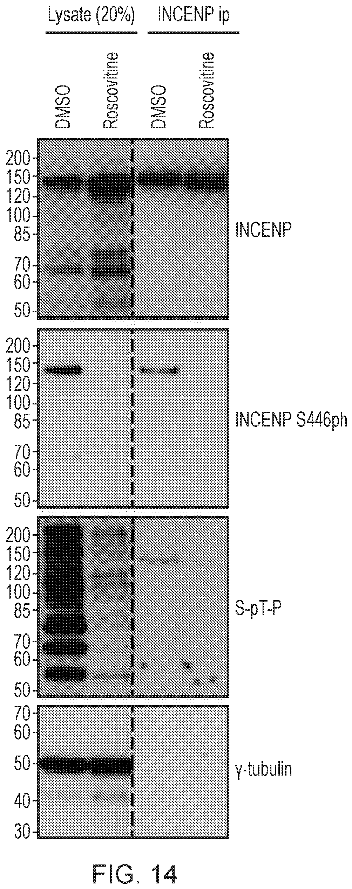

**Figure 14****.** INCENP S446ph in cells requires Cyclin-dependent kinase activity. INCENP was immunoprecipitated from nocodazole-arrested mitotic HeLa 1C8 cell lysates (Dai et al., 2005) that were treated with or without the Cdk inhibitor Roscovitine for 2 h. After SDS-PAGE, immunoblotting with antibodies to INCENP, INCENP S446ph, the S-pT-P motif (which detects a subset of Cdk phosphorylation sites), or γ-tubulin was carried out.

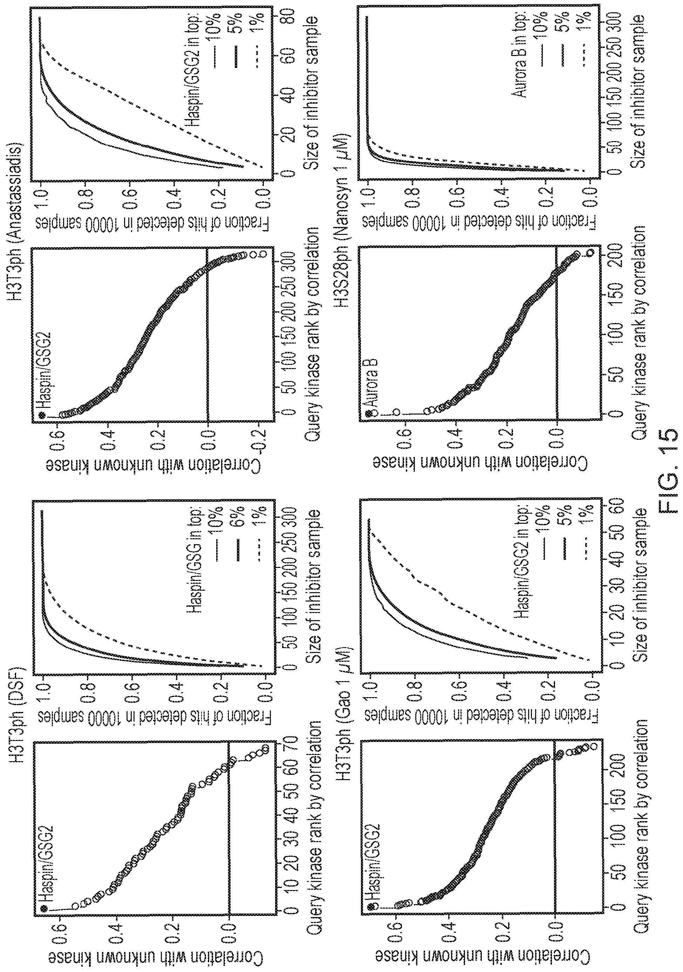

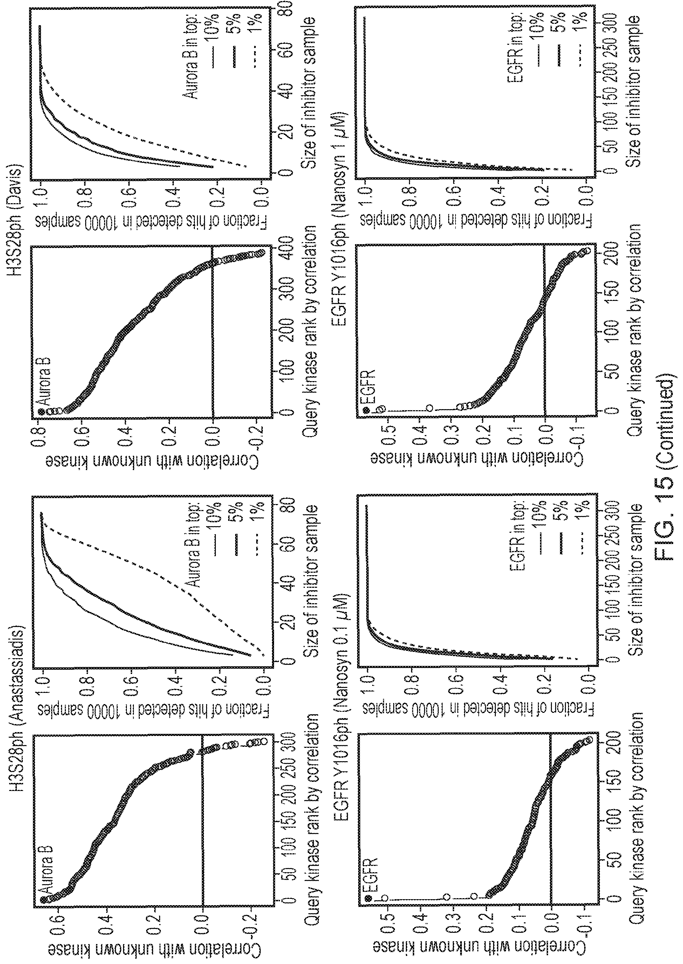

**Figure 15****.** Downsampling reveals the effect of inhibitor library size on the robustness of KiPIK. The righthand plot of each pair shows the fraction of times that the expected kinase was found to be in the top 1%, 5% or 10% of all kinases in the profiling dataset, as a function of inhibitor library size. This allows results from different datasets (which have different numbers of kinases) to be compared. Lefthand plots show the ranked correlation scores for the unknown kinase with each query kinase in the library.

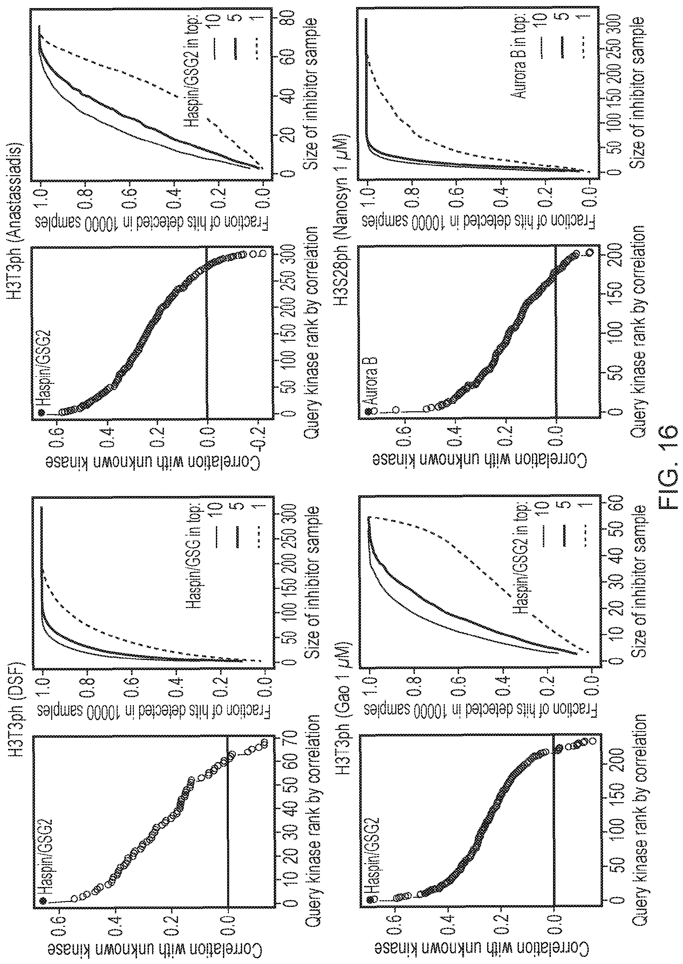

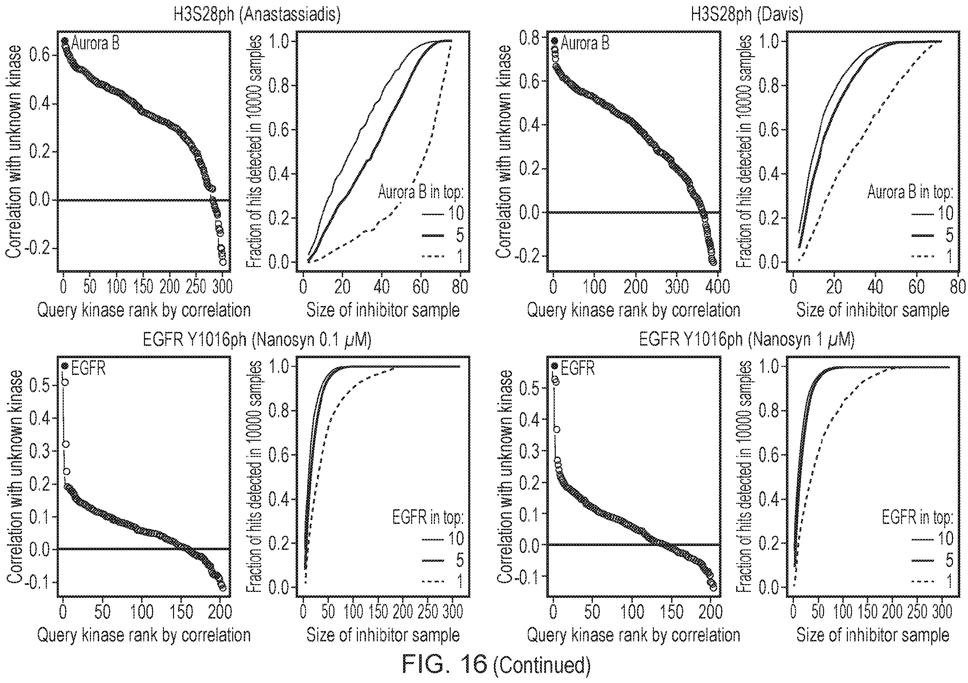

**Figure 16****.** Downsampling reveals the effect of inhibitor library size on the robustness of KiPIK. The righthand plot of each pair shows the fraction of times that the expected kinase was found to be ranked in the top 1, 5 or 10 of all kinases in the profiling dataset, as a function of inhibitor library size. This does not take into account of the fact that different datasets have different numbers of kinases. Lefthand plots show the ranked correlation scores for the expected kinase with each query kinase in the library

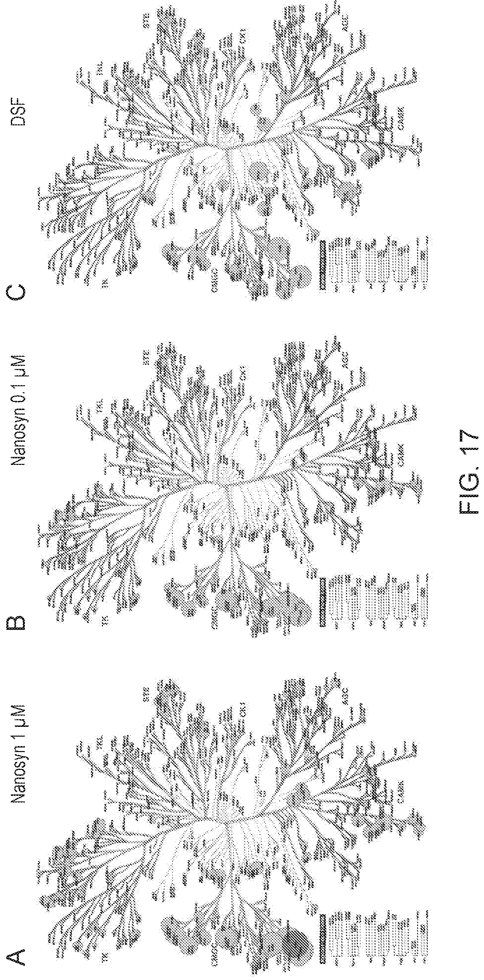

**Figure 17****.** Kinome trees showing KiPlK correlation coefficients for the INCENP S446ph screen with the PKIS1 inhibitor library. **A.** Nanosyn screen at 1 µM inhibitor concentration. **B.** Nanosyn screen at 0.1 µM inhibitor concentration. C. DSF screen. Circle sizes indicate KiPIK correlation coefficients (from no circle at r = 0 to the largest circle at r = 1.0). The highest scoring kinase overall, CDK1-Cyclin B, is indicated by the dark circle.
**Figure 18****.** Kinase dendrograms constructed from inhibition profiling data and KiPlK results for the H3T3ph kinase. **A.** DSF dataset. Note that, for the DSF tree only, the KiPlK data are shown as %inhibition/10 so that they are on a similar scale. **B.** Nanosyn 0.1 µM dataset. **C.** Nanosyn 1 µM dataset.

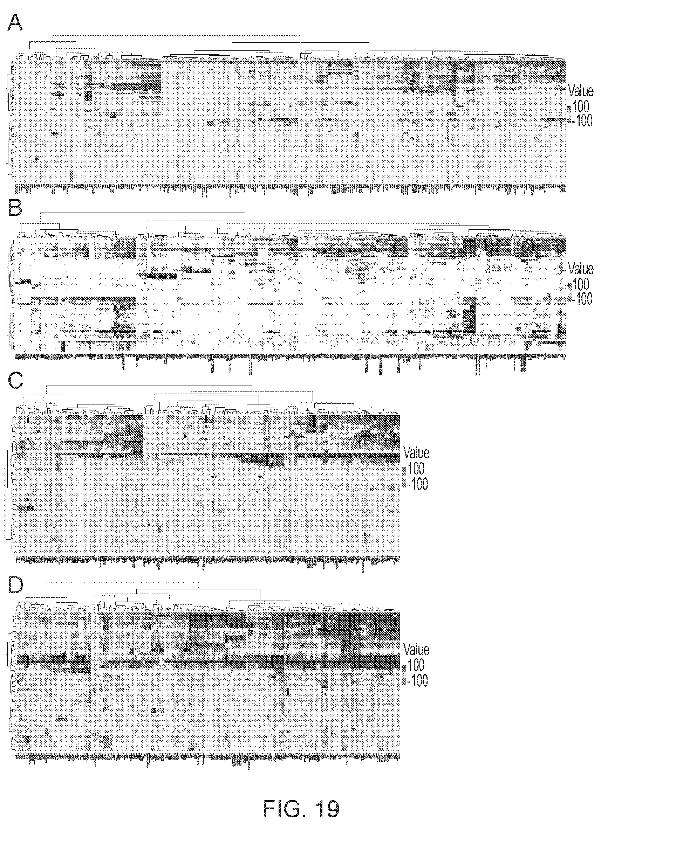

**Figure 19****.** Kinase dendrograms constructed from inhibition profiling data and KiPlK results for the H3T3ph kinase. **A.** Anastassiadis dataset. **B.** Davis dataset. **C.** Gao 1 µM dataset. **D.** Gao 10 µM dataset.

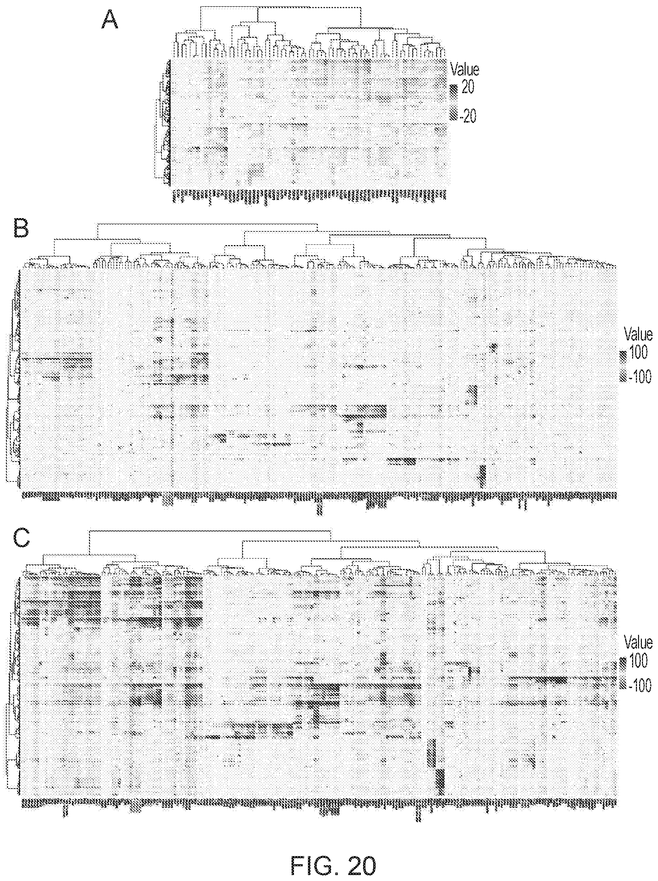

**Figure 20****.** Kinase dendrograms constructed from inhibition profiling data and KiPlK results for the H3S28ph kinase. **A.** DSF dataset. Note that, for the DSF tree only, the KiPlK data are shown as %inhibition/10 so that they are on a similar scale. **B.** Nanosyn 0.1 µM dataset. **C.** Nanosyn 1 µM dataset.

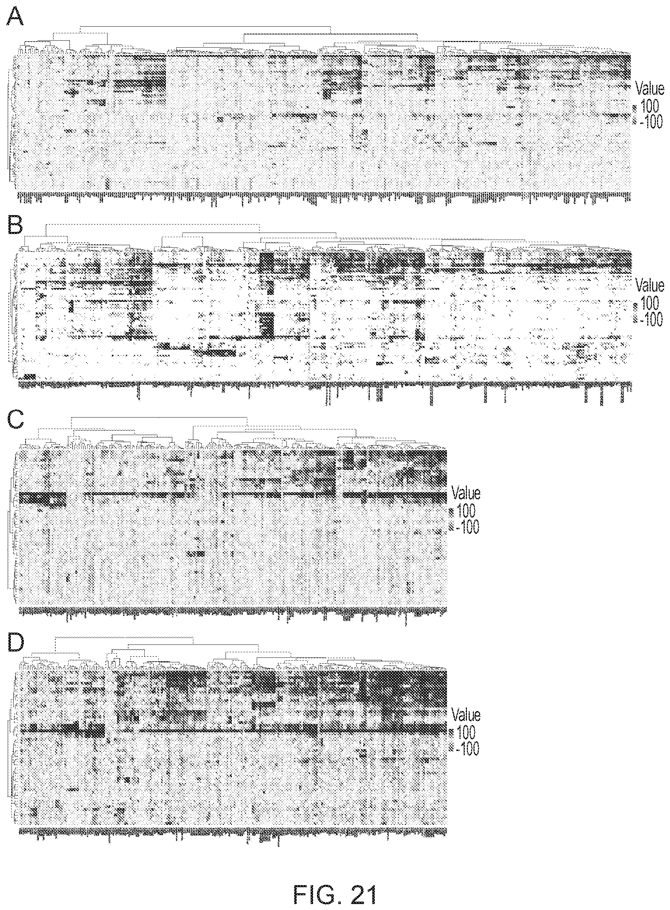

**Figure 21****.** Kinase dendrograms constructed from inhibition profiling data and KiPlK results for the H3S28ph kinase. **A.** Anastassiadis dataset. **B.** Davis dataset. **C.** Gao 1 µM dataset. **D.** Gao 10 µM dataset.

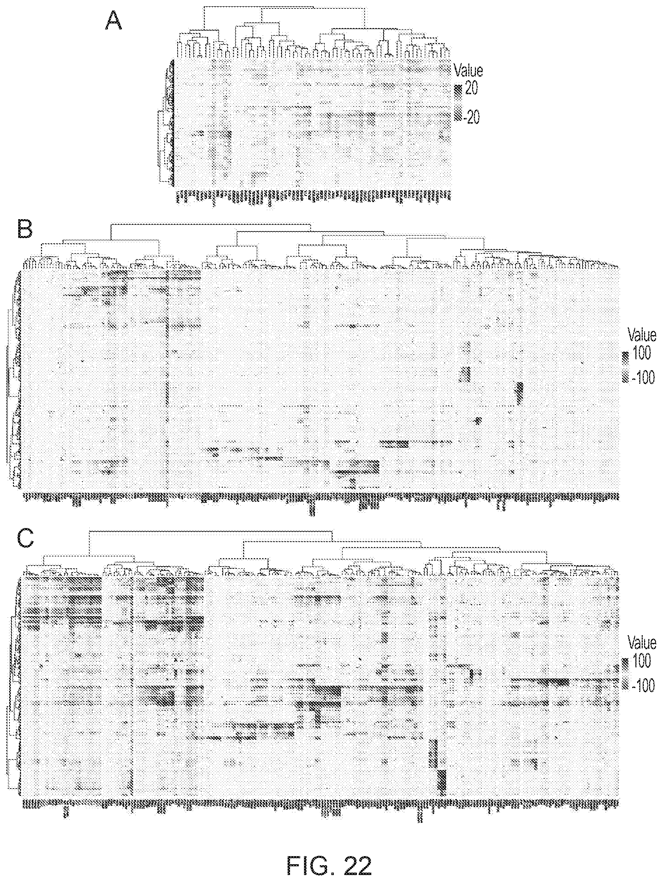

**Figure 22****.** Kinase dendrograms constructed from inhibition profiling data and KiPlK results for the Integrin β1A Y795ph kinase. **A.** DSF dataset. Note that, for the DSF tree only, the KiPlK data are shown as %inhibition/10 so that they are on a similar scale. **B.** Nanosyn 0.1 µM dataset. **C.** Nanosyn 1 µM dataset.

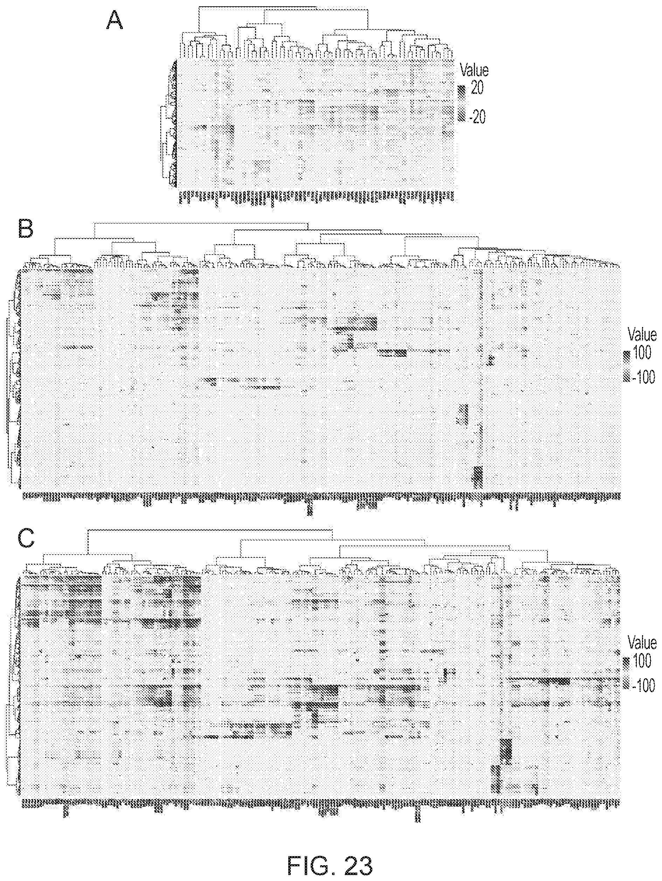

**Figure 23****.** Kinase dendrograms constructed from inhibition profiling data and KiPlKresults for the EGFR Y1016ph kinase.**A.** DSF dataset. Note that, for the DSF tree only, the KiPIK data are shown as %inhibition/10 so that they are on a similar scale. **B.** Nanosyn 0.1 µM dataset. **C.** Nanosyn 1 µM dataset.

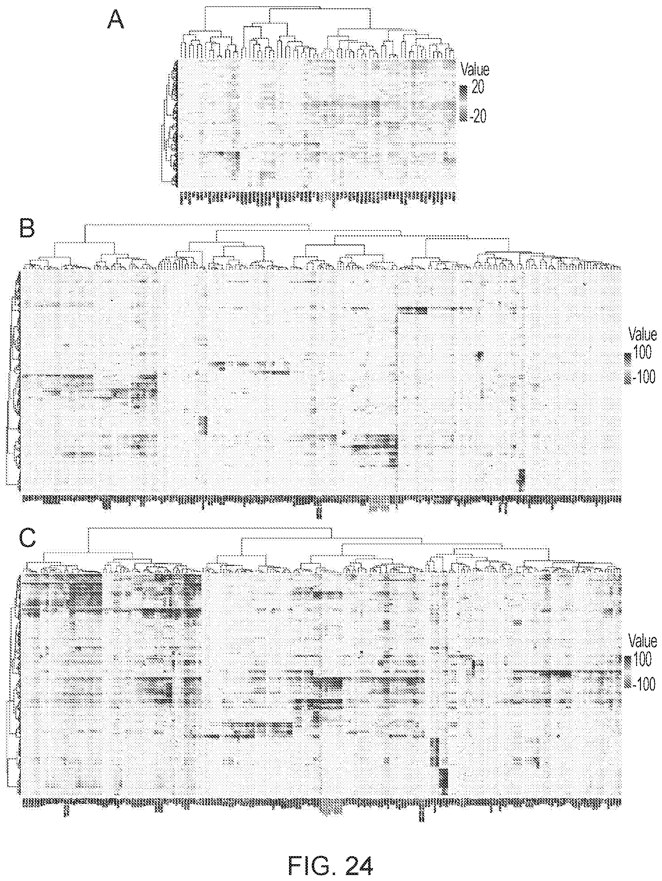

**Figure 24****.** Kinase dendrograms constructed from inhibition profiling data and KiPlK results for the INCENP S446ph kinase. **A.** DSF dataset. Note that, for the DSF tree only, the KiPIK data are shown as %inhibition/10 so that they are on a similar scale. **B.** Nanosyn 0.1 µM dataset. **C.** Nanosyn 1 µM dataset.
**Figure 25****.** Kinase dendrograms constructed from inhibition profiling data and KiPlK results for the H3T3ph kinase, with bootstrapping (10,000x). **A.** DSF. **B.** Nanosyn 0.1 µM. C. Nanosyn 1 µM. **D.** Anastassiadis. **E.** Davis. **F.** Gao 1 µM. G. Gao 10 µM. AU values are to the top left, BP values to the top right, and edge numbers beneath each branch point.

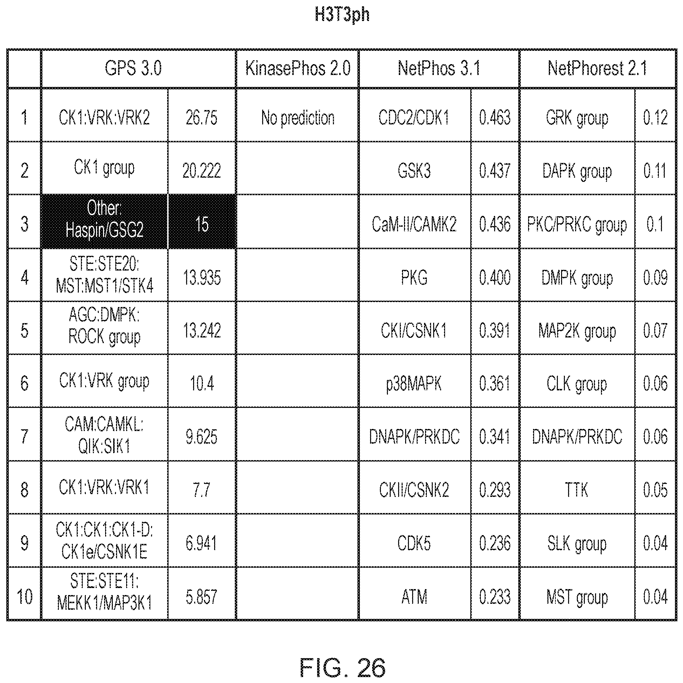

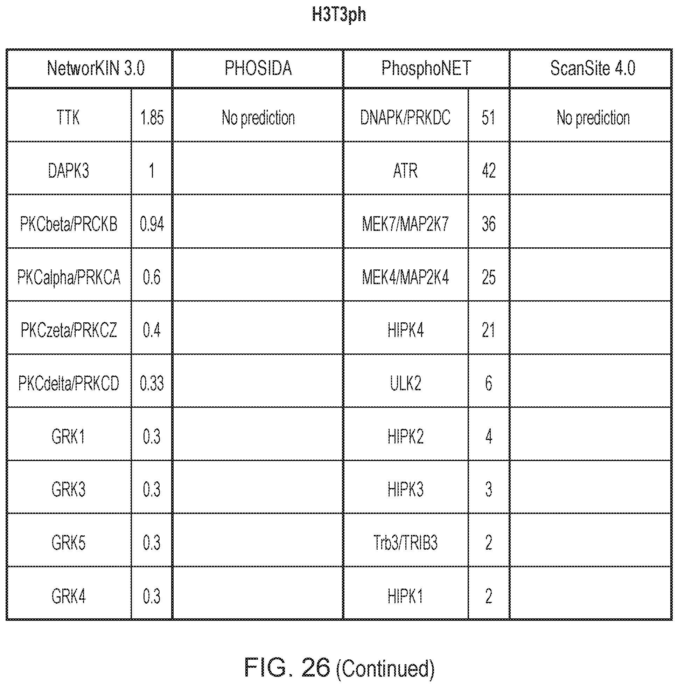

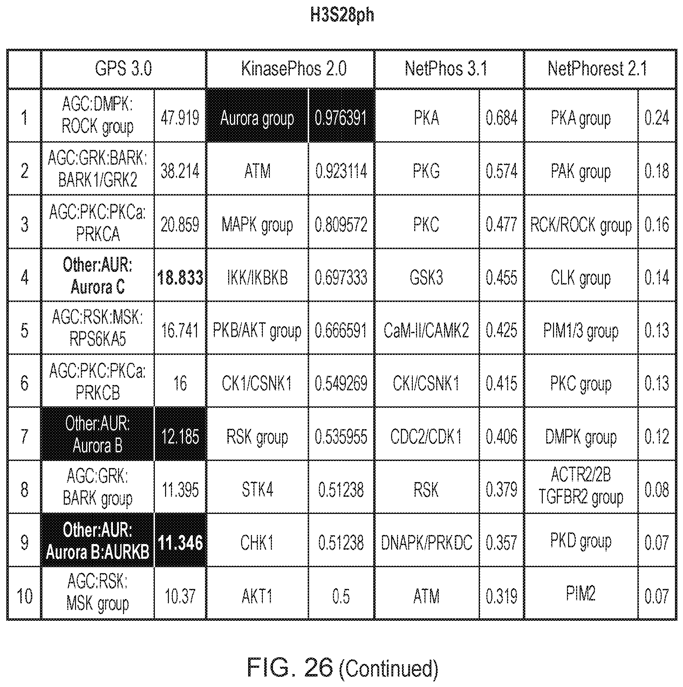

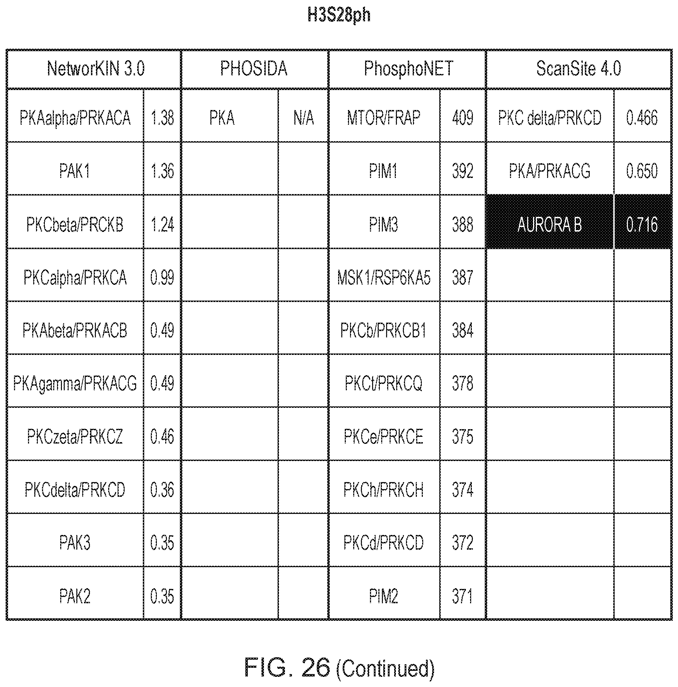

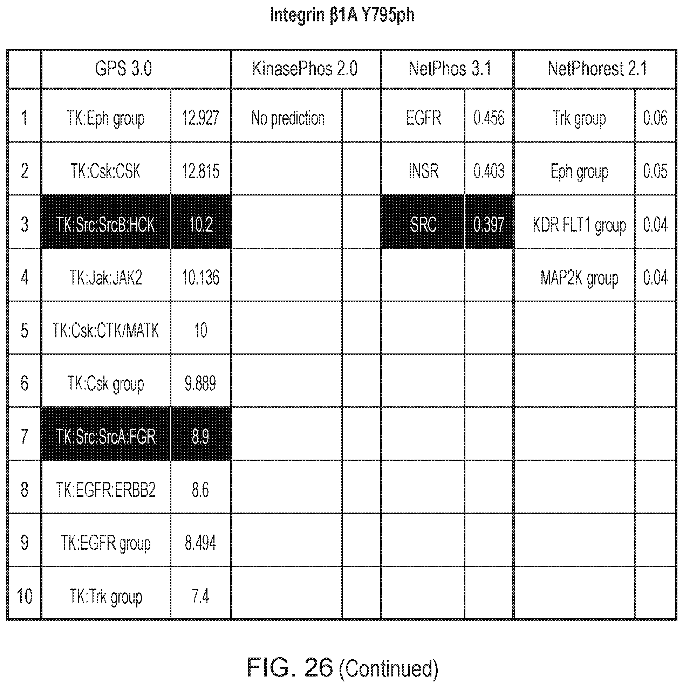

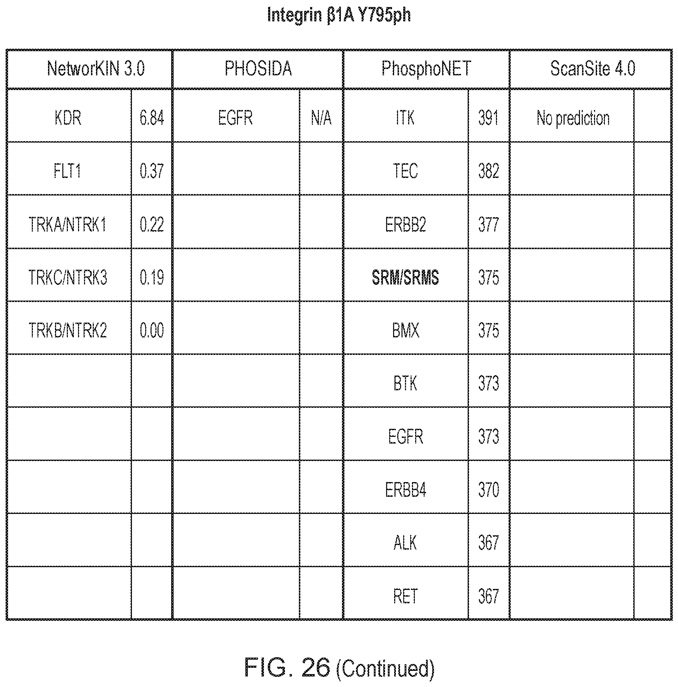

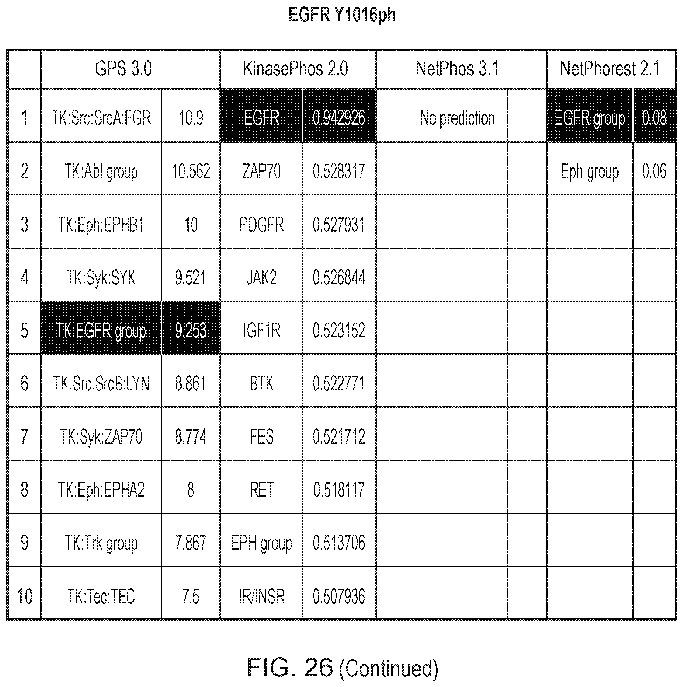

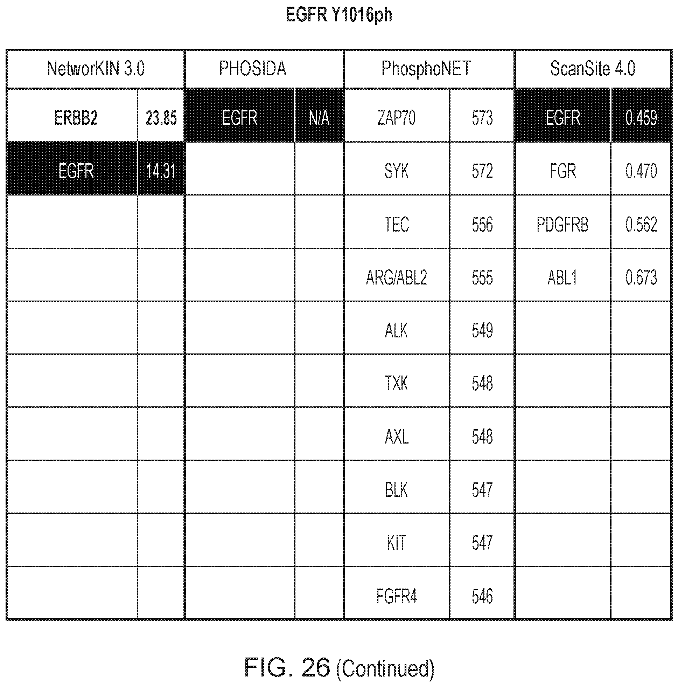

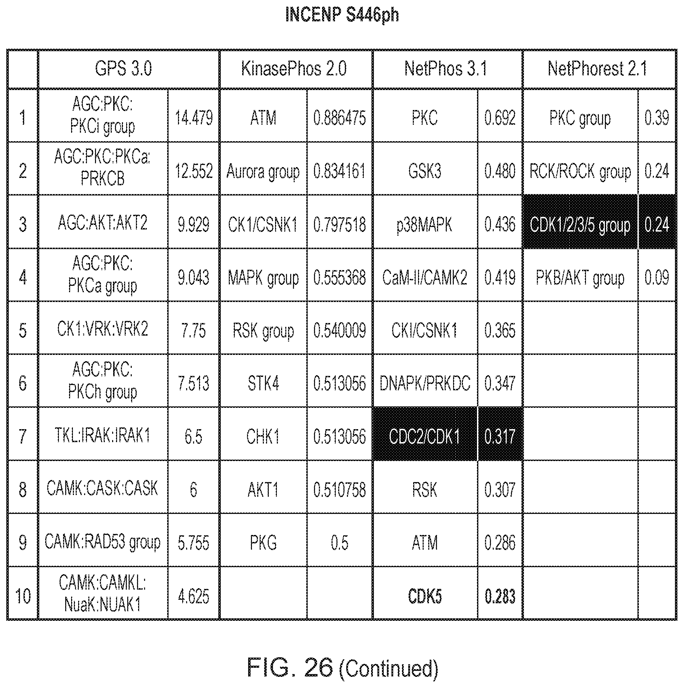

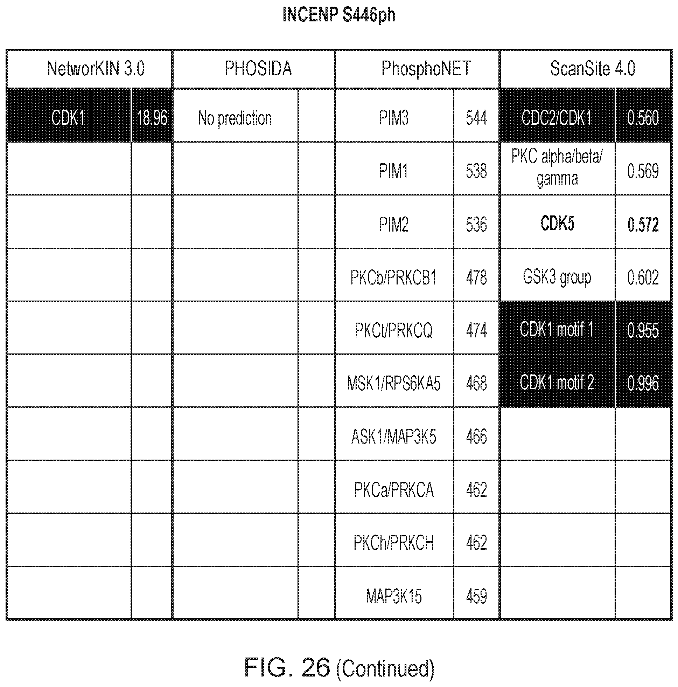

**Figure 26****.** Predicted kinases for specific phosphorylation sites using in silico approaches.

Note that for all Figures showing kinome trees, kinases are named according to their HGNC gene names. Sequence-based kinome trees were produced using KinMap (Eid et al., 2017), and the underlying tree illustration is reproduced courtesy of Cell Signaling Technology, Inc. (www.cellsignal.com).

### Detailed description

The terms "KiPIK", KiPIK method" and "methods of the invention", are used interchangeably herein.

A simple and rapid method for identifying a kinase responsible for a specific phosphorylation event is provided herein. The inventors have shown that it canidentify the previously determined kinase for all examples tested, including serine/threonine kinases that act on histones in mitosis, and both receptor and non-receptor tyrosine kinases.

The inventors also tested the method on an "orphan" phosphorylation site, making use of a residue in the Chromosomal Passenger Complex protein INCENP (S446) that sits within a consensus motif commonly phosphorylated in mitosis by unknown kinase(s) (Dephoure et al., 2008; Hegemann et al., 2011). KiPIK unambiguously identified Cyclin-dependent kinases as candidates, and Cyclin B1-Cdk1 was the top hit. Follow up studies supported the view that this kinase is responsible for INCENP S446ph in cells. Together, these results suggested that the P-X-S-X-X-[K/R] motif is a common non-canonical target for Cdk1 action in mitosis, an idea independently supported by findings that such motifs are favourable *in vitro* targets for Cyclin B-Cdk1 (Suzuki et al., 2015).

### Overview and Rationale for the KiPIK method

KiPIK screening uses two key principles to allow identification of direct kinases for particular phosphorylation sites. First, it makes use of detailed selectivity profiles of small molecule kinase inhibitors obtained using large panels of recombinant kinases *in vitro* (Anastassiadis et al., 2011; Gao et al., 2013; Davis et al., 2011; Elkins et al., 2016). Together these provide unique inhibition patterns, or "fingerprints", for approximately 80% of human protein kinases. Second, it utilizes the rapid action of kinase inhibitors in cell lysates or cell extracts to focus on defined biological states and to minimize indirect effects. Advantageously, inhibition profiles of kinase inhibitors in cell lysates or cell extracts are generally similar to those established *in vitro* (Bantscheff et al., 2007; Sharma et al., 2009; Patricelli et al., 2011). In addition, kinase reactions in cell lysates or cell extracts preserve physiological kinase-phosphosite dependencies. Furthermore, cell lysates or extracts retain the ability to carry out complex processes such as steps of the cell cycle (Pomerening et al., 2003; Deibler and Kirschner, 2010; Wang et al., 2011).

An example of the KiPIK method described herein is shown in Figure 1. Initially, cell conditions can be identified in which the phosphorylation of a protein residue of interest is robust. Cells at a particular cell cycle stage can then be enriched, or a signaling pathway leading to phosphorylation can be triggered. Cell lysates or cell extracts of these cells may then be prepared in the presence of phosphatase inhibitors to prevent dephosphorylation of active kinases. These cell lysates or cell extracts are then used as the source of all potentially relevant kinases for *in vitro* kinase reactions using the substrate of interest. Multiple such *in vitro* kinase reactions may be carried out in parallel, in the presence of each member of a panel of well-characterized kinase inhibitors. This yields an inhibition fingerprint that characterizes the cellular kinase mainly responsible for the observed phosphorylation. Finally, to identify candidate kinases, this fingerprint can be compared to the known inhibition patterns of all kinases in the available profiling datasets.

### The KiPIK method

An informative KIPIK screen uses a library of kinase inhibitors for which *in vitro* inhibitory information data is available across a range of recombinant kinases. Suitable libraries include the Protein Kinase Inhibitor Set (PKIS1) compiled from GlaxoSmithKline compounds by the Structural Genomics Consortium (Elkins et al., 2016) and a library of inhibitors assembled by Calbiochem (Anastassiadis et al., 2011; Gao et al., 2013). The inhibitory properties of compounds in these libraries have been determined on large kinase panels *in vitro* using radiolabeled ATP incorporation assays (Anastassiadis et al., 2011; Gao et al., 2013), substrate electrophoretic mobility shift assays (Elkins et al., 2016), or inhibitor binding assays (Elkins et al., 2016; Davis et al., 2011). Together, these profiles provide inhibition fingerprints for kinases encoded by 409 of the approximately 535 human protein kinase genes (Manning et al., 2002; Wilson et al., 2018).

A cell lysate or cell extract containing the phosphorylation activity being screened is typically prepared. In principle, any cell or tissue could be used as a source, but herein the inventors have focused on cultured cell lines. Prior to lysis, the cells can be treated to enhance the phosphorylation signal, or to probe phosphorylation dependencies in a particular context. For example, when investigating a mitotic phosphorylation, enriching the mitotic cell population by nocodazole treatment may be beneficial, while EGF stimulation can be used to explore phosphorylation downstream of EGFR. After lysis in the presence of phosphatase inhibitors, lysates or extracts are immediately flash frozen. A number of methods are available to assay substrate phosphorylation in cell lysates or cell extracts and a number of these are likely to be compatible with the KiPlK procedure. As described in detail in the examples section below, the invention has been exemplified using selected substrates comprising short biotinylated peptides encompassing phosphosites of interest as substrates, coupled with the use of phospho-specific antibodies to detect peptide phosphorylation in Enzyme-Linked Immunosorbent Assays (ELISA). However, as would be clear to a person of skill in the art, any suitable selected substrate and method for detecting its phosphorylation may be utilized within the context of the invention. All suitable selected substrates are therefore encompassed within the invention.

The inventors have used microplate robotics as one way to demonstrate that the invention can be performed on many extract kinase reactions in parallel, each in the presence of a single kinase inhibitor from the characterized inhibitor library. Quantification of substrate phosphorylation in the presence of each inhibitor yields the required inhibition fingerprint that is characteristic of the predominant kinase acting on the substrate in the extract. This fingerprint is then compared with the known inhibition patterns previously determined for recombinant kinases challenged with the same set of kinase inhibitors. The kinase(s) with inhibition patterns showing the highest similarity to that of the unknown kinase activity (determined using Pearson's correlation coefficient) are the top "hits" in the screen.

### Advantages of the method

KiPIK has a number of advantages over current methods for identifying kinases for phosphorylation sites. For example, biochemical approaches that exploit substrate-kinase interactions or attempt kinase purification by tracing enzyme activity require large samples and customized methods that are not easily transferable to additional substrates. In contrast, KiPIK is a relatively standardized approach that can be applied to a variety of substrates. Methods such as RNAi and CRISPR/Cas9 (or overexpression) can be used, but they require long timeframes, causing indirect effects and limiting analysis at different stages of a process such as the cell cycle. These methods also require efficient transfection, and genetic manipulation prohibits simple analysis of the latter stages of a cellular or developmental process if there is a defect in an earlier step. The small molecule inhibitors used in KiPlK, in contrast, act in minutes and can be used to determine the effect of kinase inhibition on a particular biological state without affecting a preceding step. Because the method utilizes extracts of cells harvested in a state where signaling pathways are already active and the presence of phosphatase inhibitors minimizes the requirement for the ongoing activity of upstream kinases, the method is biased towards identifying the direct kinases for particular substrate residues. This allows, for example, the successful identification of Cyclin B1-Cdk1 as a direct kinase for INCENP S446 kinase, even though loss of Cdk1 prevents cells from entering mitosis (Moffat et al., 2006), and allows the direct and indirect contributions of Haspin and Aurora B to H3T3ph to be uncoupled (see examples section below). Inhibitors also allow analysis of a broad range of cells and tissues which may be difficult to transfect. In addition, numerous kinases have kinase-activity-independent functions, and inhibitors provide a powerful way to focus on the function of kinase activity. In practice, however, many kinase inhibitors are poorly selective, and this significantly hampers their use in conventional screening approaches. In KiPIK screening, however, the inventors harness knowledge of this incomplete selectivity to enhance the information content of inhibitor screens to identify candidate kinases for particular phosphorylation sites.

KiPlK has a number of theoretical advantages over *in silico* approaches. In particular, it utilizes cell lysates or cell extracts and so provides biological context that is absent from most prediction methods. For KiPIK, cells can be treated prior to lysate or extract preparation to ensure that the relevant kinase and its regulatory subunit(s) are present and active, and over 400 human kinases are currently covered by the method. Kinases that are not expressed in the relevant cell are excluded, and only kinases that efficiently phosphorylate the substrate in competition with other kinases and substrates in the extract will be identified. In contrast, the majority of computational methods are limited in the number of kinases for which they can make predictions, and they do not account for factors such as the activation state or expression level of the kinase (Linding et al., 2007). Indeed, compared with a spectrum of *in silico* prediction methods, including those that attempt to integrate information on biological context, KiPlK was clearly the most reliable approach for identifying the kinases for the five substrates examined here (see Figure 26).

### Alternative approaches to analyse correlation data

In cases where the target kinase is present in the profiling dataset, the inventors have shown that KiPIK is able to identify the correct kinase or kinase sub-family in the five cases tested. However, because only 80% of known protein kinases are covered by the profiling data it is possible, for some screens, that the unknown kinase is not present in the profiling data. Nevertheless, the top hits are still likely to share properties with the unknown kinase, and therefore can help prioritize candidate kinases for further study. As the inventors have shown in the Examples section, a simple ranking of correlation coefficients readily identifies relevant candidates. The inventors have also explored two additional approaches to enhance this analysis.

First, the well-known sequence-based kinome dendrogram of Manning *et al.* (Manning et al., 2002) can be annotated with correlation coefficients derived from KiPlK screening (for example, see Figure 6). This allows kinases that are closely related in sequence to the top hits in the screen to be easily located, and sub-families of kinases for further testing can then be identified. For example, the DSF profiling set does not contain the kinase Cdk1, but strong hits on the cyclin-dependent kinase (CDK) sub-branch of the CMGC kinase family clearly highlight the CDK sub-family as a source of excellent candidates for the INCENP S446ph kinase (Figure 6E, Figure 17).

Second, hierarchical clustering of all kinases based on the similarity of their inhibition fingerprints can be carried out (using Pearson correlation coefficients). This generates kinome trees in which kinases are clustered more closely if they are inhibited by similar sets of inhibitors (eg Figures 7 and 18 to 24). As previously described, such inhibition-based trees have general similarities to sequence-based trees, but also notable differences, such as separating the p38α and p38β kinases from the closely-related p38δ and p38γ kinases (Vieth et al., 2004; Bamborough et al., 2008). A caveat of this approach is that, although bootstrapping gives an indication of the reliability of the branching pattern (eg Figure 25), a kinase can only be in one place in a tree, so alternative possible kinase clusters are obscured. Nevertheless, if the inhibition fingerprint of the unknown kinase is included in the clustering process, then kinases that have similar inhibition profiles, but may be distant in sequence relatedness, can be identified. For example, Haspin is not present in the Davis profiling set, but the inhibition fingerprint of H3T3ph kinase activity clusters in a group that contains DYRK2 (Figure 7A). This kinase is a common off-target of Haspin inhibitors (Cuny et al., 2010; Cuny et al., 2012; De Antoni et al., 2012) and it clusters close to Haspin in profiling sets which do contain both Haspin and DYRK2 (ie Anastassiadis and Gao 1 µM; Figure 7B, Figure 19). In this way, information derived from the properties of kinases and inhibitors that were not included in the experimental screening process can be exploited to identify kinases for further testing.

### Definitions of terms

A method for identifying or profiling a target cellular kinase that phosphorylates a selected substrate is provided herein. The method uses pre-determined inhibitor profiles from known kinases and compares them to the inhibitor profile generated by a target kinase. The comparison identifies which known kinases have a closely matched inhibitor profile to the target kinase. The method can therefore be used to home in on (i.e. profile) the kinase that is directly responsible for phosphorylating a substrate of interest. Preferably, the method can be used to identify the kinase that is directly responsible for phosphorylating a substrate of interest (i.e. the "target kinase").

As used herein, a "cellular kinase" is a kinase that is present within a cell. The cellular kinase is responsible for phosphorylating a substrate of interest (wherein the substrate of interest is typically also present in the cell i.e. it is a natural substrate for the target kinase in a cell). In the context of the invention, the substrate of interest is known and the cellular kinase that acts directly on the substrate to phosphorylate it is not known/needs validating/confirming. The cellular kinase of interest is therefore discussed herein as a "target cellular kinase" or a "target kinase" (as the method is targeted at identifying it). The terms "kinase" and "cellular kinase" are used interchangeably herein unless the context specifies otherwise.

Kinases catalyze the phosphorylation of proteins, lipids, sugars, nucleosides and other cellular metabolites and play key roles in all aspects of eukaryotic cell physiology. For example, protein kinases and lipid kinases participate in the signaling events which control the activation, growth, differentiation and survival of cells in response to extracellular mediators or stimuli such as growth factors, cytokines or chemokines. In general, protein kinases are classified in two groups, those that preferentially phosphorylate tyrosine residues and those that preferentially phosphorylate serine and/or threonine residues.

As used herein, the term "kinase" encompasses kinases that phosphorylate proteins, lipids, sugars, nucleosides, cellular metabolites etc. It therefore encompasses protein kinases, lipid kinases etc.

The term "protein kinase" is used to refer to an enzyme that catalyzes the transfer of the γ-phosphoryl group of ATP (ATP-Mg²⁺ complex) to the oxygen atom of the hydroxyl group of serine, threonine or tyrosine residues in peptides and polypeptides (substrates). Protein kinases play a crucial role in signal transduction, cellular proliferation, differentiation, and various regulatory mechanisms. About 3% of the total coding sequences within the human genome encode protein kinases. The complement of protein kinases encoded in the human genome comprises approximately 540 family members (kinome) which can be grouped into several subfamilies according to sequence similarity (Review: Manning et al., 2002, The Protein Kinase Complement of the Human Genome, Science 298, 1912-1934; (Wilson et al., 2018); see also Table 1 of PCT WO03/081210 , which includes the type (Ser/Thr or Tyr), SwissProt an NCBI Accession numbers, length, and literature references, and SEQ ID NOs for the amino acid sequence of human protein kinases).

Phosphorylation can occur on serine, threonine and tyrosine side chains (often called 'residues') through phosphoester bond formation, on histidine, lysine and arginine through phosphoramidate bonds, and on aspartic acid and glutamic acid through mixed anhydride linkages.

As used herein, the term "phosphosite" refers to a site (on a substrate) that is modified by phosphorylation. The phosphosite may be present within any suitable substrate e.g. proteins, lipids, sugars, nucleosides, cellular metabolites etc.

The term "tyrosine kinase" is used to refer to an enzyme that catalyzes the transfer of the γ-phosphoryl group from an ATP-Mg2+ complex to the oxygen atom of the hydroxyl group of tyrosine residues in another protein (substrate). The term "serine-threonine kinase" is used to refer to an enzyme that catalyzes the transfer of the γ-phosphoryl group from an ATP-Mg2+ complex to the oxygen atom of the hydroxyl group of serine/threonine residues in another protein (substrate). The term "dual specificity kinase" is used to refer to kinases that have the ability to phosphorylate both tyrosine and serine/threonine residues of targeted protein substrates.

As used herein, a "direct kinase" is the kinase that is directly responsible for phosphorylating the substrate of interest (rather than being part of an upstream signalling pathway that ultimately results in substrate phosphorylation). For example in the context of protein kinases, a direct kinase acts on the substrate itself by catalyzing the transfer of the γ-phosphoryl group of ATP (ATP-Mg²⁺ complex) to the oxygen atom of the hydroxyl group of serine, threonine or tyrosine residues in the substrate. A direct kinase can therefore be considered to be directly responsible for a particular phosphorylation event in respect of the substrate.

As used herein, a "phosphorylation event" refers to a phosphorylation reaction that occurs within a specific context (e.g. at a specific time, under certain conditions). As an example, a kinase may be capable of phosphorylating a substrate, however, it will only be able to do so when the kinase is active and the substrate is available at the same time and place. In the context of a cell, the methods described herein can be used to identify which kinase is responsible for a phosphorylation event of interest (e.g. phosphorylating the substrate of interest when the cell is e.g. at a certain stage of the cell cycle, stimulated by EGF, etc). The methods of the invention therefore allow the user to distinguish between kinases that are merely capable of phosphorylating a substrate of interest and those that are directly responsible for a phosphorylation event in the cellular context applied.

The methods described herein incubate a sample ("protein preparation") containing the target kinase with a "selected substrate". The selected substrate is added to the sample ("protein preparation") during the method. The selected substrate comprises the phosphosite that the target kinase acts on in the cell (i.e. the phosphosite of interest). The selected substrate may comprise the entire substrate of interest (e.g. the entire sequence of the natural substrate of a target kinase in a cell). Alternatively, the selected substrate may only comprise a portion of the substrate of interest, wherein the portion contains the phosphosite that the target kinase acts upon in the cell (i.e. the phosphosite of interest) including surrounding portions of the substrate that are sufficient to allow its recognition by relevant kinases. Selected substrates therefore may be synthetic, recombinant or natural molecules or proteins (e.g. peptides) that comprise the phosphosite of interest, synthetic, recombinant or natural molecules or proteins in complexes with other proteins or co-factors, or synthetic, recombinant, or natural portions of the substrate of interest that retain the phosphosite of interest etc. As stated previously, the substrate of interest may be a protein, lipid, sugar, nucleoside, cellular metabolite etc, for example fructose, glucose, phosphatidylinositol, sphingosine, riboflavin, or thymidine.

As used herein, a phosphorylated substrate is denoted by "ph". For example, "H3S28ph" refers to human histone H3 which has been phosphorylated at serine 28 (i.e. it refers to phosphoserine 28 of human histone H3).

The selected substrate may be any substrate (e.g. a protein, lipid, sugar, nucleoside, cellular metabolite etc) that is capable of being phosphorylated by a target kinase. The selected substrate may also be more than one molecule e.g. a complex of molecules (such as a complex of proteins).

In one example, the selected substrate may comprise a peptide. Advantageously, the peptide may contain a single phosphosite (e.g. a single tyrosine, threonine or serine). In such examples, a generic phospho-specific antibody can be used effectively for KiPlK screening, avoiding the need for phosphosite-specific antibodies.

A selected substrate may be labelled to allow for detection. By "labelled" is meant that the selected substrate is either directly labelled (tagged) or indirectly labelled with a molecule which provides a detection signal, e.g. radioisotope, fluorescent tag, chemiluminescent tag, a peptide or specific binding molecules. Specific binding molecules include pairs, such as biotin and streptavidin, digoxin and antidigoxin. The label can directly or indirectly provide a detectable signal. The label can be a peptide which can be used, for example, in an enzyme fragment complementation assay. Guidance for the selection and methods for the attachment of fluorescent tags (e.g. fluorescein, rhodamine, dansyl, NBD (nitrobenz-2-oxa-1,3-diazole), BODIPY (dipyrromethene boron difluoride), and cyanine (Cy)-dyes) to small molecule ligands are generally known in the art (Vedvik et al., 2004. Assay Drug Dev. Technol. 2(2): 193-203; Zhang et al., 2005. Analytical Biochemistry 343(I):76-83).

The methods described herein comprise providing a plurality of test protein preparations comprising the target cellular kinase and at least one phosphatase inhibitor. The test protein preparations may be generated from a cell sample as part of the method (see below). Alternatively, the test protein preparations may be provided/obtained from another source (e.g. they may be provided as cell lysates or cell extracts per se, for the user to test). Typically, when provided as a cell lysate or cell extract, the protein preparations have been subjected to phosphatase inhibitors during the lysis procedure.

The methods described herein may include the step of lysing cells comprising the target cellular kinase in the presence of at least one phosphatase inhibitor to generate a plurality of test protein preparations comprising the target cellular kinase.

Any suitable cell may be lysed to generate the plurality of test protein preparations. Suitable cells include any cell wherein kinases of interest are expressed. The choice of the cell may be influenced by the purpose of the study. For example, the method can be used to identify or profile kinases and signalling pathways associated with disease in individual patient (e.g. cancer patient) samples. This can provide an individual diagnostic or therapeutic approach for these patients.

In one example, the cell is human. Alternative sources of cells include those of any mammalian species, for example non-human primates such as apes or monkeys, or other mammals including, but not limited to mice, rats, sheep, cows, pigs, guinea pigs, camels, goats, donkeys, cats, dogs, rabbits, meerkats etc. Non-mammalian cells may also be used.

For example, primary animal or human cells may be used. Suitable cells include at least one intact cell isolated from a cell tissue, a biopsy, or a fluid sample (e.g. blood, cerebrospinal fluid, peritoneal fluid and urine) from a subject (e.g. human). By way of a non-limiting example, cells (e.g. lymphocytes and lymphocyte subpopulations) may be isolated from a peripheral blood sample obtained from a patient. For example, density gradient centrifugation is a method for the separation of lymphocytes from other blood cell populations (e.g. erythrocytes and granulocytes). Human lymphocyte subpopulations can be isolated via their specific cell surface receptors which can be recognized by monoclonal antibodies. The physical separation method involves coupling of these antibody reagents to magnetic beads which allow the enrichment of cells that are bound by these antibodies (positive selection).

As stated above, a suitable cell may be obtained from an organism, e.g. by biopsy. Corresponding methods are known in the art. For example, a biopsy is a diagnostic procedure used to obtain a small amount of tissue, which can then be examined microscopically or with biochemical methods. Biopsies are important to diagnose, classify and stage a disease, but also to evaluate and monitor drug treatment.

As an alternative to primary human cells, cultured cell lines (e.g. MOLT-4 cells, A431, Jurkat, Ramos or HeLa cells) can also be used. For example, the cells may be part of a cell culture system and methods for the harvest of a cell out of a cell culture system are known in the art (literature supra). The choice of the cell will mainly depend on the substrate (and target kinase/signalling pathway) of interest. In order to determine whether a given cell is a suitable starting system for the methods of the invention, methods like Western blots, PCR-based nucleic acids detection methods, Northern blots and DNA- microarray methods ("DNA chips") might be suitable in order to determine whether a given protein of interest is present in the cell.

The method described herein may include the step of harvesting the cells prior to lysis.

Cells comprising the target cellular kinase may be lysed in the presence of at least one phosphatase inhibitor to generate a plurality of test protein preparations comprising the target cellular kinase.

As used herein a "protein preparation" is a sample which comprises proteins and no longer comprises intact cells. A protein preparation may also include several other molecules such as lipids, metabolites etc. Well known examples of protein preparations generated from cell samples include cell lysates and cell extracts. Accordingly, in one example, the plurality of protein preparations are cell lysates or cell extracts.

Lysis refers to the breaking down of the cell, often by physical, viral, enzymic, chemical or osmotic mechanisms that compromise its integrity. A fluid containing the contents of lysed cells is called a "cell lysate". Cell lysates are generated by lysis of a cell sample (i.e. a sample having intact cells). Methods for the lysis of cells are known in the art (Karwa and Mitra: Sample preparation for the extraction, isolation, and purification of Nuclei Acids; chapter 8 in "Sample Preparation Techniques in Analytical Chemistry", Wiley 2003, Editor: Somenath Mitra, print ISBN: 0471328456; online ISBN: 0471457817). Lysis of different cell types and tissues can be achieved by homogenizers (e.g. Potter-homogenizer), ultrasonic disintegrators, enzymatic lysis, detergents (e.g. NP-40, Triton X-100, CHAPS, SDS), osmotic shock, repeated freezing and thawing, or a combination of these methods.

As a non-limiting example, cells may be lysed at 4°C in a suitable lysis buffer. Several suitable lysis buffers are known in the art, for example the following may be used: 50 mM Tris, 0.25 M NaCl, 0.1% Triton X100, 10 mM MgCl₂, 2 mM EDTA, 1 mM DTT, pH 7.5 with protease inhibitor cocktail (Sigma P8340), Phosphostop (Roche), 1 mM PMSF, 0.1 µM okadaic acid, 10 mM NaF, 20 mM β-glycerophosphate. Typically, lysates are immediately flash frozen in liquid nitrogen. In the context of the methods described herein, a plurality of frozen lysates or cell extracts may be provided in step (a). In other words, frozen test protein preparations (e.g. frozen lysates or cell extracts) comprising the target cellular kinase and at least one phosphatase inhibitor may be provided as the starting material for the method described.

A protein preparation described herein may also be a partial cell lysate (also called a cell extract herein). Partial cell lysates or cell extracts are obtained by lysing the cell of interest and centrifuging out the cell walls, DNA genome, and other debris. Methods for generating cell extracts are well known. Cell extracts can be further fractionated to generate a protein preparation which is enriched in specific types of proteins such as cytoplasmic or membrane proteins (Chapter 4.3 Subcellular Fractionation of Tissue Culture Cells in "Current Protocols in Protein Science", Editors: John E. Coligan, Ben M. Dunn, Hidde L. Ploegh, David W. Speicher, Paul T. Wingfield; Wiley, ISBN: 0-471-14098-8).

It is advantageous to add at least one phosphatase inhibitor during the process of generating a protein preparation from a cell sample (e.g. during lysis of the cells in the preparation of a cell lysate or cell extract). The phosphatase inhibitors prevent dephosphorylation of active kinases, which ensures that kinases that directly act on the selected substrate are identified in the subsequent assay. Suitable phosphatase inhibitors and their appropriate concentrations are well known in the art. For example, appropriate phosphatase inhibitors include okadaic acid, sodium fluoride, β-glycerophosphate, calyculin A, microcystin LR, cantharidin, sodium molybdate, sodium tartrate, (-)-p-bromotetramisole oxalate, sodium orthovanadate, sodium pyrophosphate, imidazole, Ascomycin, Cyclosporin A, FK506, Fostriecin, Fumonisin B1, GSK2830371, 9,10-Dihydro-9,10[1',2']-benzenoanthracene-1,4-dione, Raphin 1, Salubrinal, Sal 003, Sanguinarine, Sephin 1, Tautomycetin, Tautomycin, DL-2-Amino-3-phosphonopropionic acid (AP-3), Arcapillin, Endothall, Levamisole, Rubratoxin or combinations thereof (eg Phosphostop (Roche)). Other suitable phosphatase inhibitors are also commercially available and may be used within the context of the invention.

By way of example only, the following concentrations of phosphatase inhibitor may be used; 0.1 µM okadaic acid, 10 mM NaF, 20 mM β-glycerophosphate, 0.1 µM calyculin A, 0.1 µM microcystin LR, 20 µM cantharidin, 2 mM sodium molybdate, 5 mM sodium tartrate, 0.1 mM (-)-p-bromotetramisole, 5 mM sodium orthovanadate, 5 mM sodium pyrophosphate, or combinations of these (eg Phosphostop (Roche)). It is noted that these concentrations may be varied and other suitable concentrations or concentration ranges may be identified by a person of skill in the art, using routine experimentation.

Advantageously, as lysis of the cells is performed in the presence of at least one phosphatase inhibitor, the target cellular kinase remains active in the test protein preparations.

Advantageously, by using protein preparations such as cell lysates or cell extracts, the methods described herein maintain the physiological relevance (i.e. the complex cellular environment and protein interactions) of a cellular assay, whilst being technically simple (e.g. compared to RNAi). Furthermore, cell lysates and cell extracts preserve physiological kinase-phosphosite dependencies and therefore are more physiologically relevant than in vitro assays. The methods described herein therefore allow the user to look at a "snap shot" of cellular phosphorylation events without the complexities of an intact cellular assay.

The methods described herein may comprise stimulating a signaling pathway associated with phosphorylation of the selected substrate and/or synchronizing the cell cycle in the cells before generating the plurality of test protein preparations. For example, it may be beneficial to test cells at a specific cell cycle stage (e.g. enrich for a mitotic cell population). This can be achieved using known compounds that block the cell cycle, such as nocodazole, which is used in the examples below for 9 hours pre-cell lysis. Such methods are standard in the art.

It may also be beneficial to trigger a signaling pathway leading to phosphorylation/enhance phosphorylation signal in the cells prior to e.g. cell lysis. For example, EGF stimulation as described in the examples below can be used to explore phosphorylation downstream of EGFR. Such methods are standard in the art.

A plurality of test protein preparations (e.g. cell lysates or cell extracts) are provided in the methods described herein. A single cell lysate or cell extract may be provided initially and then aliquoted to provide a plurality of equivalent test protein preparations. Alternatively, a plurality of cell samples may all have been lysed in parallel to generate the plurality of test protein preparations, which are then provided for the assay. In the latter example, it may be advantageous for the plurality of cell samples to have been treated before lysis e.g. to ensure they are all/predominantly at the same stage of the cell cycle etc.

In this context, a "plurality" of test protein preparations refers to two or more, five or more, ten or more, twenty or more, thirty or more, forty or more, fifty or more, seventy five or more, one hundred or more protein preparations (cell lysate or cell extract samples/aliquots). Preferably, the minimum number of test protein preparations is in the 10 to 100 range (i.e. so that at a minimum of a 10-100 different inhibitors are tested). The plurality of protein preparations that are tested may be at least 10, at least 20, at least 30, at least 40, at least 50, at least 100, at least 150, at least 200, at least 250, at least 300 etc. As will be described in more detail below, each of the test protein preparations is for incubation with a distinct known kinase inhibitor in the presence of a selected substrate. The method provided herein encompasses situations wherein the plurality of test protein preparations are tested in parallel (i.e. simultaneously) as well as situations wherein the plurality of protein preparations are tested sequentially (i.e. not at exactly the same time). For example, the method encompasses situations wherein the test protein preparations are tested in batches of e.g. 10, wherein each batch of 10 is tested sequentially until a total batch of e.g. 100 test protein preparations is tested using different kinase inhibitors to generate an inhibitor profile for the target cellular kinase.

The methods described herein may also include further "control" protein preparations. For example, "control" protein preparations may be equivalent to the test protein preparations but are not for incubation with the distinct known kinase inhibitors described above. A control protein preparation is typically an aliquot from the cell lysate or cell extract used to generate the test preparations or is a protein preparation that was generated simultaneously to (and using the same methodology as was used for) the test protein preparations. Control protein preparations may be incubated with selected substrate only (in the absence of a known kinase inhibitor), for example to confirm that the conditions used in the method are suitable for phosphorylation of the substrate. Alternative "control" protein preparations may be duplicates or triplicates of the test protein preparations.

The methods described herein comprise incubating each test protein preparations with a selected substrate and a kinase inhibitor, wherein the kinase inhibitor is different for each test protein preparation.

As used herein, "incubating" has its standard meaning, i.e. contacting the test protein preparation, selected substrate and kinase inhibitor together for a period of time. In this context, the period of incubation is one that is sufficient to enable phosphorylation of the selected substrate to occur if the target kinase is active (i.e. under conditions where there is no kinase inhibitor present that prevents the target kinase acting on the selected substrate). The minimum incubation time required will differ depending on a number of factors, including the concentration of selected substrate, concentration and activity status of the kinase, concentration of accessory proteins and co-factors etc. The minimum incubation time required can be deduced by a person of skill in the art using routine experimentation and known methodology (such as testing different incubation times and temperatures for incubating a control protein preparation with a selected substrate, without kinase inhibitors, to identify a minimum incubation time and temperature required to detect phosphorylation of the selected substrate by the protein preparation (and more specifically the kinase contained therein).

By way of a non-limiting example, suitable incubation times may be at least 5 minutes, at least ten minutes, at least fifteen minutes, at least twenty minutes, at least twenty-five minutes, at least thirty minutes etc. In one example, the incubation time may be 10 to 120 minutes, such as 10 to 60 minutes, or 30 to 45 minutes. Typically, incubation of test protein preparations with a selected substrate and a kinase inhibitor should not exceed 2 hours.

By way of a non-limiting example, suitable incubation temperatures are 15 to 40°C, typically 15 to 37°C. Typically, incubation of test protein preparations with a selected substrate and a kinase inhibitor should not exceed 40°C.

Typically, incubation of test protein preparations with a selected substrate and a kinase inhibitor is therefore for at least 5 minutes but not exceeding 2 hours at a temperature of 15 to 40°C. More typically, incubation of test protein preparations with a selected substrate and a kinase inhibitor is for at least 5 minutes but not exceeding 2 hours at a temperature of 15 to 37°C.

Incubation of the test protein preparations with a selected substrate and a kinase inhibitor may also be in the presence of appropriate phosphatase inhibitors. Suitable phosphatase inhibitors are discussed above.

Suitable selected substrates are discussed above. In some examples, a test protein preparation may be incubated with more than one selected substrate simultaneously, for example during a multiplexing assay.

The selected substrate may be used in the methods described herein at any appropriate concentration. A person of skill in the art can easily deduce a suitable selected substrate concentration using the methods described herein e.g. by testing different selected substrate concentrations with a control protein preparation, without kinase inhibitors, to identify the selected substrate concentration required to detect phosphorylation of the selected substrate by the protein preparation (and more specifically the kinase contained therein). The concentration required will vary depending on the selected substrate. Typically, the selected substrate may be at a concentration of from about 0.01 µM to about 10 µM.

The test protein preparation (e.g. cell lysate or cell extract) and the selected substrate are incubated together in the presence of a kinase inhibitor. The method is performed in parallel with a plurality of (e.g. at least two or more, five or more, ten or more, twenty or more, thirty or more, forty or more, fifty or more, seventy five or more, one hundred or more etc) protein preparations, where each test protein preparation is incubated with the selected substrate and a different kinase inhibitor. In other words, the samples tested in the method have the same or equivalent source of kinase (the protein preparation sample), the same selected substrate, but a different kinase inhibitor. This allows the effect of several kinase inhibitors on the phosphorylation of the selected substrate to be tested simultaneously, which provides an inhibitor profile for the kinase(s) in the test preparation sample. Preferably, the minimum number of test protein preparations is in the 10 to 100 range (i.e. so that at a minimum of 10-100 different inhibitors are tested). The plurality of protein preparations that are tested may be at least 10, at least 20, at least 30, at least 40, at least 50, at least 100, at least 150, at least 200, at least 250, at least 300 etc.

The kinase inhibitors used in the methods described herein are known kinase inhibitors e.g. obtained as part of a kinase inhibitor library. Advantageously, libraries with a plurality of different known kinase inhibitors are publicly available such as e.g. PKIS1 (protein kinase inhibitor set compiled from GlaxoSmithKline compounds by the Structural Genomics Consortium (Elkins et al., 2016)), PKIS2 (also from the Structural Genomics Consortium; https://journals.plos.org/plosone/article?id=10.1371/journal.pone.0181585) and a library of inhibitors assembled by Calbiochem (Anastassiadis et al., 2011; Gao et al., 2013). Any appropriate kinase inhibitor library may be used.

The methods described herein generate an inhibition profile for the target cellular kinase. The inhibition profile is generated for the inhibitory information generated from individually incubating the test protein preparations with several (e.g. tens, or hundreds as described elsewhere herein) different kinase inhibitors in the presence of the selected substrate. Any suitable number of different kinase inhibitors may individually be incubated with a test protein preparation in the presence of the selected substrate in order to generate the inhibition profile for the target cellular kinase. In one example, at least ten different kinase inhibitors are used individually to determine the inhibition profile of the target cellular kinase. In further examples, at least twenty, at least thirty, at least forty, at least fifty, at least seventy-five, at least one hundred, at least one hundred and fifty etc different kinase inhibitors are used individually. Each kinase inhibitor may be used singularly, or may be tested in duplicate, triplicate etc.

Kinase inhibitors may be used in the methods described herein at any appropriate concentration. A person of skill in the art can easily deduce a suitable kinase inhibitor concentration using the methods described herein. The concentration required may vary depending on the kinase inhibitor. Typically, the kinase inhibitor may be at a concentration of from about 0.1 µM to about 50 µM.

A "library" refers to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

The term "inhibitor" is used in the broadest sense and includes any ligand that partially or fully blocks, inhibits or neutralizes a biological activity exhibited by a kinase. In this context, a "ligand" is an entity which has an intrinsic binding affinity for the kinase. The ligand can be a molecule, or a portion of a molecule which binds the kinase. The ligands are typically small organic molecules which have an intrinsic binding affinity for the target molecule, but may also be other sequence-specific binding molecules, such as peptides (D-, L- or a mixture of D- and L-), peptidomimetics, complex carbohydrates or other oligomers of individual units or monomers which bind specifically to the target. The term also includes various derivatives and modifications that are introduced in order to enhance binding to the kinase. "Small molecules" are usually less than about 10 kDa molecular weight, and include but are not limited to synthetic organic or inorganic compounds, peptides, (poly) nucleotides, (oligo) saccharides and the like. Small molecules specifically include small non-polymeric (i.e. not peptide or polypeptide) organic and inorganic molecules. Many pharmaceutical companies have extensive libraries of such molecules. Preferred small molecules have molecular weights of less than about 1000 Da, more preferably about 750 Da, and most preferably about 500 Da.

The kinase inhibitor is preferably brought into contact with the test protein preparation prior to (or at the same time as) contact between the test protein preparation and the selected substrate.

Incubation of a test protein preparation with the selected substrate and a kinase inhibitor occurs under conditions that enable phosphorylation of the selected substrate to occur if the target kinase is active (i.e. under conditions where there is no kinase inhibitor present that prevents the target kinase acting on the selected substrate). The skilled person will know which conditions can be applied.

By way of example, incubation of a test protein preparation, selected substrate and a kinase inhibitor may be carried out under essentially physiological conditions. The physical state of proteins within cells is described in Petty, 1998 (Howard R. Petty, Chapter 1, Unit 1.5 in: Juan S. Bonifacino, Mary Dasso, Joe B. Harford, Jennifer Lippincott-Schwartz, and Kenneth M. Yamada (eds.) Current Protocols in Cell Biology Copyright © 2003 John Wiley & Sons, Inc. All rights reserved. DPI: 10.1002/0471143030.cb0101s00 Online Posting Date: May, 2001 Print Publication Date: October, 1998).

The contacting under essentially physiological conditions has the advantage that the interactions between the selected substrate, the protein preparation (i.e. the kinase to be characterized) and the kinase inhibitor reflect as much as possible the natural conditions. "Physiological conditions" are inter alia those conditions which are present in the original, unprocessed sample material. They include the physiological pH, salt concentration, buffer capacity and post-translational modifications of the proteins involved. The term "essentially physiological conditions" does not require conditions identical to those in the original living organism, wherefrom the sample is derived, but essentially cell-like conditions or conditions close to cellular conditions. The person skilled in the art will, of course, realize that certain constraints may arise due to the experimental set-up which will eventually lead to less cell-like conditions. For example, the eventually necessary disruption of cell walls or cell membranes when taking and processing a sample from a living organism may require conditions which are not identical to the physiological conditions found in the organism. For example, for cell lysates (which are always dilutes), essentially physiological conditions may comprise using relative protein concentrations that are similar to those present in the original, unprocessed sample material. Suitable variations of physiological conditions for practicing the methods of the invention will be apparent to those skilled in the art and are encompassed by the term "essentially physiological conditions" as used herein. In summary, it is to be understood that the term "essentially physiological conditions" relates to conditions close to physiological conditions, as e. g. found in natural cells, but does not necessarily require that these conditions are identical.

For example, "essentially physiological conditions" may comprise 10-200 mM (e.g. 12.5 to 125 mM) NaCl or KCI, pH 6.5-8.5, 20-37°C, and 0.001-10 mM divalent cation (e.g. Mg++, Ca++,); more preferably about 150 m NaCl or KCI, pH7.2 to 7.6, 5 to 10 mM Mg++ and often include 0.01-1.0 percent non-specific protein (e.g. BSA). A non-ionic detergent (Tween, NP-40, Triton-X100) can often be present, usually at about 0.001 to 2%, typically 0.05-0.2% (volume/volume). For general guidance, the following buffered aqueous conditions may be applicable: 10-250 mM NaCl, 5-10 mM MgCl₂, 5-50 mM Tris HCl, pH5-8, with optional addition of other divalent cation(s) and/or metal chelators and/or non-ionic detergents.

Preferably, "essentially physiological conditions" refers to a pH of from 6.5 to 7.5, preferably from 7.0 to 7.5, and / or a buffer concentration of from 10 to 50 mM, preferably from 25 to 50 mM, and / or a concentration of monovalent salts (e.g. Na or K) of from 120 to 170 mM, preferably 150 mM, and / or MgCl₂ at 1 to 10 mM, preferably 5 to 10 mM. Other divalent salts (e.g. Mn or Ca) may further be present at a concentration of from 1 to 10 mM, preferably 1 to 2 mM, wherein more preferably the buffer is selected from the group consisting of Tris-HCl or HEPES.

In a specific, but non-limiting example, incubations between a test protein preparation, a selected substrate and a kinase inhibitor may be carried out using the following conditions: 10 µM kinase inhibitor, 0.1 µM selected substrate (peptide), 0.2 mM ATP, 0.5 to 5% cell extract, in KiPIK buffer (50 mM Tris, 10 mM MgCl₂, 1 mM EGTA, 10 mM NaF, 20 mM β-glycerophosphate, 1 mM PMSF, pH7.5 with Phosphostop (Roche). Reactions can be carried out at 30°C for 30 min, in a total volume of 35 µl/well.

As will be well known in the art, ATP is required for kinase activity. A suitable range of ATP concentrations are well known in the art. For example, ATP may be at a concentration of about 0.05 to 50 mM during step (b) of the method.

As will be well known in the art, divalent salts (e.g. Mg, Mn or Ca) may act as co-factors for kinase activity. A suitable range of divalent salt concentrations are well known in the art. For example, the divalent salt (e.g. Mg, Mn etc) may be at a concentration of about 1 to 50 mM, preferably 5 to 10 mM during step (b) of the method.

The methods described herein also comprise determining whether the selected substrate is phosphorylated in each of the test protein preparations thereby obtaining an inhibition profile for the target cellular kinase.

Numerous high-throughput screening assays for measuring kinase activity have been developed and are well established in the art. Some assays use the detection of radioactively labeled (e. g. ³²P or ³³P) phosphate incorporated into the substrate to quantify kinase activity, or use chromatographic or mass spectrometric approaches to detect size changes caused by phosphorylation. In other assays, quantification of the kinase activity is based on the use of phospho-specific antibodies. In the latter case, typically a fluorescent label is attached to the antibody or the substrate, or both, a HRP or AP enzyme is conjugated to the antibody and the antibody is detected with a colorimetric readout, e. g. time-resolved fluorescence (TRF), fluorescence resonance energy transfer (FRET), or fluorescence polarization (FP). For example, the HitHunter assay (Discoverx) for identifying serine-threonine kinase inhibitors measures substrate phosphorylation in a competitive immunoassay format, using an anti-phospho-peptide antibody and chemiluminescent or fluorescent readout. An antibody-free fluorescence polarization-based kinase assay is available, for example, from Chromagen (San Diego, CA). Protein tyrosine kinase assay systems are also available, for example, from Invitrogen (Pan Vera FP Assay Kit).

Suitable antibody-based assays include but are not limited to Western blots, ELISA assays, sandwich ELISA assays and antibody arrays or a combination thereof. The establishment of such assays is known in the art (Chapter 11, Immunology, pages 11-1 to 11-30 in: Short Protocols in Molecular Biology. Fourth Edition, Edited by F.M. Ausubel et al., Wiley, New York, 1999).

The selected substrate may have been engineered to have a tag, such as a GFP tag, a hemagglutinin (HA) tag, a histidine (His) TAG, a V5-tag, a yc-tag, a glutathione S-transferase (GST) tag or a maltose- binding protein (MBP) tag, which allows the substrate to be isolated, for example by immunoprecipitation. Substrates may also be isolated by using an antibody which recognises them or the phosphosite directly.

In a particular example, phosphorylation of the selected substrate may be determined using ³²P-orthophosphate radiolabelling, detection of mass changes (eg mass spectrometry), phospho-specific antibodies, ELISA, immunoblotting or a combination thereof.

The percent inhibition scores for all inhibitors can then be calculated and compiled as the inhibition profile for the target cellular kinase.

As used herein, "inhibition profile" refers to the inhibition "fingerprint" or "signature" for the kinase (in other words, it is the unique pattern of inhibition observed for that particular kinase when subjected to the known kinase inhibitors used in the assay. The inhibition profile can be used as a unique identifier for the kinase. Inhibition profile, inhibition fingerprint, inhibition signature and inhibition pattern are used herein interchangeably.

The methods described herein comprise the step of comparing the target cellular kinase inhibition profile with the inhibition profiles of known kinases, wherein the most similar known kinase inhibition profile identifies or profiles the target cellular kinase. In this context, "most similar" may also be referred to as "closest match", "nearest", or "top hit". The level of similarity between the inhibition profile for the target cellular kinase and the inhibition profile(s) of known kinases may be determined using any method known in the art. For example, Prism (GraphPad) can be used to calculate the Pearson's correlation for the target kinase against the inhibition profiles of the known kinases. Alternative methods may also be used, for example Spearman correlation coefficients, Euclidean distances, or any other measures of correlation known in the art. Machine learning/artificial intelligence may also be used, for example a machine could be trained to recognize the differences between kinases based on inhibition profiles (without specifying correlation as the method) which can then can be used to classify unknowns from the KiPlK screens.

The inhibition profiles of various known kinases have already been identified and are publicly available as part of known "kinase profiling datasets". Examples of such profiling datasets include the Nanosyn profiling dataset (see Figure 2 for example); the DSF profiling dataset (see Figure 2 for example); and the Anastassiadis, Davis and Gao profiling datasets used in the examples section below (see Figure 5 for example). These inhibition profile datasets (and others) can be compared to the target cellular kinase inhibition profile, wherein the most similar known kinase inhibition profile identifies or profiles the target cellular kinase.. These profiling datasets have been generated using libraries of small molecule kinase inhibitors on large panels of recombinant kinases in vitro. They provide unique inhibition patterns/fingerprints for approximately 80% of human protein kinases.

As will be clear to a person of skill in the art, the target cellular kinase can only be identified by the methods provided herein if the known kinases that its inhibition profile is compared to include the target kinase itself. In other words, if the known kinase profiling datasets used in the method do not include the unknown target kinase, the method will not identify the exact kinase that is responsible for the phosphorylation event of interest, but will rather provide, as the top hit in the comparison (i.e. with the highest level of similarity) the most closely related kinase that is present within the kinase profiling dataset being used. Therefore, even in circumstances where the kinase profiling dataset used is not optimal, the methods described herein allow the user to profile the target cellular kinase by identifying the "closest match" to the target kinase. This concept is considered as "profiling the target cellular kinase" herein. As described in more detail below in the examples section, related kinases have been shown to have similar inhibition profiles. Accordingly, by comparing the target cellular kinase inhibition profile with the inhibition profiles of known kinases in a kinase profiling dataset, and optionally performing additional analysis such as presenting this information on a kinome tree, the user can "home in on" a subset of kinases that are likely candidates for the target cellular kinase (thus allowing the user to profile the target kinase without completely identifying it per se).

In one example, the target cellular kinase inhibition profile is compared to the inhibition profiles of at least ten, at least fifty, at least seventy-five, at least one hundred etc known kinases. In other words, the kinase profiling dataset that is compared to the target cellular kinase inhibition profile may comprise at least ten, at least fifty, at least seventy-five, at least one hundred etc known kinases. For example, the target cellular kinase inhibition profile may be compared to the inhibition profiles of at least 10, at least 50, at least 75, at least 100, at least 120, at least 140, at least 130, at least 150, at least 160, at least 170, at least 180, at last 190, at least 200, at least 220, at least 250, at least 300, at least 350, at least 400 etc inhibition profiles for known kinases.

Advantageously, simple ranking of correlation coefficients generated from the comparison of inhibition profiles between the target kinase and known kinases can readily identify relevant candidates (see Figures 2 to 5). Optionally, a Pearson's correlation coefficient score of at least 0.5 and/or correlations that are well-separated from other non-related hits (for example, using Grubbs' outlier test) are indicative and/or determinative of a match between the target cellular kinase and the corresponding known kinase.

Plotting kinase inhibition correlation plots provides additional information on the quality of the observed correlations (see Figure 9).

Displaying the correlation coefficients on a sequence-based kinome dendrogram allows related kinases not present in the profiling datasets to be identified (see Figure 6).

In addition, hierarchical clustering of all kinases based on the similarity of their inhibition profiles can also be carried out using Pearson correlation coefficients. This can be used to generate a kinome dendogram (see Figure 7) that allows kinases that have similar inhibition footprints to be identified in an alternative way.

In a further example, comparing the target cellular kinase inhibition profile with the inhibition profiles of known kinases comprises generating at least one of the following:
(i) a ranking of correlation coefficients (see e.g. Figures 2, 3B, 4A, 5), supported by kinase inhibition correlation plots (see e.g. Figs 9-13);
(ii) plotting correlation coefficients on a sequence-based human kinome tree (see e.g. Figures 6, 17);
(iii) an inhibition-based kinome dendrogram derived by hierarchical clustering using correlation coefficients, optionally with bootstrapping, (see e.g. Fig 7, 18-24, optionally as in Fig 25); or
(iv) a combination of (i) to (iii).

The methods of the invention provide a physiologically relevant method for identifying or profiling a target cellular kinase that phosphorylates a selected substrate. As stated elsewhere herein, the method uses lysed cell samples for the identification or profiling of target kinases within cells. As will be clear to a person of skill in the art, the cellular environment comprises complex interactions where more than one kinase may act on a specific substrate. Furthermore, as will be clear to a person of skill in the art, more than one kinase may also be capable of phosphorylating a specific substrate of interest. The methods used herein provide a "snap shot" of the kinase activity present in the cell prior to lysis. The methods described herein therefore advantageously identify or profile the predominant kinase acting on the substrate of interest in the cell at a specified time.

Advantageously, the method can be performed as a medium or high throughput screening assay.

### Medical uses

Kinases play a diverse role in cell regulation and have also been implicated in a number of diseases. For example, it has been established that small molecule kinase inhibitors have great potential as new anticancer therapeutics. Kinases are also important therapeutic targets for the development of anti-inflammatory drugs (Cohen, 2009. Current Opinion in Cell Biology 21, 1-8), for example kinases that are involved in the orchestration of adaptive and innate immune responses. The methods of the invention may therefore also be useful in identifying kinases involved in disease.

As a specific example, it is well known that tyrosine kinase inhibitors are particularly important in cancer therapy. Tyrosine kinases represent a gene family widely implicated in cancer pathogenesis and dysregulation of specific tyrosine kinases has been observed in most hematologic malignancies including chronic myeloid leukemia (CML), chronic myelomonocytic leukemia (CMML), other myeloproliferative neoplasms (MPN), acute myeloid leukemia (AML), and chronic lymphocytic leukemia (CLL). Although a minority of patients with haematologic malignancies are successfully treated with kinase inhibitors, most patients remain ineligible for this form of targeted therapy due to lack of knowledge of the specific kinase pathway involved.

The FDA have also approved several small molecule protein kinase inhibitors as therapeutic agents, targeting protein kinases implicated in disease. However, such kinase inhibitors are only effective in a subset of patients. The methods described herein may be used to identify an aberrant kinase specific to a particular patient/tumor, enabling such patients to be treated more effectively with inhibitors that are specific for the kinase at issue.

Examples of kinases that have been implicated in disease (and their corresponding FDA approved kinase inhibitor) include: CDK4/6 in breast cancer (Abemaciclib; palbociclib); BTK in Mantle cell lymphomas (Acalabrutinib); EGFR in NSCLC (Afatinib); ALK in NSCLC (Alectinib; Brigatinib; Ceritinib; Crizotinib); VEGFR in Advanced renal cell carcinomas, soft tissue sarcomas (Axitinib; Pazopanib); JAK1/2/3 and Tyk in Rheumatoid arthritis, myelofibrosis, polycythemia vera (Baricitinib; Ruxolitinib); MEK1/2 in Melanomas (Binimetinib); BCR-Abl in Chronic myelogenous leukemias (Bosutinib); RET in advanced medullary thyroid cancers (Cabozantinib); B-raf in BRAF mutation positive melanomas and NSCLC (Dabrafenib); EGFR in EGFR-mutant NSCLC (Dacomitinib); BCR-Abl in Chronic myelogenous leukemias (dasatinib); B-Raf in melanoma (Encorfenib); EGFR in NSCLC and pancreatic cancers (Erlotinib); FKBP12/mTOR in breast cancer, pancreatic neuroendocrine tumors, renal cell carcinomas, angiolyolipomas, subependymal giant cell astrocytomas (Everolimus); Syk in Chronic immune thrombocytopenia (Fostamatinib); EGFR in NSCLC (Gefitinib); Flt3 in acute myelogenous leukemias (Gilteritinib); BTK in chronic lymphocytic leukemias, mantle cell lymphomas, marginal zone lymphomas, graft vs host disease (Ibrutinib); BCR-Abl in CML or ALL, aggressive systemic mastocytosis, chronic eosinophilic leukemias, dermatofibrosarcoma protuberans, hypereosinophilic syndrome, gastrointestinal stromal tumours, myelodysplastic/myeloproliferative disease (Imatinib); EGFR in HER2 positive breast cancer (Lapatinib); TRK in solid tumours (Larotrectinib); VEGFR and Ret in differentiated thyroid cancers (Lenvatinib); Flt3 in acute myelogenous leukemias, mastocytosis, mast cell leukemias (Midostaurin); ErbB2 in HER2-positive breast cancers (Neratinib); ROCK1/2 in glaucoma (Netrsudil); BCR-Abl in Philadelphia chromosome positive CML (Nilotinib; ponatinib); FGFR in idiopathic pulmonary fibrosis (Nintedanib); EGFR in NSCLC (osimertinib); VEGFR in colorectal cancers (Regorafenib); FKBP12/mTOR in kidney transplant, lymphangioleiomyomatosis (Sirolimus); VEGFR in hepatocellular carcinomas, renal cell carcinomas, thyroid cancer (differentiated) (Sorafenib); VEGFR in gastrointestinal stromal tumors, pancreatic neuroendocrine tumors, renal cell carcinomas (Sunitinib); FKBP12/mTOR in advanced renewal cell carcinomas (Temsirolimus); JAK3 in rheumatoid arthritis (Tofacitinib); MEK1/2 in melanoma (trametinib); VEGFR in medullary thyroid cancers (Vandetanib); B-Raf in melanoma (Vemurafenib) etc.

As described above, the methods described herein can be used on clinical samples e.g. small biopsies, to identify the kinase involved in disease-relevant signalling pathways, such as inhibitor-resistant cancers. This would allow kinase inhibitors that inhibit particular phosphorylation events in a specific patient/tumour to be identified as a candidate therapeutic for that patient. One example might be if a patient is resistant to a particular therapeutic kinase inhibitor, the methods described herein may be used to screen for alternative inhibitors (and therefore kinases) that were good targets to prevent certain key phosphorylation events. In other words, the methods would provide a way to simultaneously determine the "bypass" pathways that lead to resistance to therapy in cancer as well as drugs suitable for inhibiting the pathway.

The methods described herein are therefore useful in identifying or profiling a target cellular kinase that is associated with aberrant phosphorylation in a disease or disorder, for example, where the disease or disorder is any of the disease or disorders listed above (e.g. breast cancer, Mantle cell lymphoma, NSCLC, advanced renal cell carcinomas, soft tissue sarcomas, rheumatoid arthritis, myelofibrosis, polycythemia vera, melanoma, chronic myelogenous leukemia, advanced medullary thyroid cancer, BRAF mutation positive melanomas, EGFR-mutant NSCLC, chronic myelogenous leukemia, pancreatic cancer, pancreatic neuroendocrine tumor, renal cell carcinoma, angiolyolipoma, subependymal giant cell astrocytoma, chronic immune thrombocytopenia, acute myelogenous leukemia, chronic lymphocytic leukemia, marginal zone lymphoma, graft vs host disease, CML or ALL, aggressive systemic mastocytosis, chronic eosinophilic leukemia, dermatofibrosarcoma protuberans, hypereosinophilic syndrome, gastrointestinal stromal tumour, myelodysplastic/myeloproliferative disease, HER2 positive breast cancer, solid tumours, differentiated thyroid cancers, acute myelogenous leukemias, mastocytosis, mast cell leukemia, HER2-positive breast cancer, glaucoma, Philadelphia chromosome positive CML, idiopathic pulmonary fibrosis, colorectal cancer, kidney transplant, lymphangioleiomyomatosis, hepatocellular carcinoma, renal cell carcinoma, thyroid cancer (differentiated), gastrointestinal stromal tumor, pancreatic neuroendocrine tumor, renal cell carcinoma, advanced renewal cell carcinoma, medullary thyroid cancer etc). In a particular example, the methods described herein may be useful in identifying or profiling a target cellular kinase that is associated with aberrant phosphorylation, herein the disease or disorder is cancer, rheumatoid arthritis, chronic immune thrombocytopenia, glaucoma, or idiopathic pulmonary fibrosis.

A kinase identified by the methods described herein may therefore be useful in therapy (e.g. where a disease or disorder is associated with reduced phosphorylation of the substrate of interest). Furthermore, an inhibitor of a kinase identified by the methods described herein may also be useful in therapy (e.g. where a disease or disorder is associated with increased phosphorylation of the substrate of interest).

Aspects of the invention are demonstrated by the following non-limiting examples.

Any of the following examples that do not fall within the scope of the appended claims are provided for comparative purposes only.

### EXAMPLES

### A KiPIK screen for the Histone H3S28 kinase identifies Aurora B

As a test of the methodology, the inventors sought to identify the direct kinase responsible for Histone 3 serine 28 (H3S28) phosphorylation in mitosis, previously reported to be Aurora B (Goto et al., 2002). The inventors made use of the PKIS1 panel of 317 kinase inhibitors (Elkins et al., 2016) that have been profiled *in vitro* on two panels of recombinant kinases: the "Nanosyn" assay assessed inhibition of 203 kinases at inhibitor concentrations of 0.1 µM and 1 µM, whereas the DSF (Differential Scanning Fluorimetry) assay assessed inhibitor binding at 1 µM to 68 kinases by thermal denaturation. Together, this provided coverage of 236 kinases (encoded by 234 unique genes). Extracts of HeLa cells that had been arrested in mitosis for 12 hours in nocodazole were used as the source of relevant kinases. To create an inhibition fingerprint for the H3S28 kinase, the phosphorylation of an H3(21-44)-GK-biotin peptide was assayed using anti-H3S28ph antibodies in the presence of each inhibitor individually at 10 µM. For the H3S28 kinase fingerprint, the top three correlation scores were all from the Nanosyn 1 µM profiling dataset, and all were with Aurora family kinases (Figure 2A; Figure 9). The highest overall correlation (r = 0.74) was with Aurora B. Notably, the top three hits within the Nanosyn 0.1 µM dataset were also all Aurora kinases. The DSF dataset did not contain the Aurora kinases, and no kinase in this dataset had a correlation score above 0.52. Therefore, the KiPlK method unambiguously identified Aurora family kinases as those most likely responsible for H3S28 phosphorylation in mitosis. Aurora B, the established kinase, was the top hit.

### KiPIK screens for tyrosine phosphorylation sites identify the expected kinases

The inventors wished to validate KiPlK screening for diverse kinases. They therefore conducted further screens to identify kinases for two tyrosine residues. The first, Y795 in the cytoplasmic tail of integrin β1A, thought to be phosphorylated by Src family kinases (Sakai et al., 2001; Calderwood et al., 2013), and the second was the Y1016 autophosphorylation site in the carboxy-terminal tail of EGFR (Y992 in mature EGFR) (Walton et al., 1990; Rotin et al., 1992). In both cases, they used generic phospho-tyrosine antibodies to detect phosphorylation of biotinylated peptides (ADE**Y**LIPQQ-biotin for EGFR and biotin-GG-KSAVTTVVNPKYEGK for integrin β1A) in extracts of A431 cells treated for 5 min with 50 ng/ml EGF, and the PKIS1 library of kinase inhibitors.

In the screen for kinases that phosphorylate integrin β1A Y795, the top 6 hits, and 10 of the top 11 overall, were members of the Src family kinase family (Figure 2B, Figure 10). Therefore, this screen unambiguously identified members of the Src family as candidate kinases responsible for integrin β1 Y795 phosphorylation, consistent with previous suggestions. In the screen for kinases that phosphorylate residue Y1016 of EGFR, there were three clear candidates (Figure 2C, Figure 11). The top hit was EGFR, and the two EGFR-related kinases ERRB2/HER2 and ERRB4/HER4 were second and third. Therefore, the KiPIK method successfully identified EGFR as the top candidate kinase for autophosphorylation of Y1016.

These results demonstrate that, for peptides containing a single tyrosine, generic phospho-specific antibodies can be used effectively for KiPlK screening, avoiding the need for phosphosite-specific antibodies. They also show that the method can be used successfully to identity kinases for a variety of specific phosphorylation sites, including on receptor and non-receptor tyrosine kinases as well as on a mitotic serine/threonine kinase.

### KiPIK screening identifies direct kinases and is resistant to indirect effects

To substantiate the idea that KiPlK screens are less susceptible to indirect upstream kinase activity than genetic screens, the inventors investigated a kinase known to be under the control of an upstream kinase (Aurora B) that is active in KiPlK conditions. Haspin is the direct kinase that phosphorylates Histone H3T3 during mitosis (Dai et al., 2005). However, in cells, Aurora B inhibition also reduces H3T3ph because Aurora B activates Haspin and inhibits the RepoMan-PP1 phosphatase that dephosphorylates H3T3ph (Wang et al., 2011; Qian et al., 2013). In contrast, in cell extracts containing phosphatase inhibitors, Aurora B inhibitors do not compromise generation of H3T3ph (Wang et al., 2011), presumably because Haspin is already phosphorylated and activated, and phosphatases are inactive. Consistent with this, when the inventors carried out a kinome-wide RNAi screen to identify kinases required for H3T3ph in mitotic HeLa cells, Haspin was the top hit, but Aurora B kinase was also identified in the top 3 (Figure 3A). However, when the inventors conducted a KiPIK screen using a H3(1-21) peptide to identify the kinase responsible for phosphorylating H3T3 in mitotic HeLa extracts (Figure 3B, Figure 12), none of the Aurora family kinases was within the top 40 (highest r = 0.32), while Haspin was the top hit (r = 0.66). In addition, the kinase Plk1, which also has a role in activating Haspin in cells (Ghenoiu et al., 2013; Zhou et al., 2014), was in the top 5 protein kinase hits in the RNAi screen but was in the bottom 20% of the KiPIK screen.

Therefore, when compared to an RNAi screen, a KiPlK screen for H3T3ph was relatively insensitive to the kinases upstream of the direct kinase.

EGFR and Src family kinases participate in intimate crosstalk in A431 cells. Src family kinases are activated in response to EGFR signaling in these cells, and EGFR inhibitors prevent Src activation (Osherov and Levitzki, 1994; Sato et al., 1995b; Weernink and Rijksen, 1995). In addition, active Src family kinases can feedback to activate EGFR (Sato et al., 1995a; Biscardi et al., 1999; Chung et al., 2009). When the inventors conducted KiPlK screens in EGF-stimulated A431 extracts for the Src-family substrate integrin β1A Y795, EGFR was present among the top ten kinases (Figure 2B). Similarly, several Src family kinases were found in the top twenty hits when they conducted a KiPlK screen for the EGFR autophosphorylation site on Y1016 (Figure 2C). Nevertheless, as described earlier, KiPIK unambiguously identified Src family kinases as the top candidates for integrin β1A Y795 phosphorylation, and EGFR as the top candidate for autophosphorylation at Y1016.

Together, these results support the contention that KiPlK screening is biased towards identifying the direct kinase responsible for a particular phosphorylation event, and is relatively insensitive to the indirect effect of kinases operating in signaling networks.

### KiPIK identification of INCENP S446 as a non-canonical substrate of Cdk1/Cyclin B

The inventors next sought to use KiPIK to identify the kinase for an unassigned phosphosite. In particular, they investigated consensus motifs which are commonly phosphorylated in mitosis (as detected by mass spectrometry), but for which the responsible kinase(s) were not identified (Dephoure et al., 2008). For one of these motifs, P-X-pS-X-X-[K/R], the inventors identified a site in the Inner Centromere Protein (INCENP) for which a phosphospecific antibody had been raised: INCENP S446ph (Hegemann et al., 2011). Phosphorylation of this site was previously shown to be sensitive to the Aurora B inhibitor Hesperadin (Hegemann et al., 2011), but this inhibitor is relatively non-selective (Bamborough et al., 2008) and the site does not match the classical Aurora kinase consensus. To identify the kinase responsible for phosphorylation of this site, the inventors conducted a KiPIK screen using a biotin-INCENP(439-453) peptide (GPREPPQSARRKRSY), and mitotic extracts of HeLa cells as a source of kinases. This screen unambiguously identified Cyclin-dependent kinases as the best hits in all three profiling datasets. The master regulator of mitosis, Cyclin B1-Cdk1, was the top hit (r = 0.81; Figure 4A, Figure 13).

To confirm that Cyclin B1-Cdk1 was a bona fide kinase for INCENP S446, the inventors first conducted *in vitro* kinase assays. Indeed, Cyclin B1-Cdk1 efficiently phosphorylated S446 on a recombinant INCENP fragment containing residues 369-583, but not the TSS motif (S893/S894) in recombinant INCENP residues 825-918. In contrast, Aurora B was able to phosphorylate its known target, the TSS motif of INCENP, but not S446 (Figure 4B). Furthermore, INCENP immunoprecipitated from nocodazole-arrested mitotic cells was recognized by the INCENP S446ph antibody, and this phosphorylation was sensitive to the Cdk inhibitor Roscovitine (Figure 14). However, determining the direct substrates of Cyclin B1-Cdk1 in cells is complicated because inhibition of Cdk1 prevents mitotic entry and/or forces mitotic exit, leading to indirect effects on multiple kinase substrates. To address this, the inventors used immunofluorescence microscopy and high content imaging to analyze the kinetics with which INCENP S446ph was lost following acute Cdk inhibition in nocodazole-arrested mitotic cells. The proteasome inhibitor MG132 was also included because it prevents the degradation of key mitotic regulators such as Cyclin B1 and, in some instances, is reported to delay mitotic exit even in the presence of Cdk1 inhibitors (Skoufias et al., 2007). Aurora B relocates to the spindle midzone at the end of mitosis so, as an additional way to focus on cells still in mitosis, the inventors analyzed only cells in which Aurora B remained on chromosomes. They found that Cdk1 inhibition using RO-3306 led to a 50% reduction in intensity of INCENP S446ph over 9 minutes, while the Aurora B phosphorylation site INCENP TSS was unaffected (Figure 4C). In contrast, inhibition of Aurora B with ZM447439 led to a rapid decline of INCENP TSSph but did not influence INCENP S446ph over the same time period (Figure 4C). Also consistent with Cyclin B1-Cdk1 being the kinase responsible, INCENP S446ph was detected on chromosomes in mitotic cells from early mitosis until metaphase when Cyclin B1-Cdk is active, but was lost at anaphase when Cyclin B is degraded (Figure 4D). This pattern mirrored that of previously reported Cyclin B1-Cdk1 target sites on INCENP, but was distinct from that of INCENP TSSph (Figure 4D) which is generated by Aurora B activity throughout mitosis, including on the central spindle in anaphase (Goto et al., 2006). Together, these results support the identification of Cyclin B-Cdk1 as the primary direct kinase for INCENP S446 in cells, and show that KiPlK is a suitable method to identify the kinases required for the generation of "orphan" phosphorylation sites.

### Inhibitor libraries for KiPIK screening

So far, the inventors have shown that a single inhibitor panel, PKIS1, is suitable for use in KiPlK screening. The inventors wished to determine if additional inhibitor panels and profiling datasets could be utilized. In particular, where alternative inhibitor panels have been profiled on additional kinases, this allows the number of kinases covered by KiPlK screening to be increased. Further, not all kinase inhibition profiles are necessarily concordant between different profiling efforts (Sutherland et al., 2013), and the extent to which different compound sets give similar results provides an indication of the robustness of the method.

To explore this, the inventors assembled a "custom" library of 128 inhibitors that have been used in other kinase profiling studies. This included all 72 inhibitors profiled on 388 different kinases by Davis *et al.* ("Davis") using a competition binding assay (Davis et al., 2011), 76 of the 178 inhibitors profiled at 0.5 µM on 300 kinases by Anastassiadis *et al.* ("Anastassiadis") using conventional *in vitro* kinase assays at a fixed concentration of 10 µM ATP (Anastassiadis et al., 2011), and 55 of the 158 inhibitors profiled at 1 µM or 10 µM on 234 kinases by Gao *et al*. ("Gao 1 µM" and "Gao 10 µM"), also using *in vitro* kinase assays, but at near-*K*ₘ concentrations of ATP (Gao et al., 2013). Together, this encompasses 431 kinases (405 unique human kinase genes).

Using this library, the inventors repeated KiPlK screens for mitotic H3T3 and H3S28 phosphorylation. For H3T3ph, the highest overall correlation coefficient (r = 0.69) was with Haspin in the Gao 1 µM dataset, and Haspin was also the top scoring kinase (r = 0.66) in the Anastassiadis dataset. Haspin was not present in the Davis dataset, and no kinase in this panel had a correlation coefficient exceeding 0.52 (Figure 5A). As before, the upstream regulatory kinases for Haspin, Plk1 and Aurora B, did not score highly in the screen (Figure 5A). For H3S28ph, the highest overall correlation coefficient (r = 0.78) was with Aurora B in the Davis dataset, and Aurora B was also the top scoring kinase (r = 0.66) in the Anastassiadis dataset (Figure 5B). Therefore, KiPlK screening with alternative inhibitor and kinase panels was able to accurately identify the appropriate kinase for both sites.

A possible reason for the varying utility of profiling datasets was the different number of inhibitors tested from each (Figure 5; 314 for Nanosyn, 315 for DSF, 76 for Anastassiadis, 72 for Davis, 55 for Gao). To more formally test the impact of inhibitor panel size, for those KiPlK screens where a clear single kinase was identified as the expected top hit (For H3T3ph: DSF, Anastassiadis, Gao 1 µM; for H3S28ph: Nanosyn 1 µM, Anastassiadis, Davis; and for EGFR Y869ph: Nanosyn 0.1 µM and 1 µM), the inventors downsampled the inhibitor panel size by computationally removing random but fixed numbers of inhibitors. This analysis revealed that, when using large inhibitor panels, the identification of the correct kinase was generally robust until the inhibitor library size decreased below about 100 inhibitors (Figures 15, 16). Consistent with this, for the smaller panels of inhibitors, identification of the correct kinase fell off quickly as the numbers of inhibitors used decreased. It is therefore unsurprising that the smallest inhibitor panel used (for the Gao dataset) is the least robust. Nevertheless, the results suggest that the use of more inhibitors is likely to increase the utility of these additional datasets.

### Comparison of KiPIK to different in silico approaches

To compare the performance of KiPIK with in silico prediction approaches, the inventors used 8 different methods for which web-based tools are available to predict the most likely kinases for each of the phosphorylation sites used in KiPlK screening (Figure 26 lists predicted kinases for specific phosphorylation sites using in silico approaches). Expected kinases are shown in inverted bold type, and closely related kinases in bold. None of the approaches was consistently able to predict the correct kinase. For example, although many methods identified EGFR as the likely kinase for EGFR Y1016ph, three of the methods failed to place this kinase in the top three (GPS 3.0, NetPhos 3.1, and PhosphoNET). In contrast, GPS 3.0 was the only method that was able to place Haspin/GSG2 in the top three places for H3T3ph. For each phosphosite, the number of predictions that placed the correct kinase(s) as the top candidate were: H3T3ph, 0/8; H3S28ph, 1/8; Integrin β1A Y795ph, 0/8; EGFR Y1016ph, 5/8; INCENP S446ph, 2/8. This failure rate occurs despite the fact that the expected kinase is available to the prediction algorithms at high frequency: Haspin, 2/8; Aurora family, 7/8; Src family, 8/8; EGFR, 8/8; Cdk1, 8/8.

GPS 3.0 (http://gps.biocuckoo.org/index.php) uses similarity to experimentally determined kinase target sequences from the phospho.ELM database. Predictions can be made that cover 464 human protein kinases although, because this is a group-based prediction system, many kinases are in subgroups and there are approximately 280 human kinase-specific predictions. The method rests on assumption that kinases that are on a similar branch of a sequence-based dendrogram have similar substrates. The higher the score, the more likely the residue is phosphorylated (Xue et al., 2011; Xue et al., 2005).

KinasePhos 2.0 (http://kinasephos2.mbc.nctu.edu.tw/index.html) uses sequence-based amino acid coupling pattern analysis and solvent accessibility in a SVM (support vector machine). Validated sites from the phospho.ELM and Swiss-Prot databases were used for training. Predictions can be made for 58 human protein kinases. The higher the score, the more likely the residue is phosphorylated (Wong et al., 2007).

NetPhos 3.1 (http://www.cbs.dtu.dk/services/NetPhos/) predicts phosphorylation sites using neural networks based on phosphorylation sites in the phospho.ELM database. Predictions are made for 17 kinases: ATM, CKI/CSNK1, CKII/CSNK2, CaM-II/CAMK2, DNAPK/PRKDC, EGFR, GSK3, INSR, PKA, PKB/AKT, PKC, PKG, RSK, SRC, CDC2/CDK1, CDK5 and p38MAPK. Output scores are in the range 0 to 1; scores above 0.5 indicate "positive predictions" (Blom et al., 2004).

NetPhorest 2.1 (http://www.netphorest.info/index.shtml) uses a collection of probabilistic classifiers based on position-specific scoring matrices (PSSM) or neural networks to classify phosphorylation sites according to the likely kinase responsible. Like GPS 3.0, it assumes that kinases that are on a similar branch of a sequence-based dendrogram have similar substrates. It makes predictions for approximately 70 subgroups covering 222 kinases. Scores are from 0 to 1, with high scores being more confident predictions (Miller et al., 2008; Horn et al., 2014). NetworKIN 3.0 (http://www.networkin.info/index.shtml) builds on NetPhorest by including contextual information on kinases and substrates from the STRING database, such as binding interactions or substrates that have other residues that are already known targets of a kinase. The theoretically neutral score is 1, and the higher the score for a given kinase, the higher the likelihood it is indeed the kinase. Kinome coverage is as for NetPhorest 2.1. Note that CDK1 is a strong "hit" for INCENP S446ph in part because the STRING database recognises that other INCENP residues are known targets for this kinase (Linding et al., 2007; Horn et al., 2014).

PHOSIDA (http://141.61.102.18/phosida/index.aspx) includes the simple Motif Matcher tool that searches for sequence matches with annotated kinase recognition patterns for 33 motifs covering 25 kinases. No score is provided (Gnad et al., 2011).

PhosphoNET (http://www.phosphonet.ca) includes Kinase Predictor V2 that makes predictions based on determinants of specificity (DoS) within the primary amino acid sequences of the catalytic domains of 488 human protein kinases. The higher the score, the better the prospect that a kinase will phosphorylate a given site, with a maximum possible score of 1000 (Safaei et al., 2011).

ScanSite 4.0 (https://scansite4.mit.edu/4.0/#home). Phosphorylation sites for particular kinases are predicted using PSSMs determined from oriented peptide library techniques. Thirty-three motifs covering 31 human kinases are included. Scores are on a scale of 0 to infinity, where 0 means a protein sequence perfectly matches the optimal binding pattern, and larger numbers indicate progressively poorer matches to the optimal consensus sequence (Yaffe et al., 2001; Obenauer et al., 2003).

### Materials and Methods

### Cell culture

HeLa Kyoto or A431 cells were grown in DMEM with 5% (v/v) FBS and 100 U/ml Penicillin-Streptomycin, at 37°C and 5% CO₂ in a humidified incubator.

### Antibodies

For KiPIK screening and immunofluorescence microscopy, rabbit polyclonal antibodies to H3T3ph (B8634)(Dai et al., 2005), INCENP (P240, Cell Signaling Technology #2807), INCENP-S446ph (Dr Jan-Michael Peters, IMP, Vienna)(Hegemann et al., 2011) and INCENP-TSSph (Dr Michael Lampson, University of Pennsylvania)(Salimian et al., 2011); rabbit monoclonal antibody to the S-pT-P motif antibody (D73F6; Cell Signaling Technology #5243); mouse monoclonal antibodies to H3S28ph (CMA315)(Hayashi-Takanaka et al., 2014) and phospho-tyrosine (P-Tyr-100; Cell Signaling Technology #9411); sheep polyclonal antibodies to Aurora B (SAB.1, Dr Stephen Taylor, University of Manchester)(Girdler et al., 2006); and human centromere autoantibodies (Immunovision) were used. Antibodies used for siRNA screening were mouse monoclonal anti-H3T3ph (16B2)(Kelly et al., 2010) and rabbit polyclonal anti-H2BS6ph(Seibert et al., 2019).

### Peptides

INCENP peptide: Biotin-GPREPPQSARRKRSY (custom, Eurogentec)
EGFR peptide: Biotin-ADEYLIPQQ (AS-29942-1, Eurogentec)
H3(1-21) peptide: ARTKQTARKSTGGKAPRKQLA-GGK-biotin (custom, Abgent)
H3(21-44) peptide: ATKAARKSAPATGGVKKPHRYRPG-GK-biotin (AS-64440, Eurogentec)
Integrin β1A tail peptide: Biotin-GG-KSAVTTVVNPKYEGK (custom, Eurogentec)

### Preparation of KiPIK cell extracts

To prepare mitotic cell extract, HeLa cells were treated with 300 nM nocodazole for 9 h and then collected by shake off. To prepare EGF-stimulated cell extracts, A431 cells were trypsinised, collected in prewarmed DMEM supplemented with 50 ng/ml human EGF (Cell Signalling Technology) and incubated at 37°C for 5 min. After these treatments, cells were washed once with PBS and lysed at 4°C in 50 mM Tris, 0.25 M NaCl, 0.1% Triton X100, 10 mM MgCl₂, 2 mM EDTA, 1 mM DTT, pH7.5 with protease inhibitor cocktail (Sigma P8340), Phosphostop (Roche), 1 mM PMSF, 0.1 µM okadaic acid, 10 mM NaF, 20 mM β-glycerophosphate, at approximately 30 x 10⁶ cells/ml. Lysates were immediately flash frozen in liquid nitrogen.

### KiPIK screens

Kinase reactions contained 10 µM kinase inhibitor or DMSO (vehicle control), 0.1 µM peptide, 0.2 mM ATP, 0.5 to 5% cell extract, in KiPIK buffer (50 mM Tris, 10 mM MgCl₂, 1 mM EGTA, 10 mM NaF, 20 mM β-glycerophosphate, 1 mM PMSF, pH7.5 with Phosphostop (Roche). Reactions were carried out in duplicate in 384-well microplates (ThermoFisher), at 30°C for 30 min, in a total volume of 35 µl/well.

For detection of phosphorylation, High Capacity Streptavidin-coated 384-well plates (Pierce) were washed thrice with TBS, 0.05% Tween20. In some cases, 10 µl/well of 500 mM EDTA was added. Then, 35 µl/well of completed KiPIK extract kinase reaction was added and incubated at room temperature for 2 h. After washing, primary phospho-specific antibodies were added at 40 µl/well in TBS, 0.05% Tween20, 0.1% BSA, for 2 h at room temperature. After washing, 40 µl/well of HRP-conjugated secondary antibodies in TBS, 0.05% Tween20, 0.1% BSA were added for 1 h at room temperature. After washing, HRP-conjugated antibody binding was detected using TMB substrate (New England Biolabs) according to the manufacturer's instructions. All pipetting was performed with a Biomek FX liquid handling robot (Beckman Coulter). Absorbance readings were made using a Polarstar Omega microplate reader (BMG Labtech).

### Analysis of KiPIK results

Using the mean of duplicate determinations, a standard score was calculated for each inhibitor treatment where standard score = (mean absorbance in presence of inhibitor - mean absorbance of multiple DMSO controls) / standard deviation of DMSO controls. The inhibitory effect of each compound was defined as follows: %inhibition = 100 x (standard score for inhibitor / lowest standard score on plate). The lowest standard score on the plate was either from the EDTA-treated control wells or from those containing the most inhibitory compound. The %inhibition scores for all inhibitors were then compiled as the inhibition 'fingerprint' of the phosphorylation event probed. Using Prism (GraphPad), Pearson's correlation was then calculated for this 'fingerprint' against the inhibition profiles of each of the kinases profiled *in vitro* against that inhibitor library, excluding mutant forms of kinases. Notably, because Davis *et al.* determined K_{d} values for each kinase-inhibitor interaction in their panels, rather than single point percent inhibition values (Davis et al., 2011), the inventors made estimates of the %inhibition expected at 0.5 µM, using the Hill equation: %inhibition = 100/(1+(Kd/500)).

### Inhibitor downsampling and cluster analysis

To investigate how robust KiPIK results were to the size of the inhibitor library used, we downsampled inhibitor libraries and tested how easy it was to identify "true hits" using the downsampled versions. From existing libraries of size N, we repeatedly sampled (without replacement) n inhibitors and checked how many times the "true hit" was contained in the top k kinases ranked by correlation with the unknown kinase (Pearson's correlation coefficient, calculated using the cor function in R). By setting k = 1, 5 or 10 (or k ≈ N/100, N/20 or N/10) and setting n = 3,...,N and sampling 10,000 times, we generated curves representing how the uncertainty in hit identification varies with library size.

For hierarchical clustering of inhibition fingerprints, we used hclust and heatmap in the ComplexHeatmap package(Gu et al., 2016) implemented in R version 3.4.0 (2017-04-21; R Core Team, http://www.R-project.org/), calculating distances using (1 - Pearson correlation coefficient) and clustering using the method Ward.D2(Ward, 1963). For bootstrapping, we used hclust within pvclust package 4 with 10,000 iterations, producing two types of p-value: Approximately Unbiased (AU) p-value and Bootstrap Probability (BP) value. The AU value is considered the best indication of robustness(Suzuki and Shimodaira, 2006).

### siRNA screen

Hela cells were grown in clear-bottomed microplates (uClear 384 well microplates, Greiner). Transfection with an siRNA kinome library (Dharmacon) was carried out using Interferin-HTS (Polyplus), according to the manufacturer's instructions. After 24 h, cells were accumulated in mitosis for 6 hours by addition of 200 nM nocodazole, immunofluorescently stained for H3T3ph and H2BS6ph, and the intensity of staining quantified in approximately 300 cells per treatment by High Content Imaging. Experiments were carried out in quadruplicate. The standard score for condition was then calculated, where standard score = mean staining intensity in presence of siRNA / standard deviation of staining intensity for the entire plate; p-values were calculated using one-sample t-test for each siRNA treatment. Approximately 300 cells were measured for each siRNA treatment.

### Immunofluorescence

Cells were fixed for 10 min with 2% paraformaldehyde in PBS, washed twice in PBS, then permeabilized for 2 min with 0.5% Triton X100, PBS. After washing twice in PBS, cells were incubated for 1 hour in 5% milk in PBS, 0.05% Tween20 at room temperature, and then with primary antibodies at 37°C in 5% milk in PBS, 0.05% Tween20. After washing twice with PBS, 0.05% Tween20, the cells were washed twice with PBS, then incubated for 45 minutes with secondary antibodies at 37°C diluted in 5% milk in PBS, 0.05% Tween20. After washing twice with PBS, 0.05% Tween20, twice with PBS, and once with milliQ H₂O, were mounted using Prolong Gold (Invitrogen) for cover slips, or Fluoromount-G with DAPI (Invitrogen) for microplates.

### Microscopy

For High Content Imaging of cells in microplates, the average fluorescence intensity (integrated) for mitotic cells (defined as cells positive in either stained mitotic histone mark) was measured using a widefield Nikon Eclipse Ti-E microscope equipped with an automated stage, DS-Qi1MC camera (Nikon) and High Content Analysis software (Nikon Elements with JOBS software package) using a Plan Apo Lambda 20x/0.8 and 1.5x internal magnification. Cells on coverslips were imaged with a Zeiss Axiolmager microscope using a Plan-Apochromat 100x/1.40 oil objective. Optical sections were acquired every 0.1 µm using an AxioCam MR R3 camera. Image stacks are displayed as maximum intensity projections.

### In vitro kinase reactions

Reactions were carried out in 50 µl 20 mM HEPES, 0.14 M NaCl, 3 mM KCI, 1 mM ATP, 5 mM MgCl₂, pH 7.4 with approximately 100 nM of each substrate and 10 nM of recombinant kinase. Purified recombinant fragments of INCENP were from Novus Biologicals (pPEPTIDE-INCENP(369-583); NBP2-37471CUSTOM) and Upstate Biotechnology (GST-INCENP(825-918); 12-534). GST-CDK1/GST-CycB kinase was from Cell Signaling Technology (#7518), and Aurora B/INbox(825-918) was from Millipore (14-835).

### INCENP Immunoprecipitation

HeLa 1C8 cells (Dai et al., 2005) were treated with 0.5 µg/ml nocodazole for 8 h. After collecting mitotic cells by "shake off" and replating, either 100 µM Roscovitine or DMSO (vehicle control) was added together with 20 µM MG132 in the continued presence of nocodazole. After 2 h, cell lysates were prepared in 50 mM Tris, 0.3 M NaCl, 0.5% Triton X-100, 10 mM MgCl₂, 5 mM EDTA, 1 mM dithiothreitol, 1 mM PMSF, 0.1 µM okadaic acid, 10 mM NaF, 20 mM β-glycerophosphate, 0.25 U/µl benzonase nuclease, cOmplete^{™} Mini EDTA-free Protease Inhibitor and subjected to immunoprecipitation with rabbit anti-INCENP and Dynabeads Protein G (ThermoFisher). After washing, bound proteins were eluted by boiling in SDS-PAGE sample buffer.

### SDS-PAGE and immunoblotting

SDS-PAGE was carried out using the NuPAGE Bis-Tris system (ThermoFisher). Proteins were transferred onto PVDF membranes using standard techniques. Membranes were blocked with 2.5% milk in PBS, 0.1% Tween20 for 1 h, then with primary antibodies in 2.5% milk, PBS, 0.1% Tween20 overnight at 4°C. After washing 3 times with PBS, 0.1 % Tween20, they were incubated for 1 h with secondary antibodies in 2.5% milk, PBS, 0.1% Tween20, washed three times with PBS, 0.1% Tween20 and once with PBS, before development using ECL substrate (Pierce) and exposure to X-ray film. Stripping of immunoblots was carried out with Restore Western Blot Stripping Buffer (ThermoFisher).

The reader's attention is directed to all papers and documents which are filed concurrently with or previous to this specification in connection with this application and which are open to public inspection with this specification

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent, or similar purpose, unless expressly stated otherwise. Thus, unless expressly stated otherwise, each feature disclosed is one example only of a generic series of equivalent or similar features.

### References

Anastassiadis, T., Deacon, S.W., Devarajan, K., Ma, H., and Peterson, J.R. (2011). Comprehensive assay of kinase catalytic activity reveals features of kinase inhibitor selectivity. Nat Biotechnol 29, 1039-1045.
Bamborough, P., Drewry, D., Harper, G., Smith, G.K., and Schneider, K. (2008). Assessment of chemical coverage of kinome space and its implications for kinase drug discovery. J Med Chem 51, 7898-7914.
Bantscheff, M., Eberhard, D., Abraham, Y., Bastuck, S., Boesche, M., Hobson, S., Mathieson, T., Perrin, J., Raida, M., Rau, C., et al. (2007). Quantitative chemical proteomics reveals mechanisms of action of clinical ABL kinase inhibitors. Nat Biotechnol 25, 1035-1044.
Biscardi, J.S., Maa, M.C., Tice, D.A., Cox, M.E., Leu, T.H., and Parsons, S.J. (1999). c-Src-mediated phosphorylation of the epidermal growth factor receptor on Tyr845 and Tyr1101 is associated with modulation of receptor function. J Biol Chem 274, 8335-8343.
Calderwood, D.A., Campbell, I.D., and Critchley, D.R. (2013). Talins and kindlins: partners in integrin-mediated adhesion. Nat Rev Mol Cell Biol 14, 503-517.
Chung, B.M., Dimri, M., George, M., Reddi, A.L., Chen, G., Band, V., and Band, H. (2009). The role of cooperativity with Src in oncogenic transformation mediated by non-small cell lung cancer-associated EGF receptor mutants. Oncogene 28, 1821-1832.
Cuny, G.D., Robin, M., Ulyanova, N.P., Patnaik, D., Pique, V., Casano, G., Liu, J.F., Xian, J., Glicksman, M.A., Stein, R.L., et al. (2010). Structure-activity relationship study of acridine analogs as haspin and DYRK2 kinase inhibitors. Bioorg. Med. Chem. Lett. 20, 3491-3494.
Cuny, G.D., Ulyanova, N.P., Patnaik, D., Liu, J.F., Lin, X., Auerbach, K., Ray, S.S., Xian, J., Glicksman, M.A., Stein, R.L., et al. (2012). Structure-activity relationship study of beta-carboline derivatives as haspin kinase inhibitors. Bioorg. Med. Chem. Lett. 22, 2015-2019.
Dai, J., Sultan, S., Taylor, S.S., and Higgins, J.M.G. (2005). The kinase haspin is required for mitotic histone H3 Thr 3 phosphorylation and normal metaphase chromosome alignment. Genes and Development 19, 472-488.
Davies, S.P., Reddy, H., Caivano, M., and Cohen, P. (2000). Specificity and mechanism of action of some commonly used protein kinase inhibitors. Biochem J 351, 95-105.
Davis, M.I., Hunt, J.P., Herrgard, S., Ciceri, P., Wodicka, L.M., Pallares, G., Hocker, M., Treiber, D.K., and Zarrinkar, P.P. (2011). Comprehensive analysis of kinase inhibitor selectivity. Nat Biotechnol 29, 1046-1051.
De Antoni, A., Maffini, S., Knapp, S., Musacchio, A., and Santaguida, S. (2012). A small molecule inhibitor of Haspin alters the kinetochore functions of Aurora B. J. Cell Biol., 201205119.
Dedigama-Arachchige, P.M., and Pflum, M.K. (2016). K-CLASP: A Tool to Identify Phosphosite Specific Kinases and Interacting Proteins. ACS Chem Biol 11, 3251-3255. Deibler, R.W., and Kirschner, M.W. (2010). Quantitative reconstitution of mitotic CDK1 activation in somatic cell extracts. Molecular cell 37, 753-767.
Dephoure, N., Zhou, C., Villen, J., Beausoleil, S.A., Bakalarski, C.E., Elledge, S.J., and Gygi, S.P. (2008). A quantitative atlas of mitotic phosphorylation. Proc Natl Acad Sci U S A 105, 10762-10767.
Downward, J., Yarden, Y., Mayes, E., Scrace, G., Totty, N., Stockwell, P., Ullrich, A., Schlessinger, J., and Waterfield, M.D. (1984). Close similarity of epidermal growth factor receptor and v-erb-B oncogene protein sequences. Nature 307, 521-527.
Elkins, J.M., Fedele, V., Szklarz, M., Abdul Azeez, K.R., Salah, E., Mikolajczyk, J., Romanov, S., Sepetov, N., Huang, X.P., Roth, B.L., et al. (2016). Comprehensive characterization of the Published Kinase Inhibitor Set. Nat Biotechnol 34, 95-103.
Friedman, A., and Perrimon, N. (2006). A functional RNAi screen for regulators of receptor tyrosine kinase and ERK signalling. Nature 444, 230-234.
Gao, Y., Davies, S.P., Augustin, M., Woodward, A., Patel, U.A., Kovelman, R., and Harvey, K.J. (2013). A broad activity screen in support of a chemogenomic map for kinase signalling research and drug discovery. Biochem J 451, 313-328.
Ghenoiu, C., Wheelock, M.S., and Funabiki, H. (2013). Autoinhibition and Polo-Dependent Multisite Phosphorylation Restrict Activity of the Histone H3 Kinase Haspin to Mitosis. Molecular cell 52, 734-745.
Girdler, F., Gascoigne, K.E., Eyers, P.A., Hartmuth, S., Crafter, C., Foote, K.M., Keen, N.J., and Taylor, S.S. (2006). Validating Aurora B as an anti-cancer drug target. Journal of cell science 119, 3664-3675.
Goto, H., Kiyono, T., Tomono, Y., Kawajiri, A., Urano, T., Furukawa, K., Nigg, E.A., and Inagaki, M. (2006). Complex formation of Plk1 and INCENP required for metaphase-anaphase transition. Nat Cell Biol 8, 180-187.
Goto, H., Yasui, Y., Nigg, E.A., and Inagaki, M. (2002). Aurora-B phosphorylates Histone H3 at serine28 with regard to the mitotic chromosome condensation. Genes Cells 7, 11-17.
Gu, Z., Eils, R., and Schlesner, M. (2016). Complex heatmaps reveal patterns and correlations in multidimensional genomic data. Bioinformatics 32, 2847-2849.
Hayashi-Takanaka, Y., Stasevich, T.J., Kurumizaka, H., Nozaki, N., and Kimura, H. (2014). Evaluation of chemical fluorescent dyes as a protein conjugation partner for live cell imaging. PLoS One 9, e106271.
Hegemann, B., Hutchins, J.R., Hudecz, O., Novatchkova, M., Rameseder, J., Sykora, M.M., Liu, S., Mazanek, M., Lenart, P., Heriche, J.K., et al. (2011). Systematic phosphorylation analysis of human mitotic protein complexes. Sci Signal 4, rs12.
Jansson, D., Ng, A.C., Fu, A., Depatie, C., Al Azzabi, M., and Screaton, R.A. (2008). Glucose controls CREB activity in islet cells via regulated phosphorylation of TORC2. Proc Natl Acad Sci U S A 105, 10161-10166.
Johnson, S.A., and Hunter, T. (2005). Kinomics: methods for deciphering the kinome. Nat Methods 2, 17-25.
Kelly, A.E., Ghenoiu, C., Xue, J.Z., Zierhut, C., Kimura, H., and Funabiki, H. (2010). Survivin reads phosphorylated histone H3 threonine 3 to activate the mitotic kinase Aurora B. Science 330, 235-239.
Kubota, K., Anjum, R., Yu, Y., Kunz, R.C., Andersen, J.N., Kraus, M., Keilhack, H., Nagashima, K., Krauss, S., Paweletz, C., et al. (2009). Sensitive multiplexed analysis of kinase activities and activity-based kinase identification. Nat Biotechnol 27, 933-940.
Lemeer, S., and Heck, A.J. (2009). The phosphoproteomics data explosion. Curr Opin Chem Biol 13, 414-420.
Linding, R., Jensen, L.J., Ostheimer, G.J., van Vugt, M.A., Jorgensen, C., Miron, I.M., Diella, F., Colwill, K., Taylor, L., Elder, K., et al. (2007). Systematic discovery of in vivo phosphorylation networks. Cell 129, 1415-1426.
Maly, D.J., Allen, J.A., and Shokat, K.M. (2004). A mechanism-based cross-linker for the identification of kinase-substrate pairs. JAm Chem Soc 126, 9160-9161.
Manning, G., Whyte, D.B., Martinez, R., Hunter, T., and Sudarsanam, S. (2002). The protein kinase complement of the human genome. Science 298, 1912-1934.
Miller, M.L., Jensen, L.J., Diella, F., Jorgensen, C., Tinti, M., Li, L., Hsiung, M., Parker, S.A., Bordeaux, J., Sicheritz-Ponten, T., et al. (2008). Linear motif atlas for phosphorylation-dependent signaling. Sci Signal 1, ra2.
Moffat, J., Grueneberg, D.A., Yang, X., Kim, S.Y., Kloepfer, A.M., Hinkle, G., Piqani, B., Eisenhaure, T.M., Luo, B., Grenier, J.K., et al. (2006). A lentiviral RNAi library for human and mouse genes applied to an arrayed viral high-content screen. Cell 124, 1283-1298.
Olsen, J.V., Blagoev, B., Gnad, F., Macek, B., Kumar, C., Mortensen, P., and Mann, M. (2006). Global, in vivo, and site-specific phosphorylation dynamics in signaling networks. Cell 127, 635-648.
Osherov, N., and Levitzki, A. (1994). Epidermal-growth-factor-dependent activation of the src-family kinases. Eur J Biochem 225, 1047-1053.
Patricelli, M.P., Nomanbhoy, T.K., Wu, J., Brown, H., Zhou, D., Zhang, J., Jagannathan, S., Aban, A., Okerberg, E., Herring, C., et al. (2011). In situ kinase profiling reveals functionally relevant properties of native kinases. Chem Biol 18, 699-710.
Pomerening, J.R., Sontag, E.D., and Ferrell, J.E., Jr. (2003). Building a cell cycle oscillator: hysteresis and bistability in the activation of Cdc2. Nat Cell Biol 5, 346-351.
Qian, J., Beullens, M., Lesage, B., and Bollen, M. (2013). Aurora B Defines Its Own Chromosomal Targeting by Opposing the Recruitment of the Phosphatase Scaffold RepoMan. Curr Biol 23, 1136-1143.
Riel-Mehan, M.M., and Shokat, K.M. (2014). A crosslinker based on a tethered electrophile for mapping kinase-substrate networks. Chem Biol 21, 585-590.
Rotin, D., Margolis, B., Mohammadi, M., Daly, R.J., Daum, G., Li, N., Fischer, E.H., Burgess, W.H., Ullrich, A., and Schlessinger, J. (1992). SH2 domains prevent tyrosine dephosphorylation of the EGF receptor: identification of Tyr992 as the high-affinity binding site for SH2 domains of phospholipase C gamma. EMBO J 11, 559-567.
Rubin, C.S., and Rosen, O.M. (1975). Protein phosphorylation. Annu Rev Biochem 44, 831-887.
Sakai, T., Jove, R., Fassler, R., and Mosher, D.F. (2001). Role of the cytoplasmic tyrosines of beta 1A integrins in transformation by v-src. Proc Natl Acad Sci U S A 98, 3808-3813.
Salimian, K.J., Ballister, E.R., Smoak, E.M., Wood, S., Panchenko, T., Lampson, M.A., and Black, B.E. (2011). Feedback control in sensing chromosome biorientation by the Aurora B kinase. Curr Biol 21, 1158-1165.
Sato, K., Sato, A., Aoto, M., and Fukami, Y. (1995a). c-Src phosphorylates epidermal growth factor receptor on tyrosine 845. Biochem Biophys Res Commun 215, 1078-1087.
Sato, K., Sato, A., Aoto, M., and Fukami, Y. (1995b). Site-specific association of c-Src with epidermal growth factor receptor in A431 cells. Biochem Biophys Res Commun 210, 844-851.
Seibert, M., Kruger, M., Watson, N.A., Sen, O., Daum, J.R., Slotman, J.A., Braun, T., Houtsmuller, A.B., Gorbsky, G.J., Jacob, R., et al. (2019). CDK1-mediated phosphorylation at H2B serine 6 is required for mitotic chromosome segregation. J Cell Biol.
Sharma, K., Weber, C., Bairlein, M., Greff, Z., Keri, G., Cox, J., Olsen, J.V., and Daub, H. (2009). Proteomics strategy for quantitative protein interaction profiling in cell extracts. Nat Methods 6, 741-744.
Skoufias, D.A., Indorato, R.L., Lacroix, F., Panopoulos, A., and Margolis, R.L. (2007). Mitosis persists in the absence of Cdk1 activity when proteolysis or protein phosphatase activity is suppressed. J Cell Biol 179, 671-685.
Song, C., Ye, M., Liu, Z., Cheng, H., Jiang, X., Han, G., Songyang, Z., Tan, Y., Wang, H., Ren, J., et al. (2012). Systematic analysis of protein phosphorylation networks from phosphoproteomic data. Mol Cell Proteomics 11, 1070-1083.
Songyang, Z., Blechner, S., Hoagland, N., Hoekstra, M.F., Piwnica-Worms, H., and Cantley, L.C. (1994). Use of an oriented peptide library to determine the optimal substrates of protein kinases. Curr Biol 4, 973-982.
Sutherland, J.J., Gao, C., Cahya, S., and Vieth, M. (2013). What general conclusions can we draw from kinase profiling data sets? Biochim Biophys Acta 1834, 1425-1433.
Suzuki, K., Sako, K., Akiyama, K., Isoda, M., Senoo, C., Nakajo, N., and Sagata, N. (2015). Identification of non-Ser/Thr-Pro consensus motifs for Cdk1 and their roles in mitotic regulation of C2H2 zinc finger proteins and Ect2. Sci Rep 5, 7929.
Suzuki, R., and Shimodaira, H. (2006). Pvclust: an R package for assessing the uncertainty in hierarchical clustering. Bioinformatics 22, 1540-1542.
Trost, B., and Kusalik, A. (2011). Computational prediction of eukaryotic phosphorylation sites. Bioinformatics 27, 2927-2935.
Vieth, M., Higgs, R.E., Robertson, D.H., Shapiro, M., Gragg, E.A., and Hemmerle, H. (2004). Kinomics-structural biology and chemogenomics of kinase inhibitors and targets. Biochim Biophys Acta 1697, 243-257.
von Stechow, L., Francavilla, C., and Olsen, J.V. (2015). Recent findings and technological advances in phosphoproteomics for cells and tissues. Expert Rev Proteomics 12, 469-487.
Walton, G.M., Chen, W.S., Rosenfeld, M.G., and Gill, G.N. (1990). Analysis of deletions of the carboxyl terminus of the epidermal growth factor receptor reveals self-phosphorylation at tyrosine 992 and enhanced in vivo tyrosine phosphorylation of cell substrates. J Biol Chem 265, 1750-1754.
Wang, F., Ulyanova, N.P., van der Waal, M.S., Patnaik, D., Lens, S.M.A., and Higgins, J.M.G. (2011). A positive feedback loop involving Haspin and Aurora B promotes CPC accumulation at centromeres in mitosis. Curr. Biol. 21, 1061-1069.
Ward, J.H. (1963). Hierarchical Grouping to Optimize an Objective Function. Journal of the American Statistical Association 58, 236-244.
Weernink, P.A., and Rijksen, G. (1995). Activation and translocation of c-Src to the cytoskeleton by both platelet-derived growth factor and epidermal growth factor. J Biol Chem 270, 2264-2267.
Wilson, L.J., Linley, A., Hammond, D.E., Hood, F.E., Coulson, J.M., MacEwan, D.J., Ross, S.J., Slupsky, J.R., Smith, P.D., Eyers, P.A., et al. (2018). New Perspectives, Opportunities, and Challenges in Exploring the Human Protein Kinome. Cancer Res 78, 15-29.
Yaffe, M.B., Leparc, G.G., Lai, J., Obata, T., Volinia, S., and Cantley, L.C. (2001). A motif-based profile scanning approach for genome-wide prediction of signaling pathways. Nat Biotechnol 19, 348-353.
Zhou, L., Tian, X., Zhu, C., Wang, F., and Higgins, J.M. (2014). Polo-like kinase-1 triggers histone phosphorylation by Haspin in mitosis. EMBO Rep 15, 273-281.

## Claims

1. A method of identifying or profiling a target cellular kinase that phosphorylates a selected substrate, comprising:
a) providing a plurality of test protein preparations comprising the target cellular kinase and at least one phosphatase inhibitor;
b) incubating each test protein preparation with the selected substrate and a kinase inhibitor, wherein the kinase inhibitor is different for each test protein preparation;
c) determining whether the selected substrate is phosphorylated in each of the test protein preparations of b), thereby obtaining an inhibition profile for the target cellular kinase; and
d) comparing the target cellular kinase inhibition profile with the inhibition profiles of known kinases, wherein the most similar known kinase inhibition profile(s) identify or profile the target cellular kinase.

2. The method of claim 1, wherein the method further comprises the step of lysing cells comprising the target cellular kinase in the presence of at least one phosphatase inhibitor to generate the plurality of protein preparations of step a).

3. The method of claim 2, wherein the cell sample is human.

4. The method of any preceding claim, wherein the protein preparations are cell lysates or cell extracts.

5. The method of any preceding claim, wherein the at least one phosphatase inhibitor is selected from: okadaic acid, sodium fluoride, β-glycerophosphate, calyculin A, microcystin LR, cantharidin, sodium molybdate, sodium tartrate, (-)-p-bromotetramisole oxalate, sodium orthovanadate, sodium pyrophosphate, imidazole, Ascomycin, Cyclosporin A, FK506, Fostriecin, Fumonisin B1, GSK2830371, 9,10-Dihydro-9,10[1',2']-benzenoanthracene-1,4-dione, Raphin 1, Salubrinal, Sal 003, Sanguinarine, Sephin 1, Tautomycetin, Tautomycin, DL-2-Amino-3-phosphonopropionic acid (AP-3), Arcapillin, Endothall, Levamisole, Rubratoxin or combinations thereof.

6. The method of any preceding claim, wherein the selected substrate has a single tyrosine, threonine or serine phosphosite; optionally wherein the selected substrate is a peptide.

7. The method of any preceding claim, wherein the selected substrate is at a concentration of from 0.01 µM to 10 µM in step b).

8. The method of any preceding claim, wherein at least ten different kinase inhibitors are used individually in step b).

9. The method of any preceding claim, wherein the kinase inhibitor is at a concentration of from 0.1 µM to 50 µM in step b).

10. The method of any preceding claim, wherein step b) is for at least 5 minutes.

11. The method of any preceding claim, wherein phosphorylation of the selected substrate is determined using ³²P-orthophosphate radiolabelling, mass changes as determined by mass spectrometry or other means, phospho-specific antibodies, ELISA, immunoblotting or a combination thereof.

12. The method of any preceding claim, wherein the target cellular kinase inhibition profile is compared to at least ten known inhibition profiles.

13. The method of any preceding claim, wherein the comparison in step d) further comprises generating at least one of the following:
(i) a ranking of correlation coefficients, supported by kinase inhibition correlation plots;
(ii) plotting correlation coefficients on a sequence-based human kinome tree;
(iii) an inhibition-based kinome dendrogram derived by hierarchical clustering using correlation coefficients, optionally with bootstrapping; or
(iv) a combination of (i) to (iii).

14. The method of any preceding claim, wherein the method further comprises stimulating a signalling pathway associated with phosphorylation of the selected substrate and/or synchronising the cell cycle in the cells before generating the plurality of test protein preparations.

15. The method of any preceding claim, wherein the target cellular kinase is associated with aberrant phosphorylation in a disease or disorder; optionally wherein the disease or disorder is cancer, rheumatoid arthritis, chronic immune thrombocytopenia, glaucoma, idiopathic pulmonary fibrosis.

## Patentansprüche

1. Verfahren zum Identifizieren oder Profilieren einer Zielzellkinase, die ein ausgewähltes Substrat phosphoryliert, umfassend:
a) Bereitstellen einer Vielzahl von Testproteinzubereitungen, welche die Zielzellkinase und zumindest einen Phosphatasehemmer umfassen;
b) Inkubieren jeder Testproteinzubereitung mit dem ausgewählten Substrat und einem Kinasehemmer, wobei der Kinasehemmer für jede Testproteinzubereitung unterschiedlich ist;
c) Bestimmen, ob das ausgewählte Substrat in jeder der Testproteinzubereitungen von b) phosphoryliert ist, wodurch ein Hemmungsprofil für die Zielzellkinase erhalten wird; und
d) Vergleichen des Zielzellkinasehemmungsprofils mit den Hemmungsprofilen von bekannten Kinasen, wobei das/die ähnlichste(n) bekannte(n) Kinasehemmungsprofil(e) die Zielzellkinase identifizieren oder profilieren.

2. Verfahren nach Anspruch 1, wobei das Verfahren ferner den Schritt des Lysierens von Zellen, welche die Zielzellkinase umfassen, in der Gegenwart von zumindest einem Phosphatasehemmer umfasst, um die Vielzahl von Proteinzubereitungen von Schritt a) zu erzeugen.

3. Verfahren nach Anspruch 2, wobei die Zellprobe menschlich ist.

4. Verfahren nach einem vorhergehenden Anspruch, wobei die Proteinzubereitungen Zelllysate oder Zellextrakte sind.

5. Verfahren nach einem vorhergehenden Anspruch, wobei der zumindest eine Phosphatasehemmer ausgewählt ist aus: Okadasäure, Natriumfluorid, β-Glycerophosphat, Calyculin A, Microcystin LR, Cantharidin, Natriummolybdat, Natriumtartrat, (-)-p-Bromtetramisoloxalat, Natriumorthovanadat, Natriumpyrophosphat, Imidazol, Ascomycin, Cyclosporin A, FK506, Fostriecin, Fumonisin B1, GSK2830371, 9,10-Dihydro-9,10[1',2']-benzoloanthracen-1,4-dion, Raphin 1, Salubrinal, Sal 003, Sanguinarin, Sephin 1, Tautomycetin, Tautomycin, DL-2-Amino-3-phosphonopropionsäure (AP-3), Arcapillin, Endothall, Levamisol, Rubratoxin oder Kombinationen davon.

6. Verfahren nach einem vorhergehenden Anspruch, wobei das ausgewählte Substrat ein einzelnes Tyrosin-, Threonin- oder Serinphosphosit aufweist; wobei optional das ausgewählte Substrat ein Peptid ist.

7. Verfahren nach einem vorhergehenden Anspruch, wobei das ausgewählte Substrat in einer Konzentration von 0,01 µM bis 10 µM in Schritt b) ist.

8. Verfahren nach einem vorhergehenden Anspruch, wobei zumindest zehn verschiedene Kinasehemmer einzeln in Schritt b) verwendet werden.

9. Verfahren nach einem vorhergehenden Anspruch, wobei der Kinasehemmer in einer Konzentration von 0,1 µM bis 50 µM in Schritt b) ist.

10. Verfahren nach einem vorhergehenden Anspruch, wobei Schritt b) für zumindest 5 Minuten ist.

11. Verfahren nach einem vorhergehenden Anspruch, wobei Phosphorylierung des ausgewählten Substrats unter Verwendung von ³²P-Orthophosphat-Radiomarkierung, Massenänderungen wie bestimmt durch Massenspektrometrie oder andere Mittel, phosphospezifische Antikörper, ELISA, Immunblotting oder einer Kombination davon bestimmt wird.

12. Verfahren nach einem vorhergehenden Anspruch, wobei das Zielzellkinasehemmungsprofil mit zumindest zehn bekannten Hemmungsprofilen verglichen wird.

13. Verfahren nach einem vorhergehenden Anspruch, wobei der Vergleich in Schritt d) ferner Erzeugen von zumindest einem des Folgenden umfasst:
(i) einer Rangfolge von Korrelationskoeffizienten, unterstützt durch Kinasehemmungskorrelationsdarstellungen;
(ii) Darstellung von Korrelationskoeffizienten auf einem sequenzbasierten menschlichen Kinombaum;
(iii) einem hemmungsbasierten Kinomdendrogramm, das durch hierarchisches Clustering unter Verwendung von Korrelationskoeffizienten, optional mit Bootstrapping, abgeleitet wird; oder
(iv) einer Kombination aus (i) bis (iii).

14. Verfahren nach einem vorhergehenden Anspruch, wobei das Verfahren ferner Stimulieren eines Signalweges, der mit Phosphorylierung des ausgewählten Substrats assoziiert ist, und/oder Synchronisieren des Zellzyklus in den Zellen vor dem Erzeugen der Vielzahl von Testproteinzubereitungen umfasst.

15. Verfahren nach einem vorhergehenden Anspruch, wobei die Zielzellkinase mit abweichender Phosphorylierung bei einer Erkrankung oder Störung assoziiert ist; wobei optional die Erkrankung oder Störung Krebs, rheumatoide Arthritis, chronische Immunthrombozytopenie, Glaukom, idiopathische Lungenfibrose ist.

## Revendications

1. Méthode d'identification ou de profilage d'une kinase cellulaire cible qui phosphoryle un substrat choisi, comprenant :
a) la fourniture d'une pluralité de préparations de protéines tests comprenant la kinase cellulaire cible et au moins un inhibiteur de phosphatase ;
b) l'incubation de chaque préparation de protéines test avec le substrat choisi et un inhibiteur de kinase, ledit inhibiteur de kinase étant différent pour chaque préparation de protéines test ;
c) la détermination pour savoir si le substrat choisi est phosphorylé dans chacune des préparations de protéines tests de b), ce qui permet d'obtenir un profil d'inhibition pour la kinase cellulaire cible ; et
d) la comparaison du profil d'inhibition de la kinase cellulaire cible avec les profils d'inhibition de kinases connues, ledit ou lesdits profils d'inhibition de kinases connues les plus similaires identifiant ou profilant la kinase cellulaire cible.

2. Méthode selon la revendication 1, ladite méthode comprenant en outre l'étape de lyse de cellules comprenant la kinase cellulaire cible en présence d'au moins un inhibiteur de phosphatase pour générer la pluralité de préparations de protéines de l'étape a).

3. Méthode selon la revendication 2, ledit échantillon de cellules étant humain.

4. Méthode selon l'une quelconque des revendications précédentes, lesdites préparations de protéines étant des lysats de cellules ou des extraits de cellules.

5. Méthode selon l'une quelconque des revendications précédentes, ledit au moins un inhibiteur de phosphatase étant choisi parmi : l'acide okadaïque, le fluorure de sodium, le β-glycérophosphate, la calyculine A, la microcystine LR, la cantharidine, le molybdate de sodium, le tartrate de sodium, l'oxalate de (-)-p-bromotétramisole, l'orthovanadate de sodium, le pyrophosphate de sodium, l'imidazole, l'ascomycine, la cyclosporine A, le FK506, la fostriécine, la fumonisine B1, le GSK2830371, la 9,10-dihydro-9,10[1',2']-benzénoanthracène-1,4-dione, la raphine 1, le salubrinal, le sal 003, la sanguinarine, la séphine 1, la tautomycétine, la tautomycine, l'acide DL-2-amino-3-phosphonopropionique (AP-3), l'arcapilline, l'endothall, le lévamisole, la rubratoxine ou leurs combinaisons.

6. Méthode selon l'une quelconque des revendications précédentes, ledit substrat choisi comportant une seule tyrosine, thréonine ou phosphosite de sérine ; éventuellement ledit substrat choisi étant un peptide.

7. Méthode selon l'une quelconque des revendications précédentes, ledit substrat choisi étant à une concentration de 0,01 µM à 10 µM à l'étape b).

8. Méthode selon l'une quelconque des revendications précédentes, au moins dix inhibiteurs de kinase différents étant utilisés individuellement à l'étape b).

9. Méthode selon l'une quelconque des revendications précédentes, ledit inhibiteur de kinase étant à une concentration de 0,1 µM à 50 µM à l'étape b).

10. Méthode selon l'une quelconque des revendications précédentes, ladite étape b) durant pendant au moins 5 minutes.

11. Méthode selon l'une quelconque des revendications précédentes, ladite phosphorylation du substrat choisi étant déterminée à l'aide d'un radiomarquage au ³²P-orthophosphate, de changements de masse tels que déterminés par spectrométrie de masse ou par d'autres moyens, d'anticorps phospho-spécifiques, du test ELISA, d'un immunotransfert ou d'une combinaison de ceux-ci.

12. Méthode selon l'une quelconque des revendications précédentes, ledit profil d'inhibition de kinase cellulaire cible étant comparé à au moins dix profils d'inhibition connus.

13. Méthode selon l'une quelconque des revendications précédentes, ladite comparaison à l'étape d) comprenant en outre la génération d'au moins l'un des suivants :
(i) un classement des coefficients de corrélation, étayé par des courbes de corrélation d'inhibition de kinase ;
(ii) la représentation graphique des coefficients de corrélation sur un arbre de kinome humain basé sur une séquence ;
(iii) un dendrogramme de kinome basé sur l'inhibition dérivé par regroupement hiérarchique à l'aide de coefficients de corrélation, éventuellement avec une méthode de bootstrap ; ou
(iv) une combinaison de (i) à (iii).

14. Méthode selon l'une quelconque des revendications précédentes, ladite méthode comprenant en outre la stimulation d'une voie de signalisation associée à la phosphorylation du substrat choisi et/ou la synchronisation du cycle cellulaire dans les cellules avant la génération de la pluralité de préparations de protéines tests.

15. Méthode selon l'une quelconque des revendications précédentes, ladite kinase cellulaire cible étant associée à une phosphorylation aberrante dans une maladie ou un trouble ; éventuellement ladite maladie ou ledit trouble étant un cancer, une polyarthrite rhumatoïde, une thrombocytopénie immunitaire chronique, un glaucome, une fibrose pulmonaire idiopathique.
